# EUROPEAN PATENT SPECIFICATION

(11) **EP 2 151 493 B1**
(45) Date of publication and mention of the grant of the patent: **13.06.2012**
(21) Application number: 09014803.2
(22) Date of filing: 29.06.2001
(51) Int. Cl.: C12N 5/10, C12N 15/87, C07K 14/02

(54) **Host cells containing multiple integrating vectors**
Wirtzellen mit mehreren integrierenden Vektoren
Cellules-hôte avec multiples vecteurs intégratifs

(30) Priority: 03.07.2000 US 215925 P
(43) Date of publication of application: 10.02.2010
(62) Divisional of application: 01950645.0
(73) Proprietor: Catalent Pharma Solutions, LLC, Somerset, NJ 08873 (US)
(72) Inventor: Bremel, Robert D., Hillpoint, WI 53937 (US); Miller, Linda U., Lodi, WI 53555 (US); Bleck, Gregory T., Cross Plain, WI 53528 (US)
(74) Representative: Glawe, Delfs, Moll

(56) References cited:
- WO-A-93/02108
- WO-A-94/24870
- HARRIS JF ET AL.: "Increased frequency of both total and specific monoclonal antibody producing hybridomas using a fusion partner that constitutively expresses recombinant IL-6" J. IMMUNOL. METHODS, vol. 148, 1992, pages 199-207, XP002560017
- WALKER PS ET AL.: "ViraL interference during simultaneous transduction with two independent helper-free retroviral vectors." HUM. GENE THER., vol. 7, 1996, XP008064317
- KUO MF ET AL.: "Integration and expression of porcine growth hormone gene after being transfected into the mouse embryonic stem cells by retroviral vector" PROC. CHINESE SOC. ANIMAL SCI, vol. 27, no. 1, 1998, pages 59-73, XP002560019
- HOEBEN RC ET AL.: "Expression of functional Factor VIII in primary human skin fibroblasts after retrovirus-mediated gene transfer." J. BIOL. CHEM., vol. 265, no. 13, 1990, pages 7318-7323, XP002560018
- SIJIAN W ET AL: "Development of a VSV-G protein pseudotyped retroviral vector system expressing dominant oncogenes from a lacO-modified inducible LTR promoter" GENE, ELSEVIER, AMSTERDAM, NL, vol. 182, no. 1-2, 5 December 1996 (1996-12-05), pages 145-150, XP004071943 ISSN: 0378-1119

### SEQUENCE LISTING

<110> Gala Design, Inc.
<120> Host Cells Containing Multiple Integrating Vectors
<130> GALD023PEP02
<140> not yet known
   <141> 2003-02-03
<150> EP 01 950 645.0
   <151> 2003-02-03
<150> PCT/US 01/20710
   <151> 2001-06-29
<150> US 60/215,925
   <151> 2000-07-03
<160> 36
<170> PatentIn version 3.5
<210> 1
   <211> 2101
   <212> DNA
   <213> Artificial
<220>
   <223> Synthetic
<400> 1
   gatcagtcct gggtggtcat tgaaaggact gatgctgaag ttgaagctcc aatactttgg      60
   ccacctgatg cgaagaactg actcatgtga taagaccctg atactgggaa agattgaagg     120
   caggaggaga agggatgaca gaggatggaa gagttggatg gaatcaccaa ctcgatggac     180
   atgagtttga gcaagcttcc aggagttggt aatgggcagg gaagcctggc gtgctgcagt     240
   ccatggggtt gcaaagagtt ggacactact gagtgactga actgaactga tagtgtaatc     300
   catggtacag aatataggat aaaaaagagg aagagtttgc cctgattctg aagagttgta     360
   ggatataaaa gtttagaata cctttagttt ggaagtctta aattatttac ttaggatggg     420
   tacccactgc aatataagaa atcaggcttt agagactgat gtagagagaa tgagccctgg     480
   cataccagaa gctaacagct attggttata gctgttataa ccaatatata accaatatat     540
   tggttatata gcatgaagct tgatgccagc aatttgaagg aaccatttag aactagtatc     600
   ctaaactcta catgttccag gacactgatc ttaaagctca ggttcagaat cttgttttat     660
   aggctctagg tgtatattgt ggggcttccc tggtggctca gatggtaaag tgtctgcctg     720
   caatgtgggt gatctgggtt cgatccctgg cttgggaaga tcccctggag aaggaaatgg     780
   caacccactc tagtactctt acctggaaaa ttccatggac agaggagcct tgtaagctac     840
   agtccatggg attgcaaaga gttgaacaca actgagcaac taagcacagc acagtacagt     900
   atacacctgt gaggtgaagt gaagtgaagg ttcaatgcag ggtctcctgc attgcagaaa   960
   gattctttac catctgagcc accagggaag cccaagaata ctggagtggg tagcctattc  1020
   cttctccagg ggatcttccc atcccaggaa ttgaactgga gtctcctgca tttcaggtgg  1080
   attcttcacc agctgaacta ccaggtggat actactccaa tattaaagtg cttaaagtcc  1140
   agttttccca cctttcccaa aaaggttggg tcactctttt ttaaccttct gtggcctact  1200
   ctgaggctgt ctacaagctt atatatttat gaacacattt attgcaagtt gttagtttta  1260
   gatttacaat gtggtatctg gctatttagt ggtattggtg gttggggatg gggaggctga  1320
   tagcatctca gagggcagct agatactgtc atacacactt ttcaagttct ccatttttgt  1380
   gaaatagaaa gtctctggat ctaagttata tgtgattctc agtctctgtg gtcatattct  1440
   attctactcc tgaccactca acaaggaacc aagatatcaa gggacacttg ttttgtttca  1500
   tgcctgggtt gagtgggcca tgacatatgt tctgggcctt gttacatggc tggattggtt  1560
   ggacaagtgc cagctctgat cctgggactg tggcatgtga tgacatacac cccctctcca  1620
   cattctgcat gtctctaggg gggaaggggg aagctcggta tagaaccttt attgtatttt  1680
   ctgattgcct cacttcttat attgccccca tgcccttctt tgttcctcaa gtaaccagag  1740
   acagtgcttc ccagaaccaa ccctacaaga aacaaagggc taaacaaagc caaatgggaa  1800
   gcaggatcat ggtttgaact ctttctggcc agagaacaat acctgctatg gactagatac  1860
   tgggagaggg aaaggaaaag tagggtgaat tatggaagga agctggcagg ctcagcgttt  1920
   ctgtcttggc atgaccagtc tctcttcatt ctcttcctag atgtagggct tggtaccaga  1980
   gcccctgagg ctttctgcat gaatataaat atatgaaact gagtgatgct tccatttcag  2040
   gttcttgggg gcgccgaatt cgagctcggt acccggggat ctcgaggggg ggcccggtac  2100
   c                                                                  2101
<210> 2
   <211> 245
   <212> DNA
   <213> Artificial
<220>
   <223> Synthetic
<400> 2
   gattacttac tggcaggtgc tgggggcttc cgagacaatc gcgaacatct acaccacaca 60
   acaccgcctc gaccagggtg agatatcggc cggggacgcg gcggtggtaa ttacaagcga 120
   ggatccgatt acttactggc aggtgctggg ggcttccgag acaatcgcga acatctacac 180
   cacacaacac cgcctcgacc agggtgagat atcggccggg gacgcggcgg tggtaattac 240
   aagcg 245
<210> 3
   <211> 680
   <212> DNA
   <213> Artificial
<220>
   <223> Synthetic
<400> 3
   ggaattcgcc cctctccctc ccccccccct aacgttactg gccgaagccg cttggaataa 60
   ggccggtgtg cgtttgtcta tatgttattt tccaccatat tgccgtcttt tggcaatgtg 120
   agggcccgga aacctggccc tgtcttcttg acgagcattc ctaggggtct ttcccctctc 180
   gccaaaggaa tgcaaggtct gttgaatgtc gtgaaggaag cagttcctct ggaagcttct 240
   tgaagacaaa caacgtctgt agcgaccctt tgcaggcagc ggaacccccc acctggcgac 300
   aggtgcctct gcggccaaaa gccacgtgta taagatacac ctgcaaaggc ggcacaaccc 360
   cagtgccacg ttgtgagttg gatagttgtg gaaagagtca aatggctctc ctcaagcgta 420
   ttcaacaagg ggctgaagga tgcccagaag gtaccccatt gtatgggatc tgatctgggg 480
   cctcggtgca catgctttac atgtgtttag tcgaggttaa aaaaacgtct aggccccccg 540
   aaccacgggg acgtggtttt cctttgaaaa acacgatgat aatatggcct cctttgtctc 600
   tctgctcctg gtaggcatcc tattccatgc cacccaggcc ggcgccatgg gatatctaga 660
   tctcgagctc gcgaaagctt 680
<210> 4
   <211> 4207
   <212> DNA
   <213> Artificial
<220>
   <223> Synthetic
<400> 4
   cggatccggc cattagccat attattcatt ggttatatag cataaatcaa tattggctat      60
   tggccattgc atacgttgta tccatatcat aatatgtaca tttatattgg ctcatgtcca     120
   acattaccgc catgttgaca ttgattattg actagttatt aatagtaatc aattacgggg     180
   tcattagttc atagcccata tatggagttc cgcgttacat aacttacggt aaatggcccg     240
   cctggctgac cgcccaacga cccccgccca ttgacgtcaa taatgacgta tgttcccata     300
   gtaacgccaa tagggacttt ccattgacgt caatgggtgg agtatttacg gtaaactgcc     360
   cacttggcag tacatcaagt gtatcatatg ccaagtacgc cccctattga cgtcaatgac     420
   ggtaaatggc ccgcctggca ttatgcccag tacatgacct tatgggactt tcctacttgg     480
   cagtacatct acgtattagt catcgctatt accatggtga tgcggttttg gcagtacatc     540
   aatgggcgtg gatagcggtt tgactcacgg ggatttccaa gtctccaccc cattgacgtc     600
   aatgggagtt tgttttggca ccaaaatcaa cgggactttc caaaatgtcg taacaactcc     660
   gccccattga cgcaaatggg cggtaggcat gtacggtggg aggtctatat aagcagagct     720
   cgtttagtga accgtcagat cgcctggaga cgccatccac gctgttttga cctccataga     780
   agacaccggg accgatccag cctccgcggc cccaagcttc tcgacggatc cccgggaatt     840
   caggacctca ccatgggatg gagctgtatc atcctcttct tggtagcaac agctacaggt     900
   gtccactccg aggtccaact ggtggagagc ggtggaggtg ttgtgcaacc tggccggtcc     960
   ctgcgcctgt cctgctccgc atctggcttc gatttcacca catattggat gagttgggtg    1020
   agacaggcac ctggaaaagg tcttgagtgg attggagaaa ttcatccaga tagcagtacg    1080
   attaactatg cgccgtctct aaaggataga tttacaatat cgcgagacaa cgccaagaac    1140
   acattgttcc tgcaaatgga cagcctgaga cccgaagaca ccggggtcta tttttgtgca    1200
   agcctttact tcggcttccc ctggtttgct tattggggcc aagggacccc ggtcaccgtc    1260
   tcctcagcct ccaccaaggg cccatcggtc ttccccctgg caccctcctc caagagcacc    1320
   tctgggggca cagcggccct gggctgcctg gtcaaggact acttccccga accggtgacg    1380
   gtgtcgtgga actcaggcgc cctgaccagc ggcgtgcaca ccttcccggc tgtcctacag    1440
   tcctcaggac tctactccct cagcagcgtg gtgaccgtgc cctccagcag cttgggcacc    1500
   cagacctaca tctgcaacgt gaatcacaag cccagcaaca ccaaggtgga caagagagtt    1560
   gagcccaaat cttgtgacaa aactcacaca tgcccaccgt gcccagcacc tgaactcctg    1620
   gggggaccgt cagtcttcct cttcccccca aaacccaagg acaccctcat gatctcccgg    1680
   acccctgagg tcacatgcgt ggtggtggac gtgagccacg aagaccctga ggtcaagttc    1740
   aactggtacg tggacggcgt ggaggtgcat aatgccaaga caaagccgcg ggaggagcag    1800
   tacaacagca cgtaccgtgt ggtcagcgtc ctcaccgtcc tgcaccagga ctggctgaat    1860
   ggcaaggagt acaagtgcaa ggtctccaac aaagccctcc cagcccccat cgagaaaacc    1920
   atctccaaag ccaaagggca gccccgagaa ccacaggtgt acaccctgcc cccatcccgg    1980
   gaggagatga ccaagaacca ggtcagcctg acctgcctgg tcaaaggctt ctatcccagc    2040
   gacatcgccg tggagtggga gagcaatggg cagccggaga acaactacaa gaccacgcct    2100
   cccgtgctgg actccgacgg ctccttcttc ctctatagca agctcaccgt ggacaagagc    2160
   aggtggcagc aggggaacgt cttctcatgc tccgtgatgc acgaggctct gcacaaccac    2220
   tacacgcaga agagcctctc cctgtctccc gggaaatgaa agccgaattc gcccctctcc    2280
   ctcccccccc cctaacgtta ctggccgaag ccgcttggaa taaggccggt gtgcgtttgt    2340
   ctatatgtta ttttccacca tattgccgtc ttttggcaat gtgagggccc ggaaacctgg    2400
   ccctgtcttc ttgacgagca ttcctagggg tctttcccct ctcgccaaag gaatgcaagg    2460
   tctgttgaat gtcgtgaagg aagcagttcc tctggaagct tcttgaagac aaacaacgtc    2520
   tgtagcgacc ctttgcaggc agcggaaccc cccacctggc gacaggtgcc tctgcggcca 2580
   aaagccacgt gtataagata cacctgcaaa ggcggcacaa ccccagtgcc acgttgtgag 2640
   ttggatagtt gtggaaagag tcaaatggct ctcctcaagc gtattcaaca aggggctgaa 2700
   ggatgcccag aaggtacccc attgtatggg atctgatctg gggcctcggt gcacatgctt 2760
   tacatgtgtt tagtcgaggt taaaaaaacg tctaggcccc ccgaaccacg gggacgtggt 2820
   tttcctttga aaaacacgat gataatatgg cctcctttgt ctctctgctc ctggtaggca 2880
   tcctattcca tgccacccag gccgacatcc agctgaccca gagcccaagc agcctgagcg 2940
   ccagcgtggg tgacagagtg accatcacct gtaaggccag tcaggatgtg ggtacttctg 3000
   tagcctggta ccagcagaag ccaggtaagg ctccaaagct gctgatctac tggacatcca 3060
   cccggcacac tggtgtgcca agcagattca gcggtagcgg tagcggtacc gacttcacct 3120
   tcaccatcag cagcctccag ccagaggaca tcgccaccta ctactgccag caatatagcc 3180
   tctatcggtc gttcggccaa gggaccaagg tggaaatcaa acgaactgtg gctgcaccat 3240
   ctgtcttcat cttcccgcca tctgatgagc agttgaaatc tggaactgcc tctgttgtgt 3300
   gcctgctgaa taacttctat cccagagagg ccaaagtaca gtggaaggtg gataacgccc 3360
   tccaatcggg taactcccag gagagtgtca cagagcagga cagcaaggac agcacctaca 3420
   gcctcagcag caccctgacg ctgagcaaag cagactacga gaaacacaaa gtctacgcct 3480
   gcgaagtcac ccatcagggc ctgagctcgc ccgtcacaaa gagcttcaac aggggagagt 3540
   gttagagatc taggcctcct aggtcgacat cgataaaata aaagatttta tttagtctcc 3600
   agaaaaaggg gggaatgaaa gaccccacct gtaggtttgg caagctagct taagtaacgc 3660
   cattttgcaa ggcatggaaa aatacataac tgagaataga gaagttcaga tcaaggtcag 3720
   gaacagatgg aacagctgaa tatgggccaa acaggatatc tgtggtaagc agttcctgcc 3780
   ccggctcagg gccaagaaca gatggaacag ctgaatatgg gccaaacagg atatctgtgg 3840
   taagcagttc ctgccccggc tcagggccaa gaacagatgg tccccagatg cggtccagcc 3900
   ctcagcagtt tctagagaac catcagatgt ttccagggtg ccccaaggac ctgaaatgac 3960
   cctgtgcctt atttgaacta accaatcagt tcgcttctcg cttctgttcg cgcgcttctg 4020
   ctccccgagc tcaataaaag agcccacaac ccctcactcg gggcgccagt cctccgattg 4080
   actgagtcgc ccgggtaccc gtgtatccaa taaaccctct tgcagttgca tccgacttgt 4140
   ggtctcgctg ttccttggga gggtctcctc tgagtgattg actacccgtc agcgggggtc 4200
   tttcatt 4207
<210> 5
   <211> 4210
   <212> DNA
   <213> Artificial
<220>
   <223> Synthetic
<400> 5
   ggatccggcc attagccata ttattcattg gttatatagc ataaatcaat attggctatt 60
   ggccattgca tacgttgtat ccatatcata atatgtacat ttatattggc tcatgtccaa 120
   cattaccgcc atgttgacat tgattattga ctagttatta atagtaatca attacggggt 180
   cattagttca tagcccatat atggagttcc gcgttacata acttacggta aatggcccgc 240
   ctggctgacc gcccaacgac ccccgcccat tgacgtcaat aatgacgtat gttcccatag 300
   taacgccaat agggactttc cattgacgtc aatgggtgga gtatttacgg taaactgccc 360
   acttggcagt acatcaagtg tatcatatgc caagtacgcc ccctattgac gtcaatgacg 420
   gtaaatggcc cgcctggcat tatgcccagt acatgacctt atgggacttt cctacttggc 480
   agtacatcta cgtattagtc atcgctatta ccatggtgat gcggttttgg cagtacatca 540
   atgggcgtgg atagcggttt gactcacggg gatttccaag tctccacccc attgacgtca 600
   atgggagttt gttttggcac caaaatcaac gggactttcc aaaatgtcgt aacaactccg 660
   ccccattgac gcaaatgggc ggtaggcatg tacggtggga ggtctatata agcagagctc 720
   gtttagtgaa ccgtcagatc gcctggagac gccatccacg ctgttttgac ctccatagaa 780
   gacaccggga ccgatccagc ctccgcggcc ccaagcttct cgacggatcc ccgggaattc 840
   aggacctcac catgggatgg agctgtatca tcctcttctt ggtagcaaca gctacaggtg 900
   tccactccca ggtccagctg gtccaatcag gggctgaagt caagaaacct gggtcatcag 960
   tgaaggtctc ctgcaaggct tctggctaca cctttactag ctactggctg cactgggtca 1020
   ggcaggcacc tggacagggt ctggaatgga ttggatacat taatcctagg aatgattata 1080
   ctgagtacaa tcagaacttc aaggacaagg ccacaataac tgcagacgaa tccaccaata 1140
   cagcctacat ggagctgagc agcctgaggt ctgaggacac ggcattttat ttttgtgcaa 1200
   gaagggatat tactacgttc tactggggcc aaggcaccac ggtcaccgtc tcctcagcct 1260
   ccaccaaggg cccatcggtc ttccccctgg caccctcctc caagagcacc tctgggggca 1320
   cagcggccct gggctgcctg gtcaaggact acttccccga accggtgacg gtgtcgtgga 1380
   actcaggcgc cctgaccagc ggcgtgcaca ccttcccggc tgtcctacag tcctcaggac 1440
   tctactccct cagcagcgtg gtgaccgtgc cctccagcag cttgggcacc cagacctaca 1500
   tctgcaacgt gaatcacaag cccagcaaca ccaaggtgga caagagagtt gagcccaaat 1560
   cttgtgacaa aactcacaca tgcccaccgt gcccagcacc tgaactcctg gggggaccgt 1620
   cagtcttcct cttcccccca aaacccaagg acaccctcat gatctcccgg acccctgagg 1680
   tcacatgcgt ggtggtggac gtgagccacg aagaccctga ggtcaagttc aactggtacg 1740
   tggacggcgt ggaggtgcat aatgccaaga caaagccgcg ggaggagcag tacaacagca 1800
   cgtaccgtgt ggtcagcgtc ctcaccgtcc tgcaccagga ctggctgaat ggcaaggagt 1860
   acaagtgcaa ggtctccaac aaagccctcc cagcccccat cgagaaaacc atctccaaag 1920
   ccaaagggca gccccgagaa ccacaggtgt acaccctgcc cccatcccgg gaggagatga 1980
   ccaagaacca ggtcagcctg acctgcctgg tcaaaggctt ctatcccagc gacatcgccg 2040
   tggagtggga gagcaatggg cagccggaga acaactacaa gaccacgcct cccgtgctgg 2100
   actccgacgg ctccttcttc ctctatagca agctcaccgt ggacaagagc aggtggcagc 2160
   aggggaacgt cttctcatgc tccgtgatgc acgaggctct gcacaaccac tacacgcaga 2220
   agagcctctc cctgtctccc gggaaatgaa agccgaattc gcccctctcc ctcccccccc 2280
   cctaacgtta ctggccgaag ccgcttggaa taaggccggt gtgcgtttgt ctatatgtta 2340
   ttttccacca tattgccgtc ttttggcaat gtgagggccc ggaaacctgg ccctgtcttc 2400
   ttgacgagca ttcctagggg tctttcccct ctcgccaaag gaatgcaagg tctgttgaat 2460
   gtcgtgaagg aagcagttcc tctggaagct tcttgaagac aaacaacgtc tgtagcgacc 2520
   ctttgcaggc agcggaaccc cccacctggc gacaggtgcc tctgcggcca aaagccacgt 2580
   gtataagata cacctgcaaa ggcggcacaa ccccagtgcc acgttgtgag ttggatagtt 2640
   gtggaaagag tcaaatggct ctcctcaagc gtattcaaca aggggctgaa ggatgcccag 2700
   aaggtacccc attgtatggg atctgatctg gggcctcggt gcacatgctt tacatgtgtt 2760
   tagtcgaggt taaaaaaacg tctaggcccc ccgaaccacg gggacgtggt tttcctttga 2820
   aaaacacgat gataatatgg cctcctttgt ctctctgctc ctggtaggca tcctattcca 2880
   tgccacccag gccgacatcc agctgaccca gtctccatca tctctgagcg catctgttgg 2940
   agatagggtc actatgagct gtaagtccag tcaaagtgtt ttatacagtg caaatcacaa 3000
   gaactacttg gcctggtacc agcagaaacc agggaaagca cctaaactgc tgatctactg 3060
   ggcatccact agggaatctg gtgtcccttc gcgattctct ggcagcggat ctgggacaga 3120
   ttttactttc accatcagct ctcttcaacc agaagacatt gcaacatatt attgtcacca 3180
   atacctctcc tcgtggacgt tcggtggagg gaccaaggtg cagatcaaac gaactgtggc 3240
   tgcaccatct gtcttcatct tcccgccatc tgatgagcag ttgaaatctg gaactgcctc 3300
   tgttgtgtgc ctgctgaata acttctatcc cagagaggcc aaagtacagt ggaaggtgga 3360
   taacgccctc caatcgggta actcccagga gagtgtcaca gagcaggaca gcaaggacag 3420
   cacctacagc ctcagcagca ccctgacgct gagcaaagca gactacgaga aacacaaagt 3480
   ctacgcctgc gaagtcaccc atcagggcct gagctcgccc gtcacaaaga gcttcaacag 3540
   gggagagtgt tagagatcta ggcctcctag gtcgacatcg ataaaataaa agattttatt 3600
   tagtctccag aaaaaggggg gaatgaaaga ccccacctgt aggtttggca agctagctta 3660
   agtaacgcca ttttgcaagg catggaaaaa tacataactg agaatagaga agttcagatc 3720
   aaggtcagga acagatggaa cagctgaata tgggccaaac aggatatctg tggtaagcag 3780
   ttcctgcccc ggctcagggc caagaacaga tggaacagct gaatatgggc caaacaggat 3840
   atctgtggta agcagttcct gccccggctc agggccaaga acagatggtc cccagatgcg 3900
   gtccagccct cagcagtttc tagagaacca tcagatgttt ccagggtgcc ccaaggacct 3960
   gaaatgaccc tgtgccttat ttgaactaac caatcagttc gcttctcgct tctgttcgcg 4020
   cgcttctgct ccccgagctc aataaaagag cccacaaccc ctcactcggg gcgccagtcc 4080
   tccgattgac tgagtcgccc gggtacccgt gtatccaata aaccctcttg cagttgcatc 4140
   cgacttgtgg tctcgctgtt ccttgggagg gtctcctctg agtgattgac tacccgtcag 4200
   gtctttcatt 4210
<210> 6
   <211> 5732
   <212> DNA
   <213> Artificial
<220>
   <223> Synthetic
<400> 6
   cgagcttggc agaaatggtt gaactcccga gagtgtccta cacctagggg agaagcagcc      60
   aaggggttgt ttcccaccaa ggacgacccg tctgcgcaca aacggatgag cccatcagac     120
   aaagacatat tcattctctg ctgcaaactt ggcatagctc tgctttgcct ggggctattg     180
   ggggaagttg cggttcgtgc tcgcagggct ctcacccttg actctttcaa taataactct     240
   tctgtgcaag attacaatct aaacaattcg gagaactcga ccttcctcct gaggcaagga     300
   ccacagccaa cttcctctta caagccgcat cgattttgtc cttcagaaat agaaataaga     360
   atgcttgcta aaaattatat ttttaccaat aagaccaatc caataggtag attattagtt     420
   actatgttaa gaaatgaatc attatctttt agtactattt ttactcaaat tcagaagtta     480
   gaaatgggaa tagaaaatag aaagagacgc tcaacctcaa ttgaagaaca ggtgcaagga     540
   ctattgacca caggcctaga agtaaaaaag ggaaaaaaga gtgtttttgt caaaatagga     600
   gacaggtggt ggcaaccagg gacttatagg ggaccttaca tctacagacc aacagatgcc     660
   cccttaccat atacaggaag atatgactta aattgggata ggtgggttac agtcaatggc     720
   tataaagtgt tatatagatc cctccccttt cgtgaaagac tcgccagagc tagacctcct     780
   tggtgtatgt tgtctcaaga aaagaaagac gacatgaaac aacaggtaca tgattatatt     840
   tatctaggaa caggaatgca cttttgggga aagattttcc ataccaagga ggggacagtg     900
   gctggactaa tagaacatta ttctgcaaaa acttatggca tgagttatta tgattagcct     960
   tgatttgccc aaccttgcgg ttcccaaggc ttaagtaagt ttttggttac aaactgttct 1020
   taaaacaagg atgtgagaca agtggtttcc tgacttggtt tggtatcaaa ggttctgatc 1080
   tgagctctga gtgttctatt ttcctatgtt cttttggaat ttatccaaat cttatgtaaa 1140
   tgcttatgta aaccaagata taaaagagtg ctgatttttt gagtaaactt gcaacagtcc 1200
   taacattcac ctcttgtgtg tttgtgtctg ttcgccatcc cgtctccgct cgtcacttat 1260
   ccttcacttt ccagagggtc cccccgcaga ccccggcgac cctcaggtcg gccgactgcg 1320
   gcagctggcg cccgaacagg gaccctcgga taagtgaccc ttgtctttat ttctactatt 1380
   ttgtgttcgt cttgttttgt ctctatcttg tctggctatc atcacaagag cggaacggac 1440
   tcacctcagg gaaccaagct agcccggggt cgacggatcc gattacttac tggcaggtgc 1500
   tgggggcttc cgagacaatc gcgaacatct acaccacaca acaccgcctc gaccagggtg 1560
   agatatcggc cggggacgcg gcggtggtaa ttacaagcga gatccgatta cttactggca 1620
   ggtgctgggg gcttccgaga caatcgcgaa catctacacc acacaacacc gcctcgacca 1680
   gggtgagata tcggccgggg acgcggcggt ggtaattaca agcgagatcc ccgggaattc 1740
   aggacctcac catgggatgg agctgtatca tcctcttctt ggtagcaaca gctacaggtg 1800
   tccactccga ggtccaactg gtggagagcg gtggaggtgt tgtgcaacct ggccggtccc 1860
   tgcgcctgtc ctgctccgca tctggcttcg atttcaccac atattggatg agttgggtga 1920
   gacaggcacc tggaaaaggt cttgagtgga ttggagaaat tcatccagat agcagtacga 1980
   ttaactatgc gccgtctcta aaggatagat ttacaatatc gcgagacaac gccaagaaca 2040
   cattgttcct gcaaatggac agcctgagac ccgaagacac cggggtctat ttttgtgcaa 2100
   gcctttactt cggcttcccc tggtttgctt attggggcca agggaccccg gtcaccgtct 2160
   cctcagcctc caccaagggc ccatcggtct tccccctggc accctcctcc aagagcacct 2220
   ctgggggcac agcggccctg ggctgcctgg tcaaggacta cttccccgaa ccggtgacgg 2280
   tgtcgtggaa ctcaggcgcc ctgaccagcg gcgtgcacac cttcccggct gtcctacagt 2340
   cctcaggact ctactccctc agcagcgtgg tgaccgtgcc ctccagcagc ttgggcaccc 2400
   agacctacat ctgcaacgtg aatcacaagc ccagcaacac caaggtggac aagagagttg 2460
   agcccaaatc ttgtgacaaa actcacacat gcccaccgtg cccagcacct gaactcctgg 2520
   ggggaccgtc agtcttcctc ttccccccaa aacccaagga caccctcatg atctcccgga 2580
   cccctgaggt cacatgcgtg gtggtggacg tgagccacga agaccctgag gtcaagttca 2640
   actggtacgt ggacggcgtg gaggtgcata atgccaagac aaagccgcgg gaggagcagt 2700
   acaacagcac gtaccgtgtg gtcagcgtcc tcaccgtcct gcaccaggac tggctgaatg 2760
   gcaaggagta caagtgcaag gtctccaaca aagccctccc agcccccatc gagaaaacca 2820
   tctccaaagc caaagggcag ccccgagaac cacaggtgta caccctgccc ccatcccggg 2880
   aggagatgac caagaaccag gtcagcctga cctgcctggt caaaggcttc tatcccagcg 2940
   acatcgccgt ggagtgggag agcaatgggc agccggagaa caactacaag accacgcctc 3000
   ccgtgctgga ctccgacggc tccttcttcc tctatagcaa gctcaccgtg gacaagagca 3060
   ggtggcagca ggggaacgtc ttctcatgct ccgtgatgca cgaggctctg cacaaccact 3120
   acacgcagaa gagcctctcc ctgtctcccg ggaaatgaaa gccgaattcg cccctctccc 3180
   tccccccccc ctaacgttac tggccgaagc cgcttggaat aaggccggtg tgcgtttgtc 3240
   tatatgttat tttccaccat attgccgtct tttggcaatg tgagggcccg gaaacctggc 3300
   cctgtcttct tgacgagcat tcctaggggt ctttcccctc tcgccaaagg aatgcaaggt 3360
   ctgttgaatg tcgtgaagga agcagttcct ctggaagctt cttgaagaca aacaacgtct 3420
   gtagcgaccc tttgcaggca gcggaacccc ccacctggcg acaggtgcct ctgcggccaa 3480
   aagccacgtg tataagatac acctgcaaag gcggcacaac cccagtgcca cgttgtgagt 3540
   tggatagttg tggaaagagt caaatggctc tcctcaagcg tattcaacaa ggggctgaag 3600
   gatgcccaga aggtacccca ttgtatggga tctgatctgg ggcctcggtg cacatgcttt 3660
   acatgtgttt agtcgaggtt aaaaaaacgt ctaggccccc cgaaccacgg ggacgtggtt 3720
   ttcctttgaa aaacacgatg ataatatggc ctcctttgtc tctctgctcc tggtaggcat 3780
   cctattccat gccacccagg ccgacatcca gctgacccag agcccaagca gcctgagcgc 3840
   cagcgtgggt gacagagtga ccatcacctg taaggccagt caggatgtgg gtacttctgt 3900
   agcctggtac cagcagaagc caggtaaggc tccaaagctg ctgatctact ggacatccac 3960
   ccggcacact ggtgtgccaa gcagattcag cggtagcggt agcggtaccg acttcacctt 4020
   caccatcagc agcctccagc cagaggacat cgccacctac tactgccagc aatatagcct 4080
   ctatcggtcg ttcggccaag ggaccaaggt ggaaatcaaa cgaactgtgg ctgcaccatc 4140
   tgtcttcatc ttcccgccat ctgatgagca gttgaaatct ggaactgcct ctgttgtgtg 4200
   cctgctgaat aacttctatc ccagagaggc caaagtacag tggaaggtgg ataacgccct 4260
   ccaatcgggt aactcccagg agagtgtcac agagcaggac agcaaggaca gcacctacag 4320
   cctcagcagc accctgacgc tgagcaaagc agactacgag aaacacaaag tctacgcctg 4380
   cgaagtcacc catcagggcc tgagctcgcc cgtcacaaag agcttcaaca ggggagagtg 4440
   ttagagatcc cccgggctgc aggaattcga tatcaagctt atcgataatc aacctctgga 4500
   ttacaaaatt tgtgaaagat tgactggtat tcttaactat gttgctcctt ttacgctatg 4560
   tggatacgct gctttaatgc ctttgtatca tgctattgct tcccgtatgg ctttcatttt 4620
   ctcctccttg tataaatcct ggttgctgtc tctttatgag gagttgtggc ccgttgtcag 4680
   gcaacgtggc gtggtgtgca ctgtgtttgc tgacgcaacc cccactggtt ggggcattgc 4740
   caccacctgt cagctccttt ccgggacttt cgctttcccc ctccctattg ccacggcgga 4800
   actcatcgcc gcctgccttg cccgctgctg gacaggggct cggctgttgg gcactgacaa 4860
   ttccgtggtg ttgtcgggga aatcatcgtc ctttccttgg ctgctcgcct gtgttgccac 4920
   ctggattctg cgcgggacgt ccttctgcta cgtcccttcg gccctcaatc cagcggacct 4980
   tccttcccgc ggcctgctgc cggctctgcg gcctcttccg cgtcttcgcc ttcgccctca 5040
   gacgagtcgg atctcccttt gggccgcctc cccgcctgat cgataccgtc aacatcgata 5100
   aaataaaaga ttttatttag tctccagaaa aaggggggaa tgaaagaccc cacctgtagg 5160
   tttggcaagc tagcttaagt aacgccattt tgcaaggcat ggaaaaatac ataactgaga 5220
   atagagaagt tcagatcaag gtcaggaaca gatggaacag ctgaatatgg gccaaacagg 5280
   atatctgtgg taagcagttc ctgccccggc tcagggccaa gaacagatgg aacagctgaa 5340
   tatgggccaa acaggatatc tgtggtaagc agttcctgcc ccggctcagg gccaagaaca 5400
   gatggtcccc agatgcggtc cagccctcag cagtttctag agaaccatca gatgtttcca 5460
   gggtgcccca aggacctgaa atgaccctgt gccttatttg aactaaccaa tcagttcgct 5520
   tctcgcttct gttcgcgcgc ttctgctccc cgagctcaat aaaagagccc acaacccctc 5580
   actcggggcg ccagtcctcc gattgactga gtcgcccggg tacccgtgta tccaataaac 5640
   cctcttgcag ttgcatccga cttgtggtct cgctgttcct tgggagggtc tcctctgagt 5700
   gattgactac ccgtcagcgg gggtctttca tt 5732
<210> 7
   <211> 9183
   <212> DNA
   <213> Artificial
<220>
   <223> Synthetic
<400> 7
   aaagacccca cccgtaggtg gcaagctagc ttaagtaacg ccactttgca aggcatggaa      60
   aaatacataa ctgagaatag aaaagttcag atcaaggtca ggaacaaaga aacagctgaa     120
   taccaaacag gatatctgtg gtaagcggtt cctgccccgg ctcagggcca agaacagatg     180
   agacagctga gtgatgggcc aaacaggata tctgtggtaa gcagttcctg ccccggctcg     240
   gggccaagaa cagatggtcc ccagatgcgg tccagccctc agcagtttct agtgaatcat     300
   cagatgtttc cagggtgccc caaggacctg aaaatgaccc tgtaccttat ttgaactaac     360
   caatcagttc gcttctcgct tctgttcgcg cgcttccgct ctccgagctc aataaaagag     420
   cccacaaccc ctcactcggc gcgccagtct tccgatagac tgcgtcgccc gggtacccgt     480
   attcccaata aagcctcttg ctgtttgcat ccgaatcgtg gtctcgctgt tccttgggag     540
   ggtctcctct gagtgattga ctacccacga cgggggtctt tcatttgggg gctcgtccgg 600
   gatttggaga cccctgccca gggaccaccg acccaccacc gggaggtaag ctggccagca 660
   acttatctgt gtctgtccga ttgtctagtg tctatgtttg atgttatgcg cctgcgtctg 720
   tactagttag ctaactagct ctgtatctgg cggacccgtg gtggaactga cgagttctga 780
   acacccggcc gcaaccctgg gagacgtccc agggactttg ggggccgttt ttgtggcccg 840
   acctgaggaa gggagtcgat gtggaatccg accccgtcag gatatgtggt tctggtagga 900
   gacgagaacc taaaacagtt cccgcctccg tctgaatttt tgctttcggt ttggaaccga 960
   agccgcgcgt cttgtctgct gcagcgctgc agcatcgttc tgtgttgtct ctgtctgact 1020
   gtgtttctgt atttgtctga aaattagggc cagactgtta ccactccctt aagtttgacc 1080
   ttaggtcact ggaaagatgt cgagcggatc gctcacaacc agtcggtaga tgtcaagaag 1140
   agacgttggg ttaccttctg ctctgcagaa tggccaacct ttaacgtcgg atggccgcga 1200
   gacggcacct ttaaccgaga cctcatcacc caggttaaga tcaaggtctt ttcacctggc 1260
   ccgcatggac acccagacca ggtcccctac atcgtgacct gggaagcctt ggcttttgac 1320
   ccccctccct gggtcaagcc ctttgtacac cctaagcctc cgcctcctct tcctccatcc 1380
   gccccgtctc tcccccttga acctcctcgt tcgaccccgc ctcgatcctc cctttatcca 1440
   gccctcactc cttctctagg cgccggaatt ccgatctgat caagagacag gatgaggatc 1500
   gtttcgcatg attgaacaag atggattgca cgcaggttct ccggccgctt gggtggagag 1560
   gctattcggc tatgactggg cacaacagac aatcggctgc tctgatgccg ccgtgttccg 1620
   gctgtcagcg caggggcgcc cggttctttt tgtcaagacc gacctgtccg gtgccctgaa 1680
   tgaactgcag gacgaggcag cgcggctatc gtggctggcc acgacgggcg ttccttgcgc 1740
   agctgtgctc gacgttgtca ctgaagcggg aagggactgg ctgctattgg gcgaagtgcc 1800
   ggggcaggat ctcctgtcat ctcaccttgc tcctgccgag aaagtatcca tcatggctga 1860
   tgcaatgcgg cggctgcata cgcttgatcc ggctacctgc ccattcgacc accaagcgaa 1920
   acatcgcatc gagcgagcac gtactcggat ggaagccggt cttgtcgatc aggatgatct 1980
   ggacgaagag catcaggggc tcgcgccagc cgaactgttc gccaggctca aggcgcgcat 2040
   gcccgacggc gaggatctcg tcgtgaccca tggcgatgcc tgcttgccga atatcatggt 2100
   ggaaaatggc cgcttttctg gattcatcga ctgtggccgg ctgggtgtgg cggaccgcta 2160
   tcaggacata gcgttggcta cccgtgatat tgctgaagag cttggcggcg aatgggctga 2220
   ccgcttcctc gtgctttacg gtatcgccgc tcccgattcg cagcgcatcg ccttctatcg 2280
   ccttcttgac gagttcttct gagcgggact ctggggttcg aaatgaccga ccaagcgacg 2340
   cccaacctgc catcacgaga tttcgattcc accgccgcct tctatgaaag gttgggcttc 2400
   ggaatcgttt tccgggacgc cggctggatg atcctccagc gcggggatct catgctggag 2460
   ttcttcgccc accccgggct cgatcccctc gcgagttggt tcagctgctg cctgaggctg 2520
   gacgacctcg cggagttcta ccggcagtgc aaatccgtcg gcatccagga aaccagcagc 2580
   ggctatccgc gcatccatgc ccccgaactg caggagtggg gaggcacgat ggccgctttg 2640
   gtcgaggcgg atcctagaac tagcgaaaat gcaagagcaa agacgaaaac atgccacaca 2700
   tgaggaatac cgattctctc attaacatat tcaggccagt tatctgggct taaaagcaga 2760
   agtccaaccc agataacgat catatacatg gttctctcca gaggttcatt actgaacact 2820
   cgtccgagaa taacgagtgg atcagtcctg ggtggtcatt gaaaggactg atgctgaagt 2880
   tgaagctcca atactttggc cacctgatgc gaagaactga ctcatgtgat aagaccctga 2940
   tactgggaaa gattgaaggc aggaggagaa gggatgacag aggatggaag agttggatgg 3000
   aatcaccaac tcgatggaca tgagtttgag caagcttcca ggagttggta atgggcaggg 3060
   aagcctggcg tgctgcagtc catggggttg caaagagttg gacactactg agtgactgaa 3120
   ctgaactgat agtgtaatcc atggtacaga atataggata aaaaagagga agagtttgcc 3180
   ctgattctga agagttgtag gatataaaag tttagaatac ctttagtttg gaagtcttaa 3240
   attatttact taggatgggt acccactgca atataagaaa tcaggcttta gagactgatg 3300
   tagagagaat gagccctggc ataccagaag ctaacagcta ttggttatag ctgttataac 3360
   caatatataa ccaatatatt ggttatatag catgaagctt gatgccagca atttgaagga 3420
   accatttaga actagtatcc taaactctac atgttccagg acactgatct taaagctcag 3480
   gttcagaatc ttgttttata ggctctaggt gtatattgtg gggcttccct ggtggctcag 3540
   atggtaaagt gtctgcctgc aatgtgggtg atctgggttc gatccctggc ttgggaagat 3600
   cccctggaga aggaaatggc aacccactct agtactctta cctggaaaat tccatggaca 3660
   gaggagcctt gtaagctaca gtccatggga ttgcaaagag ttgaacacaa ctgagcaact 3720
   aagcacagca cagtacagta tacacctgtg aggtgaagtg aagtgaaggt tcaatgcagg 3780
   gtctcctgca ttgcagaaag attctttacc atctgagcca ccagggaagc ccaagaatac 3840
   tggagtgggt agcctattcc ttctccaggg gatcttccca tcccaggaat tgaactggag 3900
   tctcctgcat ttcaggtgga ttcttcacca gctgaactac caggtggata ctactccaat 3960
   attaaagtgc ttaaagtcca gttttcccac ctttcccaaa aaggttgggt cactcttttt 4020
   taaccttctg tggcctactc tgaggctgtc tacaagctta tatatttatg aacacattta 4080
   ttgcaagttg ttagttttag atttacaatg tggtatctgg ctatttagtg gtattggtgg 4140
   ttggggatgg ggaggctgat agcatctcag agggcagcta gatactgtca tacacacttt 4200
   tcaagttctc catttttgtg aaatagaaag tctctggatc taagttatat gtgattctca 4260
   gtctctgtgg tcatattcta ttctactcct gaccactcaa caaggaacca agatatcaag 4320
   ggacacttgt tttgtttcat gcctgggttg agtgggccat gacatatgtt ctgggccttg 4380
   ttacatggct ggattggttg gacaagtgcc agctctgatc ctgggactgt ggcatgtgat 4440
   gacatacacc ccctctccac attctgcatg tctctagggg ggaaggggga agctcggtat 4500
   agaaccttta ttgtattttc tgattgcctc acttcttata ttgcccccat gcccttcttt 4560
   gttcctcaag taaccagaga cagtgcttcc cagaaccaac cctacaagaa acaaagggct 4620
   aaacaaagcc aaatgggaag caggatcatg gtttgaactc tttctggcca gagaacaata 4680
   cctgctatgg actagatact gggagaggga aaggaaaagt agggtgaatt atggaaggaa 4740
   gctggcaggc tcagcgtttc tgtcttggca tgaccagtct ctcttcattc tcttcctaga 4800
   tgtagggctt ggtaccagag cccctgaggc tttctgcatg aatataaata tatgaaactg 4860
   agtgatgctt ccatttcagg ttcttggggg cgccgaattc gagctcggta cccggggatc 4920
   tcgacggatc cgattactta ctggcaggtg ctgggggctt ccgagacaat cgcgaacatc 4980
   tacaccacac aacaccgcct cgaccagggt gagatatcgg ccggggacgc ggcggtggta 5040
   attacaagcg agatccgatt acttactggc aggtgctggg ggcttccgag acaatcgcga 5100
   acatctacac cacacaacac cgcctcgacc agggtgagat atcggccggg gacgcggcgg 5160
   tggtaattac aagcgagatc cccgggaatt caggacctca ccatgggatg gagctgtatc 5220
   atcctcttct tggtagcaac agctacaggt gtccactccg aggtccaact ggtggagagc 5280
   ggtggaggtg ttgtgcaacc tggccggtcc ctgcgcctgt cctgctccgc atctggcttc 5340
   gatttcacca catattggat gagttgggtg agacaggcac ctggaaaagg tcttgagtgg 5400
   attggagaaa ttcatccaga tagcagtacg attaactatg cgccgtctct aaaggataga 5460
   tttacaatat cgcgagacaa cgccaagaac acattgttcc tgcaaatgga cagcctgaga 5520
   cccgaagaca ccggggtcta tttttgtgca agcctttact tcggcttccc ctggtttgct 5580
   tattggggcc aagggacccc ggtcaccgtc tcctcagcct ccaccaaggg cccatcggtc 5640
   ttccccctgg caccctcctc caagagcacc tctgggggca cagcggccct gggctgcctg 5700
   gtcaaggact acttccccga accggtgacg gtgtcgtgga actcaggcgc cctgaccagc 5760
   ggcgtgcaca ccttcccggc tgtcctacag tcctcaggac tctactccct cagcagcgtg 5820
   gtgaccgtgc cctccagcag cttgggcacc cagacctaca tctgcaacgt gaatcacaag 5880
   cccagcaaca ccaaggtgga caagagagtt gagcccaaat cttgtgacaa aactcacaca 5940
   tgcccaccgt gcccagcacc tgaactcctg gggggaccgt cagtcttcct cttcccccca 6000
   aaacccaagg acaccctcat gatctcccgg acccctgagg tcacatgcgt ggtggtggac 6060
   gtgagccacg aagaccctga ggtcaagttc aactggtacg tggacggcgt ggaggtgcat 6120
   aatgccaaga caaagccgcg ggaggagcag tacaacagca cgtaccgtgt ggtcagcgtc 6180
   ctcaccgtcc tgcaccagga ctggctgaat ggcaaggagt acaagtgcaa ggtctccaac 6240
   aaagccctcc cagcccccat cgagaaaacc atctccaaag ccaaagggca gccccgagaa 6300
   ccacaggtgt acaccctgcc cccatcccgg gaggagatga ccaagaacca ggtcagcctg 6360
   acctgcctgg tcaaaggctt ctatcccagc gacatcgccg tggagtggga gagcaatggg 6420
   cagccggaga acaactacaa gaccacgcct cccgtgctgg actccgacgg ctccttcttc 6480
   ctctatagca agctcaccgt ggacaagagc aggtggcagc aggggaacgt cttctcatgc 6540
   tccgtgatgc acgaggctct gcacaaccac tacacgcaga agagcctctc cctgtctccc 6600
   gggaaatgaa agccgaattc gcccctctcc ctcccccccc cctaacgtta ctggccgaag 6660
   ccgcttggaa taaggccggt gtgcgtttgt ctatatgtta ttttccacca tattgccgtc 6720
   ttttggcaat gtgagggccc ggaaacctgg ccctgtcttc ttgacgagca ttcctagggg 6780
   tctttcccct ctcgccaaag gaatgcaagg tctgttgaat gtcgtgaagg aagcagttcc 6840
   tctggaagct tcttgaagac aaacaacgtc tgtagcgacc ctttgcaggc agcggaaccc 6900
   cccacctggc gacaggtgcc tctgcggcca aaagccacgt gtataagata cacctgcaaa 6960
   ggcggcacaa ccccagtgcc acgttgtgag ttggatagtt gtggaaagag tcaaatggct 7020
   ctcctcaagc gtattcaaca aggggctgaa ggatgcccag aaggtacccc attgtatggg 7080
   atctgatctg gggcctcggt gcacatgctt tacatgtgtt tagtcgaggt taaaaaaacg 7140
   tctaggcccc ccgaaccacg gggacgtggt tttcctttga aaaacacgat gataatatgg 7200
   cctcctttgt ctctctgctc ctggtaggca tcctattcca tgccacccag gccgacatcc 7260
   agctgaccca gagcccaagc agcctgagcg ccagcgtggg tgacagagtg accatcacct 7320
   gtaaggccag tcaggatgtg ggtacttctg tagcctggta ccagcagaag ccaggtaagg 7380
   ctccaaagct gctgatctac tggacatcca cccggcacac tggtgtgcca agcagattca 7440
   gcggtagcgg tagcggtacc gacttcacct tcaccatcag cagcctccag ccagaggaca 7500
   tcgccaccta ctactgccag caatatagcc tctatcggtc gttcggccaa gggaccaagg 7560
   tggaaatcaa acgaactgtg gctgcaccat ctgtcttcat cttcccgcca tctgatgagc 7620
   agttgaaatc tggaactgcc tctgttgtgt gcctgctgaa taacttctat cccagagagg 7680
   ccaaagtaca gtggaaggtg gataacgccc tccaatcggg taactcccag gagagtgtca 7740
   cagagcagga cagcaaggac agcacctaca gcctcagcag caccctgacg ctgagcaaag 7800
   cagactacga gaaacacaaa gtctacgcct gcgaagtcac ccatcagggc ctgagctcgc 7860
   ccgtcacaaa gagcttcaac aggggagagt gttagagatc ccccgggctg caggaattcg 7920
   atatcaagct tatcgataat caacctctgg attacaaaat ttgtgaaaga ttgactggta 7980
   ttcttaacta tgttgctcct tttacgctat gtggatacgc tgctttaatg cctttgtatc 8040
   atgctattgc ttcccgtatg gctttcattt tctcctcctt gtataaatcc tggttgctgt 8100
   ctctttatga ggagttgtgg cccgttgtca ggcaacgtgg cgtggtgtgc actgtgtttg 8160
   ctgacgcaac ccccactggt tggggcattg ccaccacctg tcagctcctt tccgggactt 8220
   tcgctttccc cctccctatt gccacggcgg aactcatcgc cgcctgcctt gcccgctgct 8280
   ggacaggggc tcggctgttg ggcactgaca attccgtggt gttgtcgggg aaatcatcgt 8340
   cctttccttg gctgctcgcc tgtgttgcca cctggattct gcgcgggacg tccttctgct 8400
   acgtcccttc ggccctcaat ccagcggacc ttccttcccg cggcctgctg ccggctctgc 8460
   ggcctcttcc gcgtcttcgc cttcgccctc agacgagtcg gatctccctt tgggccgcct 8520
   ccccgcctga tcgataccgt caacatcgat aaaataaaag attttattta gtctccagaa 8580
   aaagggggga atgaaagacc ccacctgtag gtttggcaag ctagcttaag taacgccatt 8640
   ttgcaaggca tggaaaaata cataactgag aatagagaag ttcagatcaa ggtcaggaac 8700
   agatggaaca gctgaatatg ggccaaacag gatatctgtg gtaagcagtt cctgccccgg 8760
   ctcagggcca agaacagatg gaacagctga atatgggcca aacaggatat ctgtggtaag 8820
   cagttcctgc cccggctcag ggccaagaac agatggtccc cagatgcggt ccagccctca 8880
   gcagtttcta gagaaccatc agatgtttcc agggtgcccc aaggacctga aatgaccctg 8940
   tgccttattt gaactaacca atcagttcgc ttctcgcttc tgttcgcgcg cttctgctcc 9000
   ccgagctcaa taaaagagcc cacaacccct cactcggggc gccagtcctc cgattgactg 9060
   agtcgcccgg gtacccgtgt atccaataaa ccctcttgca gttgcatccg acttgtggtc 9120
   tcgctgttcc ttgggagggt ctcctctgag tgattgacta cccgtcagcg ggggtctttc 9180
   att 9183
<210> 8
   <211> 5711
   <212> DNA
   <213> Artificial
<220>
   <223> Synthetic
<400> 8
   gatcagtcct gggtggtcat tgaaaggact gatgctgaag ttgaagctcc aatactttgg 60
   ccacctgatg cgaagaactg actcatgtga taagaccctg atactgggaa agattgaagg 120
   caggaggaga agggatgaca gaggatggaa gagttggatg gaatcaccaa ctcgatggac 180
   atgagtttga gcaagcttcc aggagttggt aatgggcagg gaagcctggc gtgctgcagt 240
   ccatggggtt gcaaagagtt ggacactact gagtgactga actgaactga tagtgtaatc 300
   catggtacag aatataggat aaaaaagagg aagagtttgc cctgattctg aagagttgta 360
   ggatataaaa gtttagaata cctttagttt ggaagtctta aattatttac ttaggatggg 420
   tacccactgc aatataagaa atcaggcttt agagactgat gtagagagaa tgagccctgg 480
   cataccagaa gctaacagct attggttata gctgttataa ccaatatata accaatatat 540
   tggttatata gcatgaagct tgatgccagc aatttgaagg aaccatttag aactagtatc 600
   ctaaactcta catgttccag gacactgatc ttaaagctca ggttcagaat cttgttttat 660
   aggctctagg tgtatattgt ggggcttccc tggtggctca gatggtaaag tgtctgcctg 720
   caatgtgggt gatctgggtt cgatccctgg cttgggaaga tcccctggag aaggaaatgg 780
   caacccactc tagtactctt acctggaaaa ttccatggac agaggagcct tgtaagctac 840
   agtccatggg attgcaaaga gttgaacaca actgagcaac taagcacagc acagtacagt 900
   atacacctgt gaggtgaagt gaagtgaagg ttcaatgcag ggtctcctgc attgcagaaa 960
   gattctttac catctgagcc accagggaag cccaagaata ctggagtggg tagcctattc 1020
   cttctccagg ggatcttccc atcccaggaa ttgaactgga gtctcctgca tttcaggtgg 1080
   attcttcacc agctgaacta ccaggtggat actactccaa tattaaagtg cttaaagtcc 1140
   agttttccca cctttcccaa aaaggttggg tcactctttt ttaaccttct gtggcctact 1200
   ctgaggctgt ctacaagctt atatatttat gaacacattt attgcaagtt gttagtttta 1260
   gatttacaat gtggtatctg gctatttagt ggtattggtg gttggggatg gggaggctga 1320
   tagcatctca gagggcagct agatactgtc atacacactt ttcaagttct ccatttttgt 1380
   gaaatagaaa gtctctggat ctaagttata tgtgattctc agtctctgtg gtcatattct 1440
   attctactcc tgaccactca acaaggaacc aagatatcaa gggacacttg ttttgtttca 1500
   tgcctgggtt gagtgggcca tgacatatgt tctgggcctt gttacatggc tggattggtt 1560
   ggacaagtgc cagctctgat cctgggactg tggcatgtga tgacatacac cccctctcca 1620
   cattctgcat gtctctaggg gggaaggggg aagctcggta tagaaccttt attgtatttt 1680
   ctgattgcct cacttcttat attgccccca tgcccttctt tgttcctcaa gtaaccagag 1740
   acagtgcttc ccagaaccaa ccctacaaga aacaaagggc taaacaaagc caaatgggaa 1800
   gcaggatcat ggtttgaact ctttctggcc agagaacaat acctgctatg gactagatac 1860
   tgggagaggg aaaggaaaag tagggtgaat tatggaagga agctggcagg ctcagcgttt 1920
   ctgtcttggc atgaccagtc tctcttcatt ctcttcctag atgtagggct tggtaccaga 1980
   gcccctgagg ctttctgcat gaatataaat atatgaaact gagtgatgct tccatttcag 2040
   gttcttgggg gcgccgaatt cgagctcggt acccggggat ctcgacggat ccgattactt 2100
   actggcaggt gctgggggct tccgagacaa tcgcgaacat ctacaccaca caacaccgcc 2160
   tcgaccaggg tgagatatcg gccggggacg cggcggtggt aattacaagc gagatccgat 2220
   tacttactgg caggtgctgg gggcttccga gacaatcgcg aacatctaca ccacacaaca 2280
   ccgcctcgac cagggtgaga tatcggccgg ggacgcggcg gtggtaatta caagcgagat 2340
   ctcgagaagc ttgttgggaa ttcaggccat cgatcccgcc gccaccatgg aatggagctg 2400
   ggtctttctc ttcttcctgt cagtaactac aggtgtccac tccgacatcc agatgaccca 2460
   gtctccagcc tccctatctg catctgtggg agaaactgtc actatcacat gtcgagcaag 2520
   tgggaatatt cacaattatt tagcatggta tcagcagaaa cagggaaaat ctcctcagct 2580
   cctggtctat aatgcaaaaa ccttagcaga tggtgtgcca tcaaggttca gtggcagtgg 2640
   atcaggaaca caatattctc tcaagatcaa cagcctgcag cctgaagatt ttgggagtta 2700
   ttactgtcaa catttttgga gtactccgtg gacgttcggt ggaggcacca agctggaaat 2760
   caaacgggct gatgctgcac caactgtatc catcttccca ccatccagtg agcagttaac 2820
   atctggaggt gcctcagtcg tgtgcttctt gaacaacttc taccccaaag acatcaatgt 2880
   caagtggaag attgatggca gtgaacgaca aaatggcgtc ctgaacagtt ggactgatca 2940
   ggacagcaaa gacagcacct acagcatgag cagcaccctc acattgacca aggacgagta 3000
   tgaacgacat aacagctata cctgtgaggc cactcacaag acatcaactt cacccattgt 3060
   caagagcttc aacaggaatg agtgttgaaa gcatcgattt cccctgaatt cgcccctctc 3120
   cctccccccc ccctaacgtt actggccgaa gccgcttgga ataaggccgg tgtgcgtttg 3180
   tctatatgtt attttccacc atattgccgt cttttggcaa tgtgagggcc cggaaacctg 3240
   gccctgtctt cttgacgagc attcctaggg gtctttcccc tctcgccaaa ggaatgcaag 3300
   gtctgttgaa tgtcgtgaag gaagcagttc ctctggaagc ttcttgaaga caaacaacgt 3360
   ctgtagcgac cctttgcagg cagcggaacc ccccacctgg cgacaggtgc ctctgcggcc 3420
   aaaagccacg tgtataagat acacctgcaa aggcggcaca accccagtgc cacgttgtga 3480
   gttggatagt tgtggaaaga gtcaaatggc tctcctcaag cgtattcaac aaggggctga 3540
   aggatgccca gaaggtaccc cattgtatgg gatctgatct ggggcctcgg tgcacatgct 3600
   ttacatgtgt ttagtcgagg ttaaaaaaac gtctaggccc cccgaaccac ggggacgtgg 3660
   ttttcctttg aaaaacacga tgataatatg gcctcctttg tctctctgct cctggtaggc 3720
   atcctattcc atgccaccca ggccgaggtt cagcttcagc agtctggggc agagcttgtg 3780
   aagccagggg cctcagtcaa gttgtcctgc acagcttctg gcttcaacat taaagacacc 3840
   tttatgcact gggtgaagca gaggcctgaa cagggcctgg agtggattgg aaggattgat 3900
   cctgcgaatg ggaatactga atatgacccg aagttccagg gcaaggccac tataacagca 3960
   gacacatcct ccaacacagt caacctgcag ctcagcagcc tgacatctga ggacactgcc 4020
   gtctattact gtgctagtgg aggggaactg gggtttcctt actggggcca agggactctg 4080
   gtcactgtct ctgcagccaa aacgacaccc ccatctgtct atccactggc ccctggatct 4140
   gctgcccaaa ctaactccat ggtgaccctg ggatgcctgg tcaagggcta tttccctgag 4200
   ccagtgacag tgacctggaa ctctggatcc ctgtccagcg gtgtgcacac cttcccagct 4260
   gtcctgcagt ttgacctcta cactctgagc agctcagtga ctgtcccctc cagcacctgg 4320
   cccagcgaga ccgtcacctg caacgttgcc cacccggcca gcagcaccaa ggtggacaag 4380
   aaaattgtgc ccagggattg tactagtgga ggtggaggta gccaccatca ccatcaccat 4440
   taatctagag ttaagcggcc gtcgagatct cgacatcgat aatcaacctc tggattacaa 4500
   aatttgtgaa agattgactg gtattcttaa ctatgttgct ccttttacgc tatgtggata 4560
   cgctgcttta atgcctttgt atcatgctat tgcttcccgt atggctttca ttttctcctc 4620
   cttgtataaa tcctggttgc tgtctcttta tgaggagttg tggcccgttg tcaggcaacg 4680
   tggcgtggtg tgcactgtgt ttgctgacgc aacccccact ggttggggca ttgccaccac 4740
   ctgtcagctc ctttccggga ctttcgcttt ccccctccct attgccacgg cggaactcat 4800
   cgccgcctgc cttgcccgct gctggacagg ggctcggctg ttgggcactg acaattccgt 4860
   ggtgttgtcg gggaaatcat cgtcctttcc ttggctgctc gcctgtgttg ccacctggat 4920
   tctgcgcggg acgtccttct gctacgtccc ttcggccctc aatccagcgg accttccttc 4980
   ccgcggcctg ctgccggctc tgcggcctct tccgcgtctt cgccttcgcc ctcagacgag 5040
   tcggatctcc ctttgggccg cctccccgcc tgatcgataa aataaaagat tttatttagt 5100
   ctccagaaaa aggggggaat gaaagacccc acctgtaggt ttggcaagct agcttaagta 5160
   acgccatttt gcaaggcatg gaaaaataca taactgagaa tagagaagtt cagatcaagg 5220
   tcaggaacag atggaacagc tgaatatggg ccaaacagga tatctgtggt aagcagttcc 5280
   tgccccggct cagggccaag aacagatgga acagctgaat atgggccaaa caggatatct 5340
   gtggtaagca gttcctgccc cggctcaggg ccaagaacag atggtcccca gatgcggtcc 5400
   agccctcagc agtttctaga gaaccatcag atgtttccag ggtgccccaa ggacctgaaa 5460
   tgaccctgtg ccttatttga actaaccaat cagttcgctt ctcgcttctg ttcgcgcgct 5520
   tctgctcccc gagctcaata aaagagccca caacccctca ctcggggcgc cagtcctccg 5580
   attgactgag tcgcccgggt acccgtgtat ccaataaacc ctcttgcagt tgcatccgac 5640
   ttgtggtctc gctgttcctt gggagggtct cctctgagtg attgactacc cgtcagcggg 5700
   ggtctttcat t 5711
<210> 9
   <211> 5130
   <212> DNA
   <213> Artificial
<220>
   <223> Synthetic
<400> 9
   tttgaaagac cccacccgta ggtggcaagc tagcttaagt aacgccactt tgcaaggcat 60
   ggaaaaatac ataactgaga atagaaaagt tcagatcaag gtcaggaaca aagaaacagc 120
   tgaataccaa acaggatatc tgtggtaagc ggttcctgcc ccggctcagg gccaagaaca 180
   gatgagacag ctgagtgatg ggccaaacag gatatctgtg gtaagcagtt cctgccccgg 240
   ctcggggcca agaacagatg gtccccagat gcggtccagc cctcagcagt ttctagtgaa 300
   tcatcagatg tttccagggt gccccaagga cctgaaaatg accctgtacc ttatttgaac 360
   taaccaatca gttcgcttct cgcttctgtt cgcgcgcttc cgctctccga gctcaataaa 420
   agagcccaca acccctcact cggcgcgcca gtcttccgat agactgcgtc gcccgggtac 480
   ccgtattccc aataaagcct cttgctgttt gcatccgaat cgtggtctcg ctgttccttg 540
   ggagggtctc ctctgagtga ttgactaccc acgacggggg tctttcattt gggggctcgt 600
   ccgggatttg gagacccctg cccagggacc accgacccac caccgggagg taagctggcc 660
   agcaacttat ctgtgtctgt ccgattgtct agtgtctatg tttgatgtta tgcgcctgcg 720
   tctgtactag ttagctaact agctctgtat ctggcggacc cgtggtggaa ctgacgagtt 780
   ctgaacaccc ggccgcaacc ctgggagacg tcccagggac tttgggggcc gtttttgtgg 840
   cccgacctga ggaagggagt cgatgtggaa tccgaccccg tcaggatatg tggttctggt 900
   aggagacgag aacctaaaac agttcccgcc tccgtctgaa tttttgcttt cggtttggaa 960
   ccgaagccgc gcgtcttgtc tgctgcagcc aagcttgggc tgcaggtcga ggactgggga 1020
   ccctgcaccg aacatggaga acacaacatc aggattccta ggacccctgc tcgtgttaca 1080
   ggcggggttt ttcttgttga caagaatcct cacaatacca cagagtctag actcgtggtg 1140
   gacttctctc aattttctag ggggagcacc cacgtgtcct ggccaaaatt cgcagtcccc 1200
   aacctccaat cactcaccaa cctcttgtcc tccaatttgt cctggctatc gctggatgtg 1260
   tctgcggcgt tttatcatat tcctcttcat cctgctgcta tgcctcatct tcttgttggt 1320
   tcttctggac taccaaggta tgttgcccgt ttgtcctcta cttccaggaa catcaactac 1380
   cagcacggga ccatgcaaga cctgcacgat tcctgctcaa ggaacctcta tgtttccctc 1440
   ttgttgctgt acaaaacctt cggacggaaa ctgcacttgt attcccatcc catcatcctg 1500
   ggctttcgca agattcctat gggagtgggc ctcagtccgt ttctcctggc tcagtttact 1560
   agtgccattt gttcagtggt tcgtagggct ttcccccact gtttggcttt cagttatatg 1620
   gatgatgtgg tattgggggc caagtctgta caacatcttg agtccctttt tacctctatt 1680
   accaattttc ttttgtcttt gggtatacat ttaaacccta ataaaaccaa acgttggggc 1740
   tactccctta acttcatggg atatgtaatt ggatgttggg gtactttacc gcaagaacat 1800
   attgtactaa aaatcaagca atgttttcga aaactgcctg taaatagacc tattgattgg 1860
   aaagtatgtc agagacttgt gggtcttttg ggctttgctg ccccttttac acaatgtggc 1920
   tatcctgcct taatgccttt atatgcatgt atacaatcta agcaggcttt cactttctcg 1980
   ccaacttaca aggcctttct gtgtaaacaa tatctgaacc tttaccccgt tgcccggcaa 2040
   cggtcaggtc tctgccaagt gtttgctgac gcaaccccca ctggatgggg cttggctatc 2100
   ggccatagcc gcatgcgcgg acctttgtgg ctcctctgcc gatccatact gcggaactcc 2160
   tagcagcttg ttttgctcgc aggcggtctg gagcgaaact tatcggcacc gacaactctg 2220
   ttgtcctctc tcggaaatac acctcctttc catggctgct agggtgtgct gccaactgga 2280
   tcccctcagg atatagtagt ttcgcttttg catagggagg gggaaatgta gtcttatgca 2340
   atacacttgt agtcttgcaa catggtaacg atgagttagc aacatgcctt acaaggagag 2400
   aaaaagcacc gtgcatgccg attggtggaa gtaaggtggt acgatcgtgc cttattagga 2460
   aggcaacaga caggtctgac atggattgga cgaaccactg aattccgcat tgcagagata 2520
   attgtattta agtgcctagc tcgatacagc aaacgccatt tttgaccatt caccacattg 2580
   gtgtgcacct tccaaagctt cacgctgccg caagcactca gggcgcaagg gctgctaaag 2640
   gaagcggaac acgtagaaag ccagtccgca gaaacggtgc tgaccccgga tgaatgtcag 2700
   ctactgggct atctggacaa gggaaaacgc aagcgcaaag agaaagcagg tagcttgcag 2760
   tgggcttaca tggcgatagc tagactgggc ggttttatgg acagcaagcg aaccggaatt 2820
   gccagctggg gcgccctctg gtaaggttgg gaagccctgc aaagtaaact ggatggcttt 2880
   cttgccgcca aggatctgat ggcgcagggg atcaagatct gatcaagaga caggatgagg 2940
   atcgtttcgc atgattgaac aagatggatt gcacgcaggt tctccggccg cttgggtgga 3000
   gaggctattc ggctatgact gggcacaaca gacaatcggc tgctctgatg ccgccgtgtt 3060
   ccggctgtca gcgcaggggc gcccggttct ttttgtcaag accgacctgt ccggtgccct 3120
   gaatgaactg caggacgagg cagcgcggct atcgtggctg gccacgacgg gcgttccttg 3180
   cgcagctgtg ctcgacgttg tcactgaagc gggaagggac tggctgctat tgggcgaagt 3240
   gccggggcag gatctcctgt catctcacct tgctcctgcc gagaaagtat ccatcatggc 3300
   tgatgcaatg cggcggctgc atacgcttga tccggctacc tgcccattcg accaccaagc 3360
   gaaacatcgc atcgagcgag cacgtactcg gatggaagcc ggtcttgtcg atcaggatga 3420
   tctggacgaa gagcatcagg ggctcgcgcc agccgaactg ttcgccaggc tcaaggcgcg 3480
   catgcccgac ggcgaggatc tcgtcgtgac ccatggcgat gcctgcttgc cgaatatcat 3540
   ggtggaaaat ggccgctttt ctggattcat cgactgtggc cggctgggtg tggcggaccg 3600
   ctatcaggac atagcgttgg ctacccgtga tattgctgaa gagcttggcg gcgaatgggc 3660
   tgaccgcttc ctcgtgcttt acggtatcgc cgctcccgat tcgcagcgca tcgccttcta 3720
   tcgccttctt gacgagttct tctgagcggg actctggggt tcgaaatgac cgaccaagcg 3780
   acgcccaacc tgccatcacg agatttcgat tccaccgccg ccttctatga aaggttgggc 3840
   ttcggaatcg ttttccggga cgccggctgg atgatcctcc agcgcgggga tctcatgctg 3900
   gagttcttcg cccaccccaa ccctggccct attattgggt ggactaacca tggggggaat 3960
   tgccgctgga ataggaacag ggactactgc tctaatggcc actcagcaat tccagcagct 4020
   ccaagccgca gtacaggatg atctcaggga ggttgaaaaa tcaatctcta acctagaaaa 4080
   gtctctcact tccctgtctg aagttgtcct acagaatcga aggggcctag acttgttatt 4140
   tctaaaagaa ggagggctgt gtgctgctct aaaagaagaa tgttgcttct atgcggacca 4200
   cacaggacta gtgagagaca gcatggccaa attgagagag aggcttaatc agagacagaa 4260
   actgtttgag tcaactcaag gatggtttga gggactgttt aacagatccc cttggtttac 4320
   caccttgata tctaccatta tgggacccct cattgtactc ctaatgattt tgctcttcgg 4380
   accctgcatt cttaatcgat tagtccaatt tgttaaagac aggatatcag tggtccaggc 4440
   tctagttttg actcaacaat atcaccagct gaagcctata gagtacgagc catagataaa 4500
   ataaaagatt ttatttagtc tccagaaaaa ggggggaatg aaagacccca cctgtaggtt 4560
   tggcaagcta gcttaagtaa cgccattttg caaggcatgg aaaaatacat aactgagaat 4620
   agagaagttc agatcaaggt caggaacaga tggaacagct gaatatgggc caaacaggat 4680
   atctgtggta agcagttcct gccccggctc agggccaaga acagatggaa cagctgaata 4740
   tgggccaaac aggatatctg tggtaagcag ttcctgcccc ggctcagggc caagaacaga 4800
   tggtccccag atgcggtcca gccctcagca gtttctagag aaccatcaga tgtttccagg 4860
   gtgccccaag gacctgaaat gaccctgtgc cttatttgaa ctaaccaatc agttcgcttc 4920
   tcgcttctgt tcgcgcgctt ctgctccccg agctcaataa aagagcccac aacccctcac 4980
   tcggggcgcc agtcctccga ttgactgagt cgcccgggta cccgtgtatc caataaaccc 5040
   tcttgcagtt gcatccgact tgtggtctcg ctgttccttg ggagggtctc ctctgagtga 5100
   ttgactaccc gtcagcgggg gtctttcatt 5130
<210> 10
   <211> 4661
   <212> DNA
   <213> Artificial
<220>
   <223> Synthetic
<400> 10
   gatcagtcct gggtggtcat tgaaaggact gatgctgaag ttgaagctcc aatactttgg      60
   ccacctgatg cgaagaactg actcatgtga taagaccctg atactgggaa agattgaagg     120
   caggaggaga agggatgaca gaggatggaa gagttggatg gaatcaccaa ctcgatggac     180
   atgagtttga gcaagcttcc aggagttggt aatgggcagg gaagcctggc gtgctgcagt     240
   ccatggggtt gcaaagagtt ggacactact gagtgactga actgaactga tagtgtaatc     300
   catggtacag aatataggat aaaaaagagg aagagtttgc cctgattctg aagagttgta 360
   ggatataaaa gtttagaata cctttagttt ggaagtctta aattatttac ttaggatggg 420
   tacccactgc aatataagaa atcaggcttt agagactgat gtagagagaa tgagccctgg 480
   cataccagaa gctaacagct attggttata gctgttataa ccaatatata accaatatat 540
   tggttatata gcatgaagct tgatgccagc aatttgaagg aaccatttag aactagtatc 600
   ctaaactcta catgttccag gacactgatc ttaaagctca ggttcagaat cttgttttat 660
   aggctctagg tgtatattgt ggggcttccc tggtggctca gatggtaaag tgtctgcctg 720
   caatgtgggt gatctgggtt cgatccctgg cttgggaaga tcccctggag aaggaaatgg 780
   caacccactc tagtactctt acctggaaaa ttccatggac agaggagcct tgtaagctac 840
   agtccatggg attgcaaaga gttgaacaca actgagcaac taagcacagc acagtacagt 900
   atacacctgt gaggtgaagt gaagtgaagg ttcaatgcag ggtctcctgc attgcagaaa 960
   gattctttac catctgagcc accagggaag cccaagaata ctggagtggg tagcctattc 1020
   cttctccagg ggatcttccc atcccaggaa ttgaactgga gtctcctgca tttcaggtgg 1080
   attcttcacc agctgaacta ccaggtggat actactccaa tattaaagtg cttaaagtcc 1140
   agttttccca cctttcccaa aaaggttggg tcactctttt ttaaccttct gtggcctact 1200
   ctgaggctgt ctacaagctt atatatttat gaacacattt attgcaagtt gttagtttta 1260
   gatttacaat gtggtatctg gctatttagt ggtattggtg gttggggatg gggaggctga 1320
   tagcatctca gagggcagct agatactgtc atacacactt ttcaagttct ccatttttgt 1380
   gaaatagaaa gtctctggat ctaagttata tgtgattctc agtctctgtg gtcatattct 1440
   attctactcc tgaccactca acaaggaacc aagatatcaa gggacacttg ttttgtttca 1500
   tgcctgggtt gagtgggcca tgacatatgt tctgggcctt gttacatggc tggattggtt 1560
   ggacaagtgc cagctctgat cctgggactg tggcatgtga tgacatacac cccctctcca 1620
   cattctgcat gtctctaggg gggaaggggg aagctcggta tagaaccttt attgtatttt 1680
   ctgattgcct cacttcttat attgccccca tgcccttctt tgttcctcaa gtaaccagag 1740
   acagtgcttc ccagaaccaa ccctacaaga aacaaagggc taaacaaagc caaatgggaa 1800
   gcaggatcat ggtttgaact ctttctggcc agagaacaat acctgctatg gactagatac 1860
   tgggagaggg aaaggaaaag tagggtgaat tatggaagga agctggcagg ctcagcgttt 1920
   ctgtcttggc atgaccagtc tctcttcatt ctcttcctag atgtagggct tggtaccaga 1980
   gcccctgagg ctttctgcat gaatataaat atatgaaact gagtgatgct tccatttcag 2040
   gttcttgggg gcgccgaatt cgagctcggt acccggggat ctcgagaagc tttaaccatg 2100
   gaatggagct gggtctttct cttcttcctg tcagtaacta caggtgtcca ctcccaggtt 2160
   cagttgcagc agtctgacgc tgagttggtg aaacctgggg cttcagtgaa gatttcctgc 2220
   aaggcttctg gctacacctt cactgaccat gcaattcact gggtgaaaca gaaccctgaa 2280
   cagggcctgg aatggattgg atatttttct cccggaaatg atgattttaa atacaatgag 2340
   aggttcaagg gcaaggccac actgactgca gacaaatcct ccagcactgc ctacgtgcag 2400
   ctcaacagcc tgacatctga ggattctgca gtgtatttct gtacaagatc cctgaatatg 2460
   gcctactggg gtcaaggaac ctcagtcacc gtctcctcag gaggcggagg cagcggaggc 2520
   ggtggctcgg gaggcggagg ctcggacatt gtgatgtcac agtctccatc ctccctacct 2580
   gtgtcagttg gcgagaaggt tactttgagc tgcaagtcca gtcagagcct tttatatagt 2640
   ggtaatcaaa agaactactt ggcctggtac cagcagaaac cagggcagtc tcctaaactg 2700
   ctgatttact gggcatccgc tagggaatct ggggtccctg atcgcttcac aggcagtgga 2760
   tctgggacag atttcactct ctccatcagc agtgtgaaga ctgaagacct ggcagtttat 2820
   tactgtcagc agtattatag ctatcccctc acgttcggtg ctgggaccaa gctggtgctg 2880
   aaacgggccg ccgagcccaa atctcctgac aaaactcaca catgcccacc gtgcccagca 2940
   cctgaactcc tggggggacc gtcagtcttc ctcttccccc caaaacccaa ggacaccctc 3000
   atgatctccc ggacccctga ggtcacatgc gtggtggtgg acgtgagcca cgaagaccct 3060
   gaggtcaagt tcaactggta cgtggacggc gtggaggtgc ataatgccaa gacaaagccg 3120
   cgggaggagc agtacaacag cacgtaccgt gtggtcagcg tcctcaccgt cctgcaccag 3180
   gactggctga atggcaagga gtacaagtgc aaggtctcca acaaagccct cccagccccc 3240
   atcgagaaaa ccatctccaa agccaaaggg cagccccgag aaccacaggt gtacaccctg 3300
   cccccatccc gggatgagct gaccaagaac caggtcagcc tgacctgcct ggtcaaaggc 3360
   ttctatccca gcgacatcgc cgtggagtgg gagagcaatg ggcagccgga gaacaactac 3420
   aagaccacgc ctcccgtgct ggactccgac ggctccttct tcctctacag caagctcacc 3480
   gtggacaaga gcaggtggca gcaggggaac gtcttctcat gctccgtgat gcatgaggct 3540
   ctgcacaacc actacacgca gaagagcctc tccctgtctc cgggtaaagg aggcggatca 3600
   ggaggtggcg cacctacttc aagttctaca aagaaaacac agctacaact ggagcattta 3660
   ctgctggatt tacagatgat tttgaatgga attaataatt acaagaatcc caaactcacc 3720
   aggatgctca catttaagtt ttacatgccc aagaaggcca cagaactgaa acatcttcag 3780
   tgtctagaag aagaactcaa acctctggag gaagtgctaa atttagctca aagcaaaaac 3840
   tttcacttaa gacccaggga cttaatcagc aatatcaacg taatagttct ggaactaaag 3900
   ggatctgaaa caacattcat gtgtgaatat gctgatgaga cagcaaccat tgtagaattt 3960
   ctgaacagat ggattacctt ttgtcaaagc atcatctcaa cactaacttg aagcttgtta 4020
   acatcgataa aataaaagat tttatttagt ctccagaaaa aggggggaat gaaagacccc 4080
   acctgtaggt ttggcaagct agcttaagta acgccatttt gcaaggcatg gaaaaataca 4140
   taactgagaa tagagaagtt cagatcaagg tcaggaacag atggaacagc tgaatatggg 4200
   ccaaacagga tatctgtggt aagcagttcc tgccccggct cagggccaag aacagatgga 4260
   acagctgaat atgggccaaa caggatatct gtggtaagca gttcctgccc cggctcaggg 4320
   ccaagaacag atggtcccca gatgcggtcc agccctcagc agtttctaga gaaccatcag 4380
   atgtttccag ggtgccccaa ggacctgaaa tgaccctgtg ccttatttga actaaccaat 4440
   cagttcgctt ctcgcttctg ttcgcgcgct tctgctcccc gagctcaata aaagagccca 4500
   caacccctca ctcggggcgc cagtcctccg attgactgag tcgcccgggt acccgtgtat 4560
   ccaataaacc ctcttgcagt tgcatccgac ttgtggtctc gctgttcctt gggagggtct 4620
   cctctgagtg attgactacc cgtcagcggg ggtctttcat t 4661
<210> 11
   <211> 5691
   <212> DNA
   <213> Artificial
<220>
   <223> Synthetic
<400> 11
   gatcagtcct gggtggtcat tgaaaggact gatgctgaag ttgaagctcc aatactttgg 60
   ccacctgatg cgaagaactg actcatgtga taagaccctg atactgggaa agattgaagg 120
   caggaggaga agggatgaca gaggatggaa gagttggatg gaatcaccaa ctcgatggac 180
   atgagtttga gcaagcttcc aggagttggt aatgggcagg gaagcctggc gtgctgcagt 240
   ccatggggtt gcaaagagtt ggacactact gagtgactga actgaactga tagtgtaatc 300
   catggtacag aatataggat aaaaaagagg aagagtttgc cctgattctg aagagttgta 360
   ggatataaaa gtttagaata cctttagttt ggaagtctta aattatttac ttaggatggg 420
   tacccactgc aatataagaa atcaggcttt agagactgat gtagagagaa tgagccctgg 480
   cataccagaa gctaacagct attggttata gctgttataa ccaatatata accaatatat 540
   tggttatata gcatgaagct tgatgccagc aatttgaagg aaccatttag aactagtatc 600
   ctaaactcta catgttccag gacactgatc ttaaagctca ggttcagaat cttgttttat 660
   aggctctagg tgtatattgt ggggcttccc tggtggctca gatggtaaag tgtctgcctg 720
   caatgtgggt gatctgggtt cgatccctgg cttgggaaga tcccctggag aaggaaatgg 780
   caacccactc tagtactctt acctggaaaa ttccatggac agaggagcct tgtaagctac 840
   agtccatggg attgcaaaga gttgaacaca actgagcaac taagcacagc acagtacagt 900
   atacacctgt gaggtgaagt gaagtgaagg ttcaatgcag ggtctcctgc attgcagaaa 960
   gattctttac catctgagcc accagggaag cccaagaata ctggagtggg tagcctattc 1020
   cttctccagg ggatcttccc atcccaggaa ttgaactgga gtctcctgca tttcaggtgg 1080
   attcttcacc agctgaacta ccaggtggat actactccaa tattaaagtg cttaaagtcc 1140
   agttttccca cctttcccaa aaaggttggg tcactctttt ttaaccttct gtggcctact 1200
   ctgaggctgt ctacaagctt atatatttat gaacacattt attgcaagtt gttagtttta 1260
   gatttacaat gtggtatctg gctatttagt ggtattggtg gttggggatg gggaggctga 1320
   tagcatctca gagggcagct agatactgtc atacacactt ttcaagttct ccatttttgt 1380
   gaaatagaaa gtctctggat ctaagttata tgtgattctc agtctctgtg gtcatattct 1440
   attctactcc tgaccactca acaaggaacc aagatatcaa gggacacttg ttttgtttca 1500
   tgcctgggtt gagtgggcca tgacatatgt tctgggcctt gttacatggc tggattggtt 1560
   ggacaagtgc cagctctgat cctgggactg tggcatgtga tgacatacac cccctctcca 1620
   cattctgcat gtctctaggg gggaaggggg aagctcggta tagaaccttt attgtatttt 1680
   ctgattgcct cacttcttat attgccccca tgcccttctt tgttcctcaa gtaaccagag 1740
   acagtgcttc ccagaaccaa ccctacaaga aacaaagggc taaacaaagc caaatgggaa 1800
   gcaggatcat ggtttgaact ctttctggcc agagaacaat acctgctatg gactagatac 1860
   tgggagaggg aaaggaaaag tagggtgaat tatggaagga agctggcagg ctcagcgttt 1920
   ctgtcttggc atgaccagtc tctcttcatt ctcttcctag atgtagggct tggtaccaga 1980
   gcccctgagg ctttctgcat gaatataaat atatgaaact gagtgatgct tccatttcag 2040
   gttcttgggg gcgccgaatt cgagctcggt acccggggat ctcgacggat ccgattactt 2100
   actggcaggt gctgggggct tccgagacaa tcgcgaacat ctacaccaca caacaccgcc 2160
   tcgaccaggg tgagatatcg gccggggacg cggcggtggt aattacaagc gagatccgat 2220
   tacttactgg caggtgctgg gggcttccga gacaatcgcg aacatctaca ccacacaaca 2280
   ccgcctcgac cagggtgaga tatcggccgg ggacgcggcg gtggtaatta caagcgagat 2340
   ctcgagttaa cagatctagg cctcctaggt cgacggatcc ccgggaattc ggcgccgcca 2400
   ccatgatgtc ctttgtctct ctgctcctgg taggcatcct attccatgcc acccaggccc 2460
   aggtccaact gcagcagtct gggcctgagc tggtgaagcc tgggacttca gtgaggatat 2520
   cctgcaaggc ttctggctac accttcacaa gctactattt acactgggtg aagcagaggc 2580
   ctggacaggg acttgagtgg attgcatgga tttatcctgg aaatgttatt actacgtaca 2640
   atgagaagtt caagggcaag gccacactga ctgcagacaa atcctccagc acagcctaca 2700
   tgcacctcaa cagcctgacc tctgaggact ctgcggtcta tttctgtgca aggggtgacc 2760
   atgatcttga ctactggggc caaggcacca ctctcacagt ctcctcagcc aaaacgacac 2820
   ccccatctgt ctatccactg gcccctggat ctgctgccca aactaactcc atggtgaccc 2880
   tgggatgcct ggtcaagggc tatttccctg agccagtgac agtgacctgg aactctggat 2940
   ccctgtccag cggtgtgcac accttcccag ctgtcctgca gtctgacctc tacactctga 3000
   gcagctcagt gactgtcccc tccagcacct ggcccagcga gaccgtcacc tgcaacgttg 3060
   cccacccggc cagcagcacc aaggtggaca agaaaattgt gcccagggat tgtactagtg 3120
   gaggtggagg tagctaaggg agatctcgac ggatccccgg gaattcgccc ctctccctcc 3180
   ccccccccta acgttactgg ccgaagccgc ttggaataag gccggtgtgc gtttgtctat 3240
   atgttatttt ccaccatatt gccgtctttt ggcaatgtga gggcccggaa acctggccct 3300
   gtcttcttga cgagcattcc taggggtctt tcccctctcg ccaaaggaat gcaaggtctg 3360
   ttgaatgtcg tgaaggaagc agttcctctg gaagcttctt gaagacaaac aacgtctgta 3420
   gcgacccttt gcaggcagcg gaacccccca cctggcgaca ggtgcctctg cggccaaaag 3480
   ccacgtgtat aagatacacc tgcaaaggcg gcacaacccc agtgccacgt tgtgagttgg 3540
   atagttgtgg aaagagtcaa atggctctcc tcaagcgtat tcaacaaggg gctgaaggat 3600
   gcccagaagg taccccattg tatgggatct gatctggggc ctcggtgcac atgctttaca 3660
   tgtgtttagt cgaggttaaa aaaacgtcta ggccccccga accacgggga cgtggttttc 3720
   ctttgaaaaa cacgatgata atatggcctc ctttgtctct ctgctcctgg taggcatcct 3780
   attccatgcc acccaggccg acattgtgct gacacaatct ccagcaatca tgtctgcatc 3840
   tccaggggag aaggtcacca tgacctgcag tgccacctca agtgtaagtt acatacactg 3900
   gtaccagcag aagtcaggca cctcccccaa aagatggatt tatgacacat ccaaactggc 3960
   ttctggagtc cctgctcgct tcagtggcag tgggtctggg acctctcact ctctcacact 4020
   cagcagcatg gaggctgaag atgctgccac ttattactgc cagcagtggg gtagttacct 4080
   cacgttcggt gcggggacca agctggagct gaaacgggct gatgctgcac caactgtatc 4140
   catcttccca ccatccagtg agcagttaac atctggaggt gcctcagtcg tgtgcttctt 4200
   gaacaacttc taccccaaag acatcaatgt caagtggaag attgatggca gtgaacgaca 4260
   aaatggcgtc ctgaacagtt ggactgatca ggacagcaaa gacagcacct acagcatgag 4320
   cagcaccctc acgttgacca aggacgagta tgaacgacat aacagctata cctgtgaggc 4380
   cactcacaag acatcaactt cacccattgt caagagcttc aacaggaatg agtgttaata 4440
   ggggagatct cgacatcgat aatcaacctc tggattacaa aatttgtgaa agattgactg 4500
   gtattcttaa ctatgttgct ccttttacgc tatgtggata cgctgcttta atgcctttgt 4560
   atcatgctat tgcttcccgt atggctttca ttttctcctc cttgtataaa tcctggttgc 4620
   tgtctcttta tgaggagttg tggcccgttg tcaggcaacg tggcgtggtg tgcactgtgt 4680
   ttgctgacgc aacccccact ggttggggca ttgccaccac ctgtcagctc ctttccggga 4740
   ctttcgcttt ccccctccct attgccacgg cggaactcat cgccgcctgc cttgcccgct    4800
   gctggacagg ggctcggctg ttgggcactg acaattccgt ggtgttgtcg gggaaatcat    4860
   cgtcctttcc ttggctgctc gcctgtgttg ccacctggat tctgcgcggg acgtccttct    4920
   gctacgtccc ttcggccctc aatccagcgg accttccttc ccgcggcctg ctgccggctc    4980
   tgcggcctct tccgcgtctt cgccttcgcc ctcagacgag tcggatctcc ctttgggccg    5040
   cctccccgcc tgatcgataa aataaaagat tttatttagt ctccagaaaa aggggggaat    5100
   gaaagacccc acctgtaggt ttggcaagct agcttaagta acgccatttt gcaaggcatg    5160
   gaaaaataca taactgagaa tagagaagtt cagatcaagg tcaggaacag atggaacagc    5220
   tgaatatggg ccaaacagga tatctgtggt aagcagttcc tgccccggct cagggccaag    5280
   aacagatgga acagctgaat atgggccaaa caggatatct gtggtaagca gttcctgccc    5340
   cggctcaggg ccaagaacag atggtcccca gatgcggtcc agccctcagc agtttctaga    5400
   gaaccatcag atgtttccag ggtgccccaa ggacctgaaa tgaccctgtg ccttatttga    5460
   actaaccaat cagttcgctt ctcgcttctg ttcgcgcgct tctgctcccc gagctcaata    5520
   aaagagccca caacccctca ctcggggcgc cagtcctccg attgactgag tcgcccgggt    5580
   acccgtgtat ccaataaacc ctcttgcagt tgcatccgac ttgtggtctc gctgttcctt    5640
   gggagggtct cctctgagtg attgactacc cgtcagcggg ggtctttcat t              5691
<210> 12
   <211> 668
   <212> DNA
   <213> Artificial
<220>
   <223> Synthetic
<400> 12
   ggaattcgcc cctctccctc ccccccccct aacgttactg gccgaagccg cttggaataa      60
   ggccggtgtg cgtttgtcta tatgttattt tccaccatat tgccgtcttt tggcaatgtg     120
   agggcccgga aacctggccc tgtcttcttg acgagcattc ctaggggtct ttcccctctc     180
   gccaaaggaa tgcaaggtct gttgaatgtc gtgaaggaag cagttcctct ggaagcttct     240
   tgaagacaaa caacgtctgt agcgaccctt tgcaggcagc ggaacccccc acctggcgac     300
   aggtgcctct gcggccaaaa gccacgtgta taagatacac ctgcaaaggc ggcacaaccc     360
   cagtgccacg ttgtgagttg gatagttgtg gaaagagtca aatggctctc ctcaagcgta     420
   ttcaacaagg ggctgaagga tgcccagaag gtaccccatt gtatgggatc tgatctgggg     480
   cctcggtgca catgctttac atgtgtttag tcgaggttaa aaaaacgtct aggccccccg     540
   aaccacgggg acgtggtttt cctttgaaaa acacgatgat aatatggcct tgctcatcct     600
   tacctgtctt gtggctgttg ctcttgccgg cgccatggga tatctagatc tcgagctcgc     660
   gaaagctt                                                              668
<210> 13
   <211> 6255
   <212> DNA
   <213> Artificial
<220>
   <223> Synthetic
<400> 13
   tttgaaagac cccacccgta ggtggcaagc tagcttaagt aacgccactt tgcaaggcat      60
   ggaaaaatac ataactgaga atagaaaagt tcagatcaag gtcaggaaca aagaaacagc     120
   tgaataccaa acaggatatc tgtggtaagc ggttcctgcc ccggctcagg gccaagaaca     180
   gatgagacag ctgagtgatg ggccaaacag gatatctgtg gtaagcagtt cctgccccgg     240
   ctcggggcca agaacagatg gtccccagat gcggtccagc cctcagcagt ttctagtgaa     300
   tcatcagatg tttccagggt gccccaagga cctgaaaatg accctgtacc ttatttgaac      360
   taaccaatca gttcgcttct cgcttctgtt cgcgcgcttc cgctctccga gctcaataaa     420
   agagcccaca acccctcact cggcgcgcca gtcttccgat agactgcgtc gcccgggtac     480
   ccgtattccc aataaagcct cttgctgttt gcatccgaat cgtggtctcg ctgttccttg     540
   ggagggtctc ctctgagtga ttgactaccc acgacggggg tctttcattt gggggctcgt     600
   ccgggatttg gagacccctg cccagggacc accgacccac caccgggagg taagctggcc     660
   agcaacttat ctgtgtctgt ccgattgtct agtgtctatg tttgatgtta tgcgcctgcg     720
   tctgtactag ttagctaact agctctgtat ctggcggacc cgtggtggaa ctgacgagtt     780
   ctgaacaccc ggccgcaacc ctgggagacg tcccagggac tttgggggcc gtttttgtgg     840
   cccgacctga ggaagggagt cgatgtggaa tccgaccccg tcaggatatg tggttctggt     900
   aggagacgag aacctaaaac agttcccgcc tccgtctgaa tttttgcttt cggtttggaa     960
   ccgaagccgc gcgtcttgtc tgctgcagcg ctgcagcatc gttctgtgtt gtctctgtct    1020
   gactgtgttt ctgtatttgt ctgaaaatta gggccagact gttaccactc ccttaagttt    1080
   gaccttaggt cactggaaag atgtcgagcg gatcgctcac aaccagtcgg tagatgtcaa    1140
   gaagagacgt tgggttacct tctgctctgc agaatggcca acctttaacg tcggatggcc    1200
   gcgagacggc acctttaacc gagacctcat cacccaggtt aagatcaagg tcttttcacc    1260
   tggcccgcat ggacacccag accaggtccc ctacatcgtg acctgggaag ccttggcttt    1320
   tgacccccct ccctgggtca agccctttgt acaccctaag cctccgcctc ctcttcctcc     1380
   atccgccccg tctctccccc ttgaacctcc tcgttcgacc ccgcctcgat cctcccttta    1440
   tccagccctc actccttctc taggcgccgg aattccgatc tgatcaagag acaggatgag    1500
   gatcgtttcg catgattgaa caagatggat tgcacgcagg ttctccggcc gcttgggtgg 1560
   agaggctatt cggctatgac tgggcacaac agacaatcgg ctgctctgat gccgccgtgt 1620
   tccggctgtc agcgcagggg cgcccggttc tttttgtcaa gaccgacctg tccggtgccc 1680
   tgaatgaact gcaggacgag gcagcgcggc tatcgtggct ggccacgacg ggcgttcctt 1740
   gcgcagctgt gctcgacgtt gtcactgaag cgggaaggga ctggctgcta ttgggcgaag 1800
   tgccggggca ggatctcctg tcatctcacc ttgctcctgc cgagaaagta tccatcatgg 1860
   ctgatgcaat gcggcggctg catacgcttg atccggctac ctgcccattc gaccaccaag 1920
   cgaaacatcg catcgagcga gcacgtactc ggatggaagc cggtcttgtc gatcaggatg 1980
   atctggacga agagcatcag gggctcgcgc cagccgaact gttcgccagg ctcaaggcgc 2040
   gcatgcccga cggcgaggat ctcgtcgtga cccatggcga tgcctgcttg ccgaatatca 2100
   tggtggaaaa tggccgcttt tctggattca tcgactgtgg ccggctgggt gtggcggacc 2160
   gctatcagga catagcgttg gctacccgtg atattgctga agagcttggc ggcgaatggg 2220
   ctgaccgctt cctcgtgctt tacggtatcg ccgctcccga ttcgcagcgc atcgccttct 2280
   atcgccttct tgacgagttc ttctgagcgg gactctgggg ttcgaaatga ccgaccaagc 2340
   gacgcccaac ctgccatcac gagatttcga ttccaccgcc gccttctatg aaaggttggg 2400
   cttcggaatc gttttccggg acgccggctg gatgatcctc cagcgcgggg atctcatgct 2460
   ggagttcttc gcccaccccg ggctcgatcc cctcgcgagt tggttcagct gctgcctgag 2520
   gctggacgac ctcgcggagt tctaccggca gtgcaaatcc gtcggcatcc aggaaaccag 2580
   cagcggctat ccgcgcatcc atgcccccga actgcaggag tggggaggca cgatggccgc 2640
   tttggtcgag gcggatccgg ccattagcca tattattcat tggttatata gcataaatca 2700
   atattggcta ttggccattg catacgttgt atccatatca taatatgtac atttatattg 2760
   gctcatgtcc aacattaccg ccatgttgac attgattatt gactagttat taatagtaat 2820
   caattacggg gtcattagtt catagcccat atatggagtt ccgcgttaca taacttacgg 2880
   taaatggccc gcctggctga ccgcccaacg acccccgccc attgacgtca ataatgacgt 2940
   atgttcccat agtaacgcca atagggactt tccattgacg tcaatgggtg gagtatttac 3000
   ggtaaactgc ccacttggca gtacatcaag tgtatcatat gccaagtacg ccccctattg 3060
   acgtcaatga cggtaaatgg cccgcctggc attatgccca gtacatgacc ttatgggact 3120
   ttcctacttg gcagtacatc tacgtattag tcatcgctat taccatggtg atgcggtttt 3180
   ggcagtacat caatgggcgt ggatagcggt ttgactcacg gggatttcca agtctccacc 3240
   ccattgacgt caatgggagt ttgttttggc accaaaatca acgggacttt ccaaaatgtc 3300
   gtaacaactc cgccccattg acgcaaatgg gcggtaggca tgtacggtgg gaggtctata 3360
   taagcagagc tcgtttagtg aaccgtcaga tcgcctggag acgccatcca cgctgttttg 3420
   acctccatag aagacaccgg gaccgatcca gcctccgcgg ccccaagctt ctcgacggat 3480
   ccccgggaat tcaggccatc gatcccgccg ccaccatgga atggagctgg gtctttctct 3540
   tcttcctgtc agtaactaca ggtgtccact ccgacatcca gatgacccag tctccagcct 3600
   ccctatctgc atctgtggga gaaactgtca ctatcacatg tcgagcaagt gggaatattc 3660
   acaattattt agcatggtat cagcagaaac agggaaaatc tcctcagctc ctggtctata 3720
   atgcaaaaac cttagcagat ggtgtgccat caaggttcag tggcagtgga tcaggaacac 3780
   aatattctct caagatcaac agcctgcagc ctgaagattt tgggagttat tactgtcaac 3840
   atttttggag tactccgtgg acgttcggtg gaggcaccaa gctggaaatc aaacgggctg 3900
   atgctgcacc aactgtatcc atcttcccac catccagtga gcagttaaca tctggaggtg 3960
   cctcagtcgt gtgcttcttg aacaacttct accccaaaga catcaatgtc aagtggaaga 4020
   ttgatggcag tgaacgacaa aatggcgtcc tgaacagttg gactgatcag gacagcaaag 4080
   acagcaccta cagcatgagc agcaccctca cattgaccaa ggacgagtat gaacgacata 4140
   acagctatac ctgtgaggcc actcacaaga catcaacttc acccattgtc aagagcttca 4200
   acaggaatga gtgttgaaag catcgatttc ccctgaattc gcccctctcc ctcccccccc 4260
   cctaacgtta ctggccgaag ccgcttggaa taaggccggt gtgcgtttgt ctatatgtta 4320
   ttttccacca tattgccgtc ttttggcaat gtgagggccc ggaaacctgg ccctgtcttc 4380
   ttgacgagca ttcctagggg tctttcccct ctcgccaaag gaatgcaagg tctgttgaat 4440
   gtcgtgaagg aagcagttcc tctggaagct tcttgaagac aaacaacgtc tgtagcgacc 4500
   ctttgcaggc agcggaaccc cccacctggc gacaggtgcc tctgcggcca aaagccacgt 4560
   gtataagata cacctgcaaa ggcggcacaa ccccagtgcc acgttgtgag ttggatagtt 4620
   gtggaaagag tcaaatggct ctcctcaagc gtattcaaca aggggctgaa ggatgcccag 4680
   aaggtacccc attgtatggg atctgatctg gggcctcggt gcacatgctt tacatgtgtt 4740
   tagtcgaggt taaaaaaacg tctaggcccc ccgaaccacg gggacgtggt tttcctttga 4800
   aaaacacgat gataatatgg cctcctttgt ctctctgctc ctggtaggca tcctattcca 4860
   tgccacccag gccgaggttc agcttcagca gtctggggca gagcttgtga agccaggggc 4920
   ctcagtcaag ttgtcctgca cagcttctgg cttcaacatt aaagacacct ttatgcactg 4980
   ggtgaagcag aggcctgaac agggcctgga gtggattgga aggattgatc ctgcgaatgg 5040
   gaatactgaa tatgacccga agttccaggg caaggccact ataacagcag acacatcctc 5100
   caacacagtc aacctgcagc tcagcagcct gacatctgag gacactgccg tctattactg 5160
   tgctagtgga ggggaactgg ggtttcctta ctggggccaa gggactctgg tcactgtctc 5220
   tgcagccaaa acgacacccc catctgtcta tccactggcc cctggatctg ctgcccaaac 5280
   taactccatg gtgaccctgg gatgcctggt caagggctat ttccctgagc cagtgacagt 5340
   gacctggaac tctggatccc tgtccagcgg tgtgcacacc ttcccagctg tcctgcagtc 5400
   tgacctctac actctgagca gctcagtgac tgtcccctcc agcacctggc ccagcgagac 5460
   cgtcacctgc aacgttgccc acccggccag cagcaccaag gtggacaaga aaattgtgcc 5520
   cagggattgt actagtggag gtggaggtag ccaccatcac catcaccatt aatctagagt 5580
   taagcggccg tcgagatcta ggcctcctag gtcgacatcg ataaaataaa agattttatt 5640
   tagtctccag aaaaaggggg gaatgaaaga ccccacctgt aggtttggca agctagctta 5700
   agtaacgcca ttttgcaagg catggaaaaa tacataactg agaatagaga agttcagatc 5760
   aaggtcagga acagatggaa cagctgaata tgggccaaac aggatatctg tggtaagcag 5820
   ttcctgcccc ggctcagggc caagaacaga tggaacagct gaatatgggc caaacaggat 5880
   atctgtggta agcagttcct gccccggctc agggccaaga acagatggtc cccagatgcg 5940
   gtccagccct cagcagtttc tagagaacca tcagatgttt ccagggtgcc ccaaggacct 6000
   gaaatgaccc tgtgccttat ttgaactaac caatcagttc gcttctcgct tctgttcgcg 6060
   cgcttctgct ccccgagctc aataaaagag cccacaaccc ctcactcggg gcgccagtcc 6120
   tccgattgac tgagtcgccc gggtacccgt gtatccaata aaccctcttg cagttgcatc 6180
   cgacttgtgg tctcgctgtt ccttgggagg gtctcctctg agtgattgac tacccgtcag 6240
   cgggggtctt tcatt 6255
<210> 14
   <211> 43
   <212> DNA
   <213> Artificial
<220>
   <223> Synthetic
<400> 14
   ctttgaaaaa cacgatgata atatggcctc ctttgtctct ctg 43
<210> 15
   <211> 30
   <212> DNA
   <213> Artificial
<220>
   <223> Synthetic
<400> 15
   ttcgcgagct cgagatctag atatcccatg 30
<210> 16
   <211> 35
   <212> DNA
   <213> Artificial
<220>
   <223> Synthetic
<400> 16
   ctacaggtgt ccacgtcgac atccagctga cccag 35
<210> 17
   <211> 34
   <212> DNA
   <213> Artificial
<220>
   <223> Synthetic
<400> 17
   ctgcagaata gatctctaac actctcccct gttg 34
<210> 18
   <211> 51
   <212> DNA
   <213> Artificial
<220>
   <223> Synthetic
<400> 18
   cagtgtgatc tcgagaattc aggacctcac catgggatgg agctgtatca t 51
<210> 19
   <211> 23
   <212> DNA
   <213> Artificial
<220>
   <223> Synthetic
<400> 19
   aggctgtatt ggtggattcg tct 23
<210> 20
   <211> 41
   <212> DNA
   <213> Artificial
<220>
   <223> Synthetic
<400> 20
   agcttctcga gttaacagat ctaggcctcc taggtcgaca t 41
<210> 21
   <211> 39
   <212> DNA
   <213> Artificial
<220>
   <223> Synthetic
<400> 21
   cgatgtcgac ctaggaggcc tagatctgtt aactcgaga 39
<210> 22
   <211> 64
   <212> DNA
   <213> Artificial
<220>
   <223> Synthetic
<400> 22
   cgaggctctg cacaaccact acacgcagaa gagcctctcc ctgtctcccg ggaaatgaaa 60
   gccg 64
<210> 23
   <211> 72
   <212> DNA
   <213> Artificial
<220>
   <223> Synthetic
<400> 23
   aattcggctt tcatttcccg ggagacaggg agaggctctt ctgcgtgtag tggttgtgca 60
   gagcctcgtg ca 72
<210> 24
   <211> 41
   <212> DNA
   <213> Artificial
<220>
   <223> Synthetic
<400> 24
   aaagcatatg ttctgggcct tgttacatgg ctggattggt t 41
<210> 25
   <211> 54
   <212> DNA
   <213> Artificial
<220>
   <223> Synthetic
<400> 25
   tgaattcggc gcccccaaga acctgaaatg gaagcatcac tcagtttcat atat 54
<210> 26
   <211> 35
   <212> DNA
   <213> Artificial
<220>
   <223> Synthetic
<400> 26
   ctacaggtgt ccacgtcgac atccagctga cccag 35
<210> 27
   <211> 34
   <212> DNA
   <213> Artificial
<220>
   <223> Synthetic
<400> 27
   ctgcagaata gatctctaac actctcccct gttg 34
<210> 28
   <211> 51
   <212> DNA
   <213> Artificial
<220>
   <223> Synthetic
<400> 28
   cagtgtgatc tcgagaattc aggacctcac catgggatgg agctgtatca t 51
<210> 29
   <211> 22
   <212> DNA
   <213> Artificial
<220>
   <223> Synthetic
<400> 29
   gtgtcttcgg gtctcaggct gt 22
<210> 30
   <211> 41
   <212> DNA
   <213> Artificial
<220>
   <223> Synthetic
<400> 30
   agcttctcga gttaacagat ctaggcctcc taggtcgaca t 41
<210> 31
   <211> 39
   <212> DNA
   <213> Artificial
<220>
   <223> Synthetic
<400> 31
   cgatgtcgac ctaggaggcc tagatctgtt aactcgaga 39
<210> 32
   <211> 64
   <212> DNA
   <213> Artificial
<220>
   <223> Synthetic
<400> 32
   cgaggctctg cacaaccact acacgcagaa gagcctctcc ctgtctcccg ggaaatgaaa 60
   gccg 64
<210> 33
   <211> 72
   <212> DNA
   <213> Artificial
<220>
   <223> Synthetic
<400> 33
   aattcggctt tcatttcccg ggagacaggg agaggctctt ctgcgtgtag tggttgtgca 60
   gagcctcgtg ca 72
<210> 34
   <211> 9511
   <212> DNA
   <213> Artificial
<220>
   <223> Synthetic
<400> 34
   gaattaattc ataccagatc accgaaaact gtcctccaaa tgtgtccccc tcacactccc     60
   aaattcgcgg gcttctgcct cttagaccac tctaccctat tccccacact caccggagcc    120
   aaagccgcgg cccttccgtt tctttgcttt tgaaagaccc cacccgtagg tggcaagcta    180
   gcttaagtaa cgccactttg caaggcatgg aaaaatacat aactgagaat agaaaagttc    240
   agatcaaggt caggaacaaa gaaacagctg aataccaaac aggatatctg tggtaagcgg    300
   ttcctgcccc ggctcagggc caagaacaga tgagacagct gagtgatggg ccaaacagga    360
   tatctgtggt aagcagttcc tgccccggct cggggccaag aacagatggt ccccagatgc    420
   ggtccagccc tcagcagttt ctagtgaatc atcagatgtt tccagggtgc cccaaggacc    480
   tgaaaatgac cctgtacctt atttgaacta accaatcagt tcgcttctcg cttctgttcg    540
   cgcgcttccg ctctccgagc tcaataaaag agcccacaac ccctcactcg gcgcgccagt    600
   cttccgatag actgcgtcgc ccgggtaccc gtattcccaa taaagcctct tgctgtttgc    660
   atccgaatcg tggtctcgct gttccttggg agggtctcct ctgagtgatt gactacccac    720
   gacgggggtc tttcatttgg gggctcgtcc gggatttgga gacccctgcc cagggaccac 780
   cgacccacca ccgggaggta agctggccag caacttatct gtgtctgtcc gattgtctag 840
   tgtctatgtt tgatgttatg cgcctgcgtc tgtactagtt agctaactag ctctgtatct 900
   ggcggacccg tggtggaact gacgagttct gaacacccgg ccgcaaccct gggagacgtc 960
   ccagggactt tgggggccgt ttttgtggcc cgacctgagg aagggagtcg atgtggaatc 1020
   cgaccccgtc aggatatgtg gttctggtag gagacgagaa cctaaaacag ttcccgcctc 1080
   cgtctgaatt tttgctttcg gtttggaacc gaagccgcgc gtcttgtctg ctgcagcgct 1140
   gcagcatcgt tctgtgttgt ctctgtctga ctgtgtttct gtatttgtct gaaaattagg 1200
   gccagactgt taccactccc ttaagtttga ccttaggtca ctggaaagat gtcgagcgga 1260
   tcgctcacaa ccagtcggta gatgtcaaga agagacgttg ggttaccttc tgctctgcag 1320
   aatggccaac ctttaacgtc ggatggccgc gagacggcac ctttaaccga gacctcatca 1380
   cccaggttaa gatcaaggtc ttttcacctg gcccgcatgg acacccagac caggtcccct 1440
   acatcgtgac ctgggaagcc ttggcttttg acccccctcc ctgggtcaag ccctttgtac 1500
   accctaagcc tccgcctcct cttcctccat ccgccccgtc tctccccctt gaacctcctc 1560
   gttcgacccc gcctcgatcc tccctttatc cagccctcac tccttctcta ggcgccggaa 1620
   ttccgatctg atcaagagac aggatgaggg agcttgtata tccattttcg gatctgatca 1680
   gcacgtgttg acaattaatc atcggcatag tatatcggca tagtataata cgacaaggtg 1740
   aggaactaaa ccatggccaa gcctttgtct caagaagaat ccaccctcat tgaaagagca 1800
   acggctacaa tcaacagcat ccccatctct gaagactaca gcgtcgccag cgcagctctc 1860
   tctagcgacg gccgcatctt cactggtgtc aatgtatatc attttactgg gggaccttgt 1920
   gcagaactcg tggtgctggg cactgctgct gctgcggcag ctggcaacct gacttgtatc 1980
   gtcgcgatcg gaaatgagaa caggggcatc ttgagcccct gcggacggtg tcgacaggtg 2040
   cttctcgatc tgcatcctgg gatcaaagcg atagtgaagg acagtgatgg acagccgacg 2100
   gcagttggga ttcgtgaatt gctgccctct ggttatgtgt gggagggcta agcacttcgt 2160
   ggccgaggag caggactgac acgtgctacg agatttcgat tccaccgccg ccttctatga 2220
   aaggttgggc ttcggaatcg ttttccggga cgccggctgg atgatcctcc agcgcgggga 2280
   tctcatgctg gagttcttcg cccaccccaa cttgtttatt gcagcttata atggttacaa 2340
   ataaagcaat agcatcacaa atttcacaaa taaagcattt ttttcactgc attctagttg 2400
   tggtttgtcc aaactcatca atgtatctta tcatgtctgt acgagttggt tcagctgctg 2460
   cctgaggctg gacgacctcg cggagttcta ccggcagtgc aaatccgtcg gcatccagga 2520
   aaccagcagc ggctatccgc gcatccatgc ccccgaactg caggagtggg gaggcacgat 2580
   ggccgctttg gtcgaggcgg atccggccat tagccatatt attcattggt tatatagcat 2640
   aaatcaatat tggctattgg ccattgcata cgttgtatcc atatcataat atgtacattt 2700
   atattggctc atgtccaaca ttaccgccat gttgacattg attattgact agttattaat 2760
   agtaatcaat tacggggtca ttagttcata gcccatatat ggagttccgc gttacataac 2820
   ttacggtaaa tggcccgcct ggctgaccgc ccaacgaccc ccgcccattg acgtcaataa 2880
   tgacgtatgt tcccatagta acgccaatag ggactttcca ttgacgtcaa tgggtggagt 2940
   atttacggta aactgcccac ttggcagtac atcaagtgta tcatatgcca agtacgcccc 3000
   ctattgacgt caatgacggt aaatggcccg cctggcatta tgcccagtac atgaccttat 3060
   gggactttcc tacttggcag tacatctacg tattagtcat cgctattacc atggtgatgc 3120
   ggttttggca gtacatcaat gggcgtggat agcggtttga ctcacgggga tttccaagtc 3180
   tccaccccat tgacgtcaat gggagtttgt tttggcacca aaatcaacgg gactttccaa 3240
   aatgtcgtaa caactccgcc ccattgacgc aaatgggcgg taggcatgta cggtgggagg 3300
   tctatataag cagagctcgt ttagtgaacc gtcagatcgc ctggagacgc catccacgct 3360
   gttttgacct ccatagaaga caccgggacc gatccagcct ccgcggcccc aagcttctcg 3420
   agttaacaga tctaggctgg cacgacaggt ttcccgactg gaaagcgggc agtgagcgca 3480
   acgcaattaa tgtgagttag ctcactcatt aggcacccca ggctttacac tttatgcttc 3540
   cggctcgtat gttgtgtgga attgtgagcg gataacaatt tcacacagga aacagctatg 3600
   accatgatta cgccaagctt ggctgcaggt cgacggatcc actagtaacg gccgccagtg 3660
   tgctggaatt caccatgggg caacccggga acggcagcgc cttcttgctg gcacccaatg 3720
   gaagccatgc gccggaccac gacgtcacgc agcaaaggga cgaggtgtgg gtggtgggca 3780
   tgggcatcgt catgtctctc atcgtcctgg ccatcgtgtt tggcaatgtg ctggtcatca 3840
   cagccattgc caagttcgag cgtctgcaga cggtcaccaa ctacttcatc acaagcttgg 3900
   cctgtgctga tctggtcatg ggcctagcag tggtgccctt tggggccgcc catattctca 3960
   tgaaaatgtg gacttttggc aacttctggt gcgagttctg gacttccatt gatgtgctgt 4020
   gcgtcacggc atcgattgag accctgtgcg tgatcgcagt cgaccgctac tttgccatta 4080
   ctagtccttt caagtaccag agcctgctga ccaagaataa ggcccgggtg atcattctga 4140
   tggtgtggat tgtgtcaggc cttacctcct tcttgcccat tcagatgcac tggtacaggg 4200
   ccacccacca ggaagccatc aactgctatg ccaatgagac ctgctgtgac ttcttcacga 4260
   accaagccta tgccattgcc tcttccatcg tgtccttcta cgttcccctg gtgatcatgg 4320
   tcttcgtcta ctccagggtc tttcaggagg ccaaaaggca gctccagaag attgacaaat 4380
   ctgagggccg cttccatgtc cagaacctta gccaggtgga gcaggatggg cggacggggc 4440
   atggactccg cagatcttcc aagttctgct tgaaggagca caaagccctc aagacgttag 4500
   gcatcatcat gggcactttc accctctgct ggctgccctt cttcatcgtt aacattgtgc 4560
   atgtgatcca ggataacctc atccgtaagg aagtttacat cctcctaaat tggataggct 4620
   atgtcaattc tggtttcaat ccccttatct actgccggag cccagatttc aggattgcct 4680
   tccaggagct tctgtgcctg cgcaggtctt ctttgaaggc ctatggcaat ggctactcca 4740
   gcaacggcaa cacaggggag cagagtggat atcacgtgga acaggagaaa gaaaataaac 4800
   tgctgtgtga agacctccca ggcacggaag actttgtggg ccatcaaggt actgtgccta 4860
   gcgataacat tgattcacaa gggaggaatt gtagtacaaa tgactcactg ctctcgagaa 4920
   tcgaggggcg gcaccaccat catcaccacg tcgaccccgg ggactacaag gatgacgatg 4980
   acaagtaagc tttatccatc acactggcgg ccgctcgagc atgcatctag cggccgctcg 5040
   aggccggcaa ggccggatcc ccgggaattc gcccctctcc ctcccccccc cctaacgtta 5100
   ctggccgaag ccgcttggaa taaggccggt gtgcgtttgt ctatatgtta ttttccacca 5160
   tattgccgtc ttttggcaat gtgagggccc ggaaacctgg ccctgtcttc ttgacgagca 5220
   ttcctagggg tctttcccct ctcgccaaag gaatgcaagg tctgttgaat gtcgtgaagg 5280
   aagcagttcc tctggaagct tcttgaagac aaacaacgtc tgtagcgacc ctttgcaggc 5340
   agcggaaccc cccacctggc gacaggtgcc tctgcggcca aaagccacgt gtataagata 5400
   cacctgcaaa ggcggcacaa ccccagtgcc acgttgtgag ttggatagtt gtggaaagag 5460
   tcaaatggct ctcctcaagc gtattcaaca aggggctgaa ggatgcccag aaggtacccc 5520
   attgtatggg atctgatctg gggcctcggt gcacatgctt tacatgtgtt tagtcgaggt 5580
   taaaaaaacg tctaggcccc ccgaaccacg gggacgtggt tttcctttga aaaacacgat 5640
   gataatatgg cctcctttgt ctctctgctc ctggtaggca tcctattcca tgccacccag 5700
   gccgagctca cccagtctcc agactccctg gctgtgtctc tgggcgagag ggccaccatc 5760
   aactgcaagt ccagccagag tgttttgtac agctccaaca ataagaacta tttagcttgg 5820
   tatcagcaga aaccaggaca gcctcctaag ctgctcattt actgggcatc tacccgggaa 5880
   tccggggtcc ctgaccgatt cagtggcagc gggtctggga cagatttcac tctcaccatc 5940
   agcagcctgc aggctgaaga tgtggcagtt tattactgtc agcaatatta tagtactcag 6000
   acgttcggcc aagggaccaa ggtggaaatc aaacgaactg tggctgcacc atctgtcttc 6060
   atcttcccgc catctgatga gcagttgaaa tctggaactg cctctgttgt gtgcctgctg 6120
   aataacttct atcccagaga ggccaaagta cagtggaagg tggataacgc cctccaatcg 6180
   ggtaactccc aggagagtgt cacagagcag gacagcaagg acagcaccta cagcctcagc 6240
   agcaccctga cgctgagcaa agcagactac gagaaacaca aactctacgc ctgcgaagtc 6300
   acccatcagg gcctgagatc gcccgtcaca aagagcttca acaaggggag agtgttagtt 6360
   ctagataatt aattaggagg agatctcgag ctcgcgaaag cttggcactg gccgtcgttt 6420
   tacaacgtcg tgactgggaa aaccctggcg ttacccaact taatcgcctt gcagcacatc 6480
   ccccttctgc cagcctccta ggtcgacatc gataaaataa aagattttat ttagtctcca 6540
   gaaaaagggg ggaatgaaag accccacctg taggtttggc aagctagctt aagtaacgcc 6600
   attttgcaag gcatggaaaa atacataact gagaatagag aagttcagat caaggtcagg 6660
   aacagatgga acagctgaat atgggccaaa caggatatct gtggtaagca gttcctgccc 6720
   cggctcaggg ccaagaacag atggaacagc tgaatatggg ccaaacagga tatctgtggt 6780
   aagcagttcc tgccccggct cagggccaag aacagatggt ccccagatgc ggtccagccc 6840
   tcagcagttt ctagagaacc atcagatgtt tccagggtgc cccaaggacc tgaaatgacc 6900
   ctgtgcctta tttgaactaa ccaatcagtt cgcttctcgc ttctgttcgc gcgcttctgc 6960
   tccccgagct caataaaaga gcccacaacc cctcactcgg ggcgccagtc ctccgattga 7020
   ctgagtcgcc cgggtacccg tgtatccaat aaaccctctt gcagttgcat ccgacttgtg 7080
   gtctcgctgt tccttgggag ggtctcctct gagtgattga ctacccgtca gcgggggtct 7140
   ttcatttggg ggctcgtccg ggatcgggag acccctgccc agggaccacc gacccaccac 7200
   cgggaggtaa gctggctgcc tcgcgcgttt cggtgatgac ggtgaaaacc tctgacacat 7260
   gcagctcccg gagacggtca cagcttgtct gtaagcggat gccgggagca gacaagcccg 7320
   tcagggcgcg tcagcgggtg ttggcgggtg tcggggcgca gccatgaccc agtcacgtag 7380
   cgatagcgga gtgtatactg gcttaactat gcggcatcag agcagattgt actgagagtg 7440
   caccatatgc ggtgtgaaat accgcacaga tgcgtaagga gaaaataccg catcaggcgc 7500
   tcttccgctt cctcgctcac tgactcgctg cgctcggtcg ttcggctgcg gcgagcggta 7560
   tcagctcact caaaggcggt aatacggtta tccacagaat caggggataa cgcaggaaag 7620
   aacatgtgag caaaaggcca gcaaaaggcc aggaaccgta aaaaggccgc gttgctggcg 7680
   tttttccata ggctccgccc ccctgacgag catcacaaaa atcgacgctc aagtcagagg 7740
   tggcgaaacc cgacaggact ataaagatac caggcgtttc cccctggaag ctccctcgtg 7800
   cgctctcctg ttccgaccct gccgcttacc ggatacctgt ccgcctttct cccttcggga 7860
   agcgtggcgc tttctcatag ctcacgctgt aggtatctca gttcggtgta ggtcgttcgc 7920
   tccaagctgg gctgtgtgca cgaacccccc gttcagcccg accgctgcgc cttatccggt 7980
   aactatcgtc ttgagtccaa cccggtaaga cacgacttat cgccactggc agcagccact 8040
   ggtaacagga ttagcagagc gaggtatgta ggcggtgcta cagagttctt gaagtggtgg 8100
   cctaactacg gctacactag aaggacagta tttggtatct gcgctctgct gaagccagtt 8160
   accttcggaa aaagagttgg tagctcttga tccggcaaac aaaccaccgc tggtagcggt 8220
   ggtttttttg tttgcaagca gcagattacg cgcagaaaaa aaggatctca agaagatcct 8280
   ttgatctttt ctacggggtc tgacgctcag tggaacgaaa actcacgtta agggattttg 8340
   gtcatgagat tatcaaaaag gatcttcacc tagatccttt taaattaaaa atgaagtttt 8400
   aaatcaatct aaagtatata tgagtaaact tggtctgaca gttaccaatg cttaatcagt 8460
   gaggcaccta tctcagcgat ctgtctattt cgttcatcca tagttgcctg actccccgtc 8520
   gtgtagataa ctacgatacg ggagggctta ccatctggcc ccagtgctgc aatgataccg 8580
   cgagacccac gctcaccggc tccagattta tcagcaataa accagccagc cggaagggcc 8640
   gagcgcagaa gtggtcctgc aactttatcc gcctccatcc agtctattaa ttgttgccgg 8700
   gaagctagag taagtagttc gccagttaat agtttgcgca acgttgttgc cattgctgca 8760
   ggcatcgtgg tgtcacgctc gtcgtttggt atggcttcat tcagctccgg ttcccaacga 8820
   tcaaggcgag ttacatgatc ccccatgttg tgcaaaaaag cggttagctc cttcggtcct 8880
   ccgatcgttg tcagaagtaa gttggccgca gtgttatcac tcatggttat ggcagcactg 8940
   cataattctc ttactgtcat gccatccgta agatgctttt ctgtgactgg tgagtactca 9000
   accaagtcat tctgagaata gtgtatgcgg cgaccgagtt gctcttgccc ggcgtcaaca 9060
   cgggataata ccgcgccaca tagcagaact ttaaaagtgc tcatcattgg aaaacgttct 9120
   tcggggcgaa aactctcaag gatcttaccg ctgttgagat ccagttcgat gtaacccact 9180
   cgtgcaccca actgatcttc agcatctttt actttcacca gcgtttctgg gtgagcaaaa 9240
   acaggaaggc aaaatgccgc aaaaaaggga ataagggcga cacggaaatg ttgaatactc 9300
   atactcttcc tttttcaata ttattgaagc atttatcagg gttattgtct catgagcgga 9360
   tacatatttg aatgtattta gaaaaataaa caaatagggg ttccgcgcac atttccccga 9420
   aaagtgccac ctgacgtcta agaaaccatt attatcatga cattaaccta taaaaatagg 9480
   cgtatcacga ggccctttcg tcttcaagaa t 9511
<210> 35
   <211> 30
   <212> DNA
   <213> Artificial
<220>
   <223> Synthetic
<400> 35
   gatccactag taacggccgc cagaattcgc 30
<210> 36
   <211> 43
   <212> DNA
   <213> Artificial
<220>
   <223> Synthetic
<400> 36
   cagagagaca aaggaggcca tattatcatc gtgtttttca aag 43

## Description

### FIELD OF THE INVENTION

The present invention relates to the production of proteins in host cells, and more particularly to host cells containing multiple integrated copies of an integrating vector.

### BACKGROUND OF THE INVENTION

The pharmaceutical biotechnology industry is based on the production of recombinant proteins in mammalian cells. These proteins are essential to the therapeutic treatment of many diseases and conditions. In many cases, the market for these proteins exceeds a billion dollars a year. Examples of proteins produced recombinantly in mammalian cells include erythropoietin, factor VIII, factor IX, and insulin. For many of these proteins, expression in mammalian cells is preferred over expression in prokaryotic cells because of the need for correct post-translational modification (*e.g.*, glycosylation or silation; *see, e.g.,* U.S. Pat. No. 5,721,121, incorporated herein by reference).

Several methods are known for creating host cells that express recombinant proteins. In the most basic methods, a nucleic acid construct containing a gene encoding a heterologous protein and appropriate regulatory regions is introduced into the host cell and allowed to integrate. Methods of introduction include calcium phosphate precipitation, microinjection, lipofection, and electroporation. In other methods, a selection scheme is used to amplify the introduced nucleic acid construct. In these methods, the cells are co-transfected with a gene encoding an amplifiable selection marker and a gene encoding a heterologous protein (*See, e.g.,* Schroder and Friedl, Biotech. Bioeng. 53(6):547-59 [1997]). After selection of the initial tranformants, the transfected genes are amplified by the stepwise increase of the selective agent (*e.g*., dihydrofolate reductase) in the culture medium. In some cases, the exogenous gene may be amplified several hundred-fold by these procedures. Other methods of recombinant protein expression in mammalian cells utilize transfection with episomal vectors (*e.g*., plasmids).

Current methods for creating mammalian cell lines for expression of recombinant proteins suffer from several drawbacks. (*See*, *e.g.,* Mielke et al., Biochem. 35:2239-52 [1996]). Episomal systems allow for high expression levels of the recombinant protein, but are frequently only stable for a short time period (*See, e.g.,* Klehr and Bode, Mol. Genet. (Life Sci. Adv.) 7:47-52 [1988]). Mammalian cell lines containing integrated exogenous genes are somewhat more stable, but there is increasing evidence that stability depends on the presence of only a few copies or even a single copy of the exogenous gene.

Standard transfection techniques favor the introduction of multiple copies of the transgene into the genome of the host cell. Multiple integration of the transgene has, in many cases, proven to be intrinsically unstable. This intrinsic instability may be due to the characteristic head-to-tail mode of integration which promotes the loss of coding sequences by homologous recombination (*See, e.g.,* Weidle et al., Gene 66:193-203 [1988]) especially when the transgenes are transcribed (*See, e.g.,* McBumey et al., Somatic Cell Molec. Genet. 20:529-40 [1994]). Host cells also have epigenetic defense mechanisms directed against multiple copy integration events. In plants, this mechanism has been termed "cosuppression." (*See, e.g.,* Allen et al., Plant Cell 5:603-13 [1993]). Indeed, it is not uncommon that the level of expression is inversely related to copy number. These observations are consistent with findings that multiple copies of exogenous genes become inactivated by methylation (*See, e.g.,* Mehtali et al., Gene 91:179-84 [1990]) and subsequent mutagenesis (*See, e.g.,* Kricker et al., Proc. Natl. Acad. Sci. 89:1075-79 [1992]) or silenced by heterochromatin formation (*See, e.g.,* Dorer and Henikoff, Cell 77:993-1002 [1994]).

Accordingly, what is needed in the art are improved methods for making host cells that express recombinant proteins. Preferably, the host cells will be stable over extended periods of time and express the protein encoded by a transgene at high levels.

### SUMMARY OF THE INVENTION

The present invention is defined in the claims and relates to the production of proteins in host cells, and more particularly to host cells containing multiple integrated copies of an integrating vector. The present disclosure is not limited to host cells transfected with a particular number of integrating vectors. Indeed, host cells containing a wide range of integrating vectors can be envisaged. In some cases a host cell may comprise a genome containing preferably at least about two integrated integrating vectors. In still further embodiments, the genome preferably comprises at least 3 integrated integrating vectors and most preferably at least 4 integrated integrating vectors, 5 integrated integrating vectors, 6 integrated integrating vectors, 7 integrated integrating vectors, 10 integrated integrating vectors, 15 integrated integrating vectors, 20 integrated integrating vectors, or 50 integrated integrating vectors.

The host cells are not limited to containing vectors encoding a single secreted protein of interest (*i.e*., exogenous protein). Indeed, it is contemplated that the host cells are transfected with vectors encoding multiple secreted proteins of interest. In some embodiments, the integrating vector comprises at least two exogenous genes. In other embodiments, the at least two exogenous genes are arranged in a polycistronic sequence. In yet other embodiments, the at least two exogenous genes are separated by an internal ribosome entry site. In other embodiments, the at least two exogenous genes are arranged in a polycistronic sequence. In still further embodiments, the two exogenous genes comprise a heavy chain of an immunoglobulin molecule and a light chain of an immunoglobulin molecule. In other embodiments, one of the at least two exogenous genes is a selectable marker. In still other embodiments, the host cells comprise at least 2 integrated copies of a first integrating vector comprising a first exogenous gene, and at least 1 integrated copy of a second integrating vector or other vector comprising a second exogenous gene. In still further embodiments, the host cells comprise at least 10 integrated copies of a first integrating vector comprising a first exogenous gene, and at least 1 integrated copy of a second integrating vector or other vector comprising a second exogenous gene.

In some preferred embodiments, the integrating vectors comprise at least one exogenous gene operably linked to a promoter. The present disclosure is not limited to vectors containing a particular promoter. Indeed, a variety of promoters are contemplated. In some embodiments, the promoter is selected from the group consisting of the alpha-lactalbumin promoter, cytomegalovirus promoter and the long terminal repeat of Moloney murine leukemia virus. In other embodiments, the integrating vectors further comprise a secretion signal operably linked to the exogenous gene. In still other embodiments, the integrating vectors further comprise an RNA export element operably linked to the exogenous gene.

The present disclosure is not limited to a particular integrating vector. Indeed, a variety of integrating vectors are contemplated. In some embodiments, the integrating vector is selected from the group consisting of a retroviral vector, a lentiviral vector, and a transposon vector. In some preferred embodiments, the retroviral vector is a pseudotyped retroviral vector. In other preferred embodiments, the pseudotyped retroviral vector comprises a G glycoprotein. The retroviral vectors are not limited to a particular G glycoprotein. Indeed, a variety of G glycoproteins are contemplated. In some embodiments, the G glycoprotein is selected from the group consisting of vesicular stomatitis virus, Piry virus, Chandipura virus, Spring viremia of carp virus and Mokola virus G glycoproteins. In still further embodiments, the retroviral vector comprises long terminal repeats. The retroviral vectors are not limited to a particular LTR. Indeed, a variety of LTRs are contemplated, including, but not limited to MoMLV, MoMuSV, MMTV long terminal repeats.

In other embodiments, the retroviral vector is a lentiviral vector. In some preferred embodiments, the lentiviral vector is pseudotyped. In some particularly preferred embodiments, the lentiviral vector comprises a G glycoprotein. In still further embodiments, the G glycoprotein is selected from the group consisting of vesicular stomatitis virus, Piry virus, Chandipura virus, Spring viremia of carp virus and Mokola virus G glycoproteins. In still other embodiments, the lentiviral vector comprises long terminal repeats selected from the group consisting of HIV and equine infectious anemia long terminal repeats.

The present disclosure is not limited to a particular host cell. Indeed, a variety of host cells are contemplated. In some embodiments, the host cell is cultured *in vitro.* In still further embodiments, the host cell is selected from chinese hamster ovary cells, baby hamster kidney cells, and bovine mammary epithelial cells. In some preferred embodiments, the host cells are clonally derived. In other embodiments, the host cells are non-clonally derived. In some embodiments, the genome of the host cell is stable for greater than 10 passages. In other embodiments, the genome is stable for greater than 50 passages, while in still other embodiments, the genome is stable for greater than 100 passages. In still other embodiments, the host cells can be an (non human) embryonic stem cell, oocyte, or embryo. In some embodiments, the integrated vector is stable in the absence of selection.

The present disclosure is not limited to vectors encoding a particular protein of interest. Indeed, vectors encoding a variety of proteins of interest encoded by exogenous genes are contemplated. In some embodiments, the protein of interest is selected from hepatitis B surface antigen, MN14 antibody, LL2 antibody, *botulinum* toxin antibody and cc49IL2. In some embodiments, the genes encoding the protein of interest are intronless, while in other embodiments, the genes encoding the protein of interest include at least one intron.

The present disclosure also provides a method for transfecting or transducing host cells comprising: 1) providing: a) a host cell comprising a genome, and b) a plurality of integrating vectors; and 2) contacting the host cell with the plurality of integrating vectors under conditions such that at least two integrating vectors integrate into the genome of the host cell. In some embodiments, the conditions comprise contacting the host cells at a multiplicity of infection of greater than 10. In other embodiments, the conditions comprise contacting the host cells at a multiplicity of infection of from about 10 to 1,000,000. In still further embodiments, the conditions comprise contacting the host cells at a multiplicity of infection of from about 100 to 10,000. In still further embodiments, the conditions comprise contacting the host cells at a multiplicity of infection of from about 100 to 1,000. In still other embodiments, the method further comprises transfecting said host cells with at least two integrating vectors, each of said two integrating comprising a different exogenous gene. In still other embodiments, the conditions comprise serial transfection or transduction or host cells wherein the host cells are transfected or transduced in at least a first transfection or transduction with a vector encoding a protein of interest and then retransfected or re-transduced in a separate transfection or transduction step.

The present disclosure further provides a method of producing a secreted protein of interest comprising: 1) providing a host cell comprising a genome, the genome comprising at least two integrated copies of at least one integrating vector comprising an exogenous gene operably linked to a promotor, wherein the exogenous gene encodes a protein of interest, and 2) culturing the host cells under conditions such that the secreted protein of interest is produced. In some preferred embodiments, the integrating vector further comprises a secretion signal sequence operably linked to said exogenous gene. In other embodiments, the methods further comprise step 3) isolating the protein of interest. The present invention is not limited to any particular culture system. Indeed, a variety of culture systems are contemplated, including, but not limited to roller bottle cultures, perfusion cultures, batch fed cultures, and petri dish cultures. In some embodiments, the cell line is clonally selected, while in other embodiments, the cells are non-clonally selected.

The methods of the present disclosure are not limited to host cells containing any particular number of integrated integrating vectors. Indeed, in some embodiments, the genome of the host cell comprises greater than 3 integrated copies of the integrating vector; in other embodiments, genome of the host cell comprises greater than 4 integrated copies of the integrating vector.; in still other embodiments, the genome of the host cell comprises greater than 5 integrated copies of the integrating vector; in further embodiments, the genome of the host cell comprises greater than 7 integrated copies of the integrating vector. In still further embodiments, the genome of the host cell comprises greater than 10 integrated copies of the integrating vector (this is the main object of the invention). In other embodiments, the genome of the host cell comprises between about 2 and 20 integrated copies of the integrating vector. In some embodiments, the genome of the host cell comprises between about 3 and 10 integrated copies of the integrating vector.

The methods of the present disclosure are not limited to any particular integrating vector. Indeed, the use of a variety of integrating vectors is contemplated. In some embodiments, the integrating vector is a retroviral vector. In other embodiments, the retroviral vector is a pseudotyped retroviral vector. In other embodiments, the retroviral vector is a lentiviral vector.

The methods of the present disclosure are not limited to the use of any particular host cell. Indeed, the use of a variety of host cells is contemplated, including, but not limited to, Chinese hamster ovary cells, baby hamster kidney cells, bovine mammary epithelial cells, oocytes, embryos, stem cells, and embryonic stem cells.

The methods of the present disclosure are not limited to the production of any particular amount of exogenous protein (*i.e*., secreted protein of interest) from the host cells. Indeed, it is contemplated that a variety of expression levels are acceptable from the methods of the present disclosure. In some embodiments, the host cells synthesize greater than about 1 picogram per cell per day of the protein of interest. In other embodiments, the host cells synthesize greater than about 10 picograms per cell per day of the protein of interest. In still further embodiments, the host cells synthesize greater than about 50 picograms per cell per day of the protein of interest.

In other embodiments, the present disclosure provides a method for screening compounds comprising: 1) providing a) a host cell comprising a genome, the genome comprising at least two integrated copies of at least one integrating vector comprising an exogenous gene operably linked to a promotor, wherein the exogenous gene encodes a secreted protein of interest; and b) one or more test compounds; 2) culturing the host cells under conditions such that the secreted protein of interest is expressed; 3) treating the host cells with one or more test compounds; and 4) assaying for the presence or absence of a response in the host cells to the test compound. In some embodiments, the exogenous gene encodes a protein selected from the group consisting of reporter proteins, membrane receptor proteins, nucleic acid binding proteins, cytoplasmic receptor proteins, ion channel proteins, signal transduction proteins, protein kinases, protein phosphatases, and proteins encoded by oncogenes.

In still further embodiments, the host cell further comprises a reporter gene. In other embodiments, the reporter gene is selected from the group consisting of green fluorescent protein, luciferase, beta-galactosidase, and beta-lactamase. In some embodiments, the assaying step further comprises detecting a signal from the reporter gene. In other embodiments, the genome of the host cell comprises at least two integrating vectors, each comprising a different exogenous gene.

In still other embodiments, the present disclosure provides methods for comparing protein activity comprising: 1) providing a) a first host cell comprising a first integrating vector comprising a promoter operably to a first exogenous gene, wherein the first exogenous gene encodes a first secreted protein of interest, and b) at least a second host cell comprising a second integrating vector comprising a promoter operably linked to a second exogenous gene, wherein the second exogenous gene encodes a second exogenous gene that is a variant of the first secreted protein of interest; 2) culturing the host cells under conditions such that the first and second secreted proteins of interest are produced; and 3) comparing the activities of the first and second secreted proteins of interest.

In some embodiments, the exogenous gene encodes a protein selected from the group consisting of membrane receptor proteins, nucleic acid binding proteins, cytoplasmic receptor proteins, ion channel proteins, signal transduction proteins, protein kinases, protein phosphatases, cell cycle proteins, and proteins encoded by oncogenes. In other embodiments, the first and second proteins of interest differ by a single amino acid. In still further embodiments, the first and second proteins of interest are greater than 95% identical, preferably greater than 90% identical, and most preferably greater than 80% identical.

In other embodiments, the present disclosure provides methods comprising: 1) providing: a) a host cell comprising a genome comprising at least one integrated exogenous gene; and b) a plurality of integrating vectors; and 2) contacting the host cell with the plurality of integrating vectors under conditions such that at least two of the integrating vectors integrate into the genome of the host cell. In some embodiments, the integrated exogenous gene comprises an integrating vector. In other embodiments, the host cell is clonally selected. In alternative embodiments, the host cell is non-clonally selected.

In still further embodiments, the present disclosure provides methods of indirectly detecting the expression of a protein of interest comprising providing a host cell transfected with a vector encoding a polycistronic sequence, wherein the polycistronic sequence comprises a signal protein and a protein of interest operably linked by an IRES, and culturing the host cells under conditions such that the signal protein and protein of interest are produced, wherein the presence of the signal protein indicates the presence of the protein of interest. The methods of the present disclosure are not limited to the expression of any particular protein of interest. Indeed, the expression of a variety of proteins of interest is contemplated, including, but not limited to, G-protein coupled receptors. The present disclosure is not limited to the use of any particular signal protein. Indeed, the use of variety of signal proteins is contemplated, including, but not limited to, immunoglobulin heavy and light chains, beta-galactosidase beta-lactamase, green fluorescent protein, and luciferase. In some embodiments, expression of the signal protein and protein of interest is driven by the same promoter and the signal protein and protein of interest are transcribed as a single transcriptional unit.

### DESCRIPTION OF THE FIGURES

Figure 1 is a western blot of a 15% SDS-PAGE gel run under denaturing conditions and probed with anti-human IgG (Fc) and anti-human IgG (Kappa).
Figure 2 is a graph of MN14 expression over time.
Figure 3 is a Western blot of a 15% PAGE run under non-denaturing conditions and probed with anti-human IgG (Fc) and anti-human IgG (Kappa).

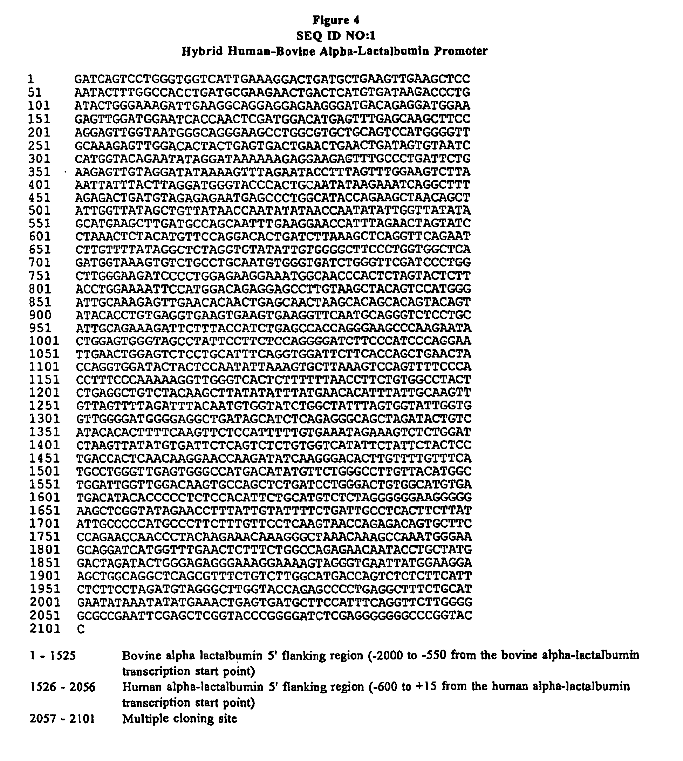

Figure 4 provides the sequence for the hybrid human-bovine alpha-lactalbumin promoter (SEQ ID NO:1).

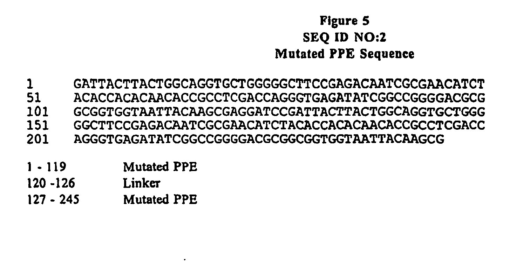

Figure 5 provides the sequence for the mutated PPE sequence (SEQ ID NO:2).

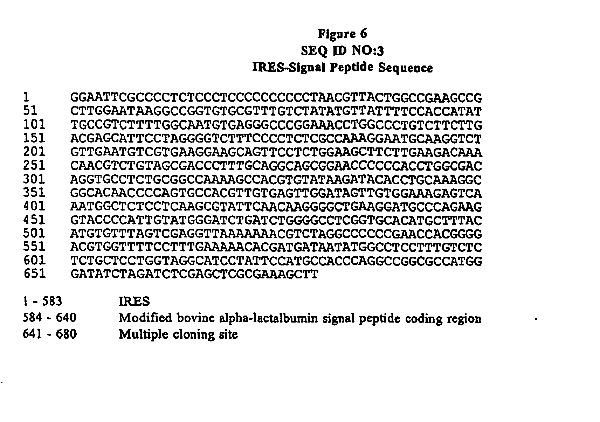

Figure 6 provides the sequence for the IRES-Signal peptide sequence (SEQ ID NO:3).

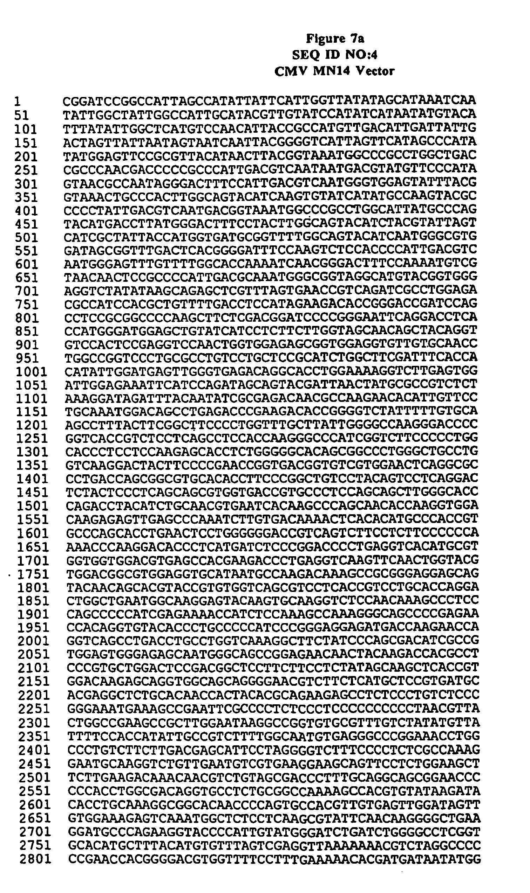

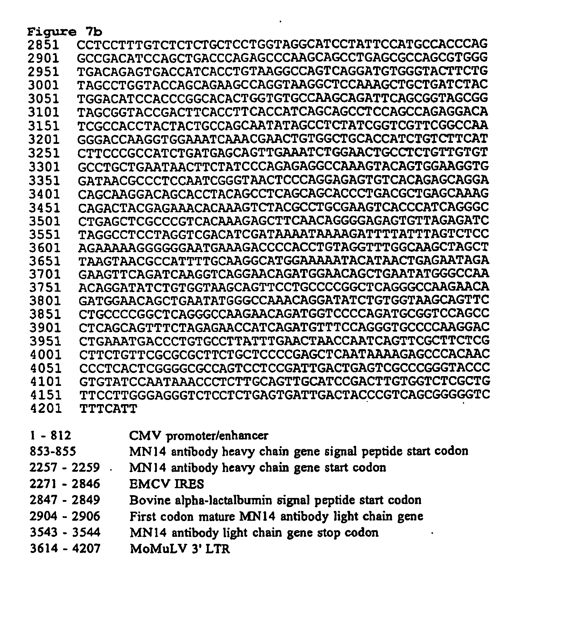

Figures 7a and 7b provide the sequence for CMV MN14 vector (SEQ ID NO:4).

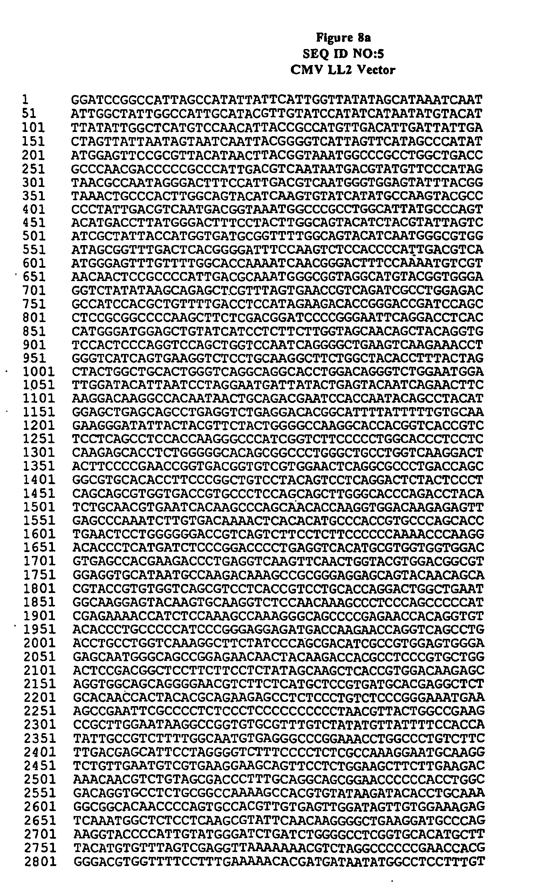

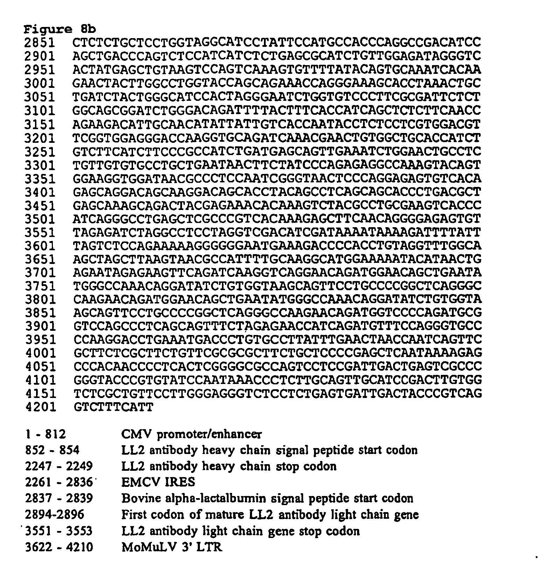

Figures 8a and 8b provide the sequence for the CMV LL2 vector (SEQ ID NO:5).

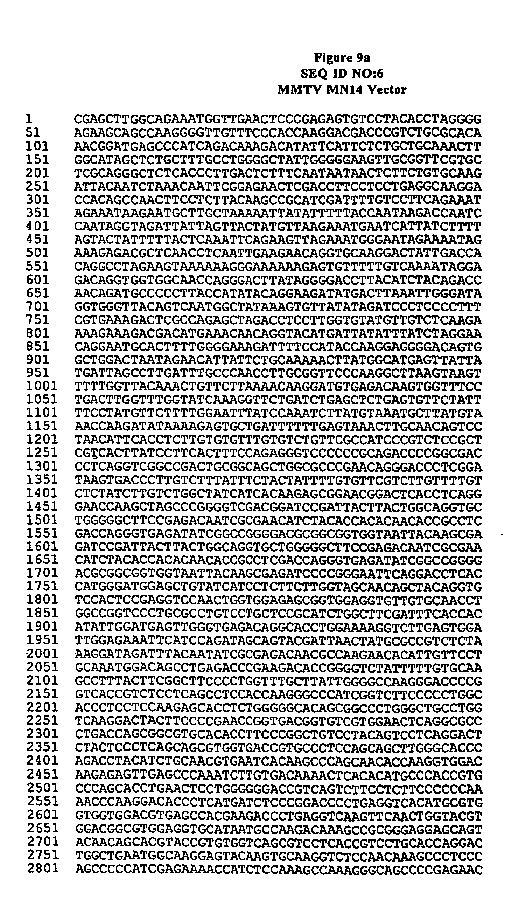

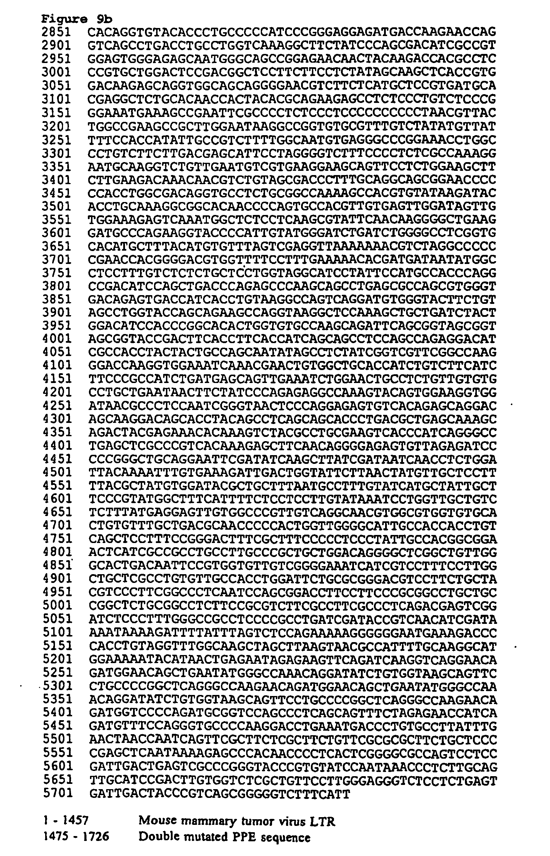

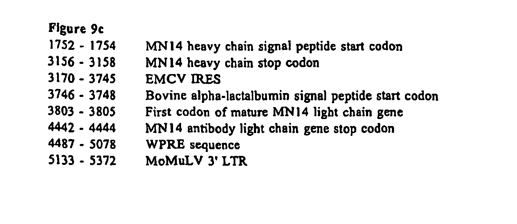

Figures 9a-c provide the sequence for the MMTV MN14 vector (SEQ ID NO:6).

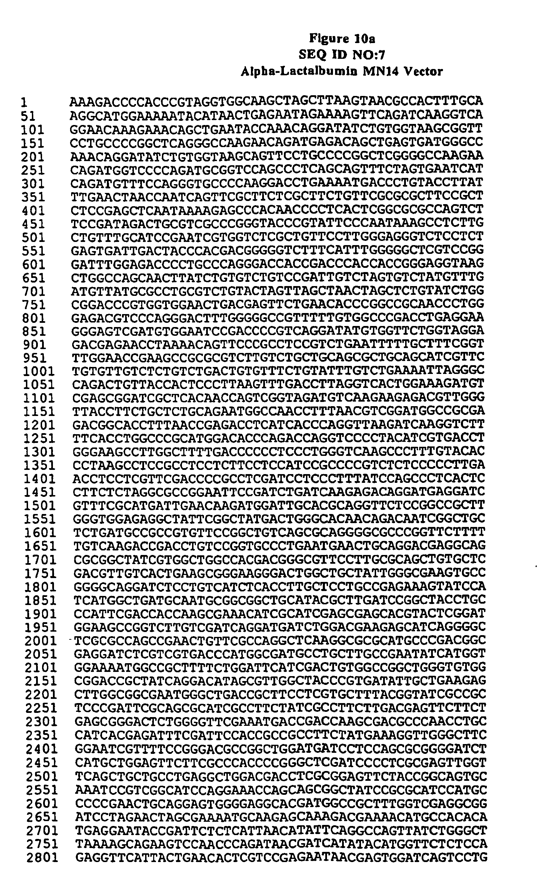

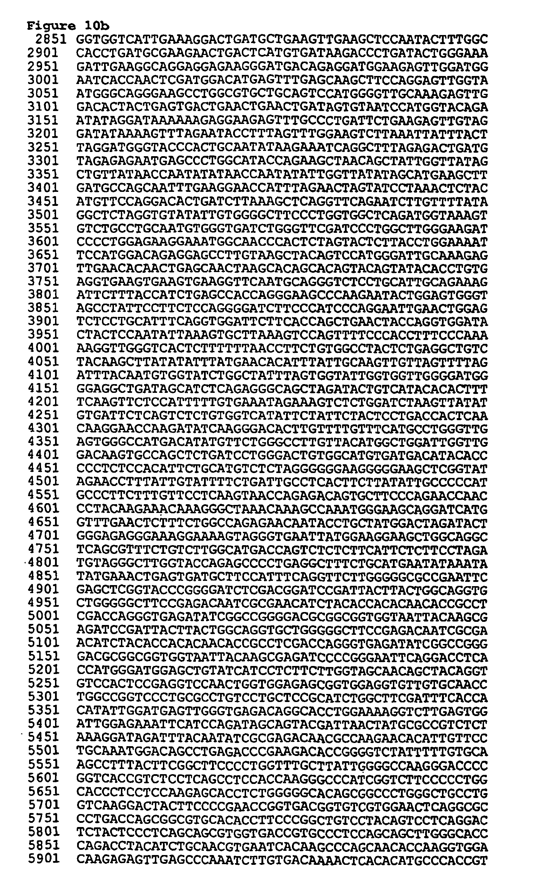

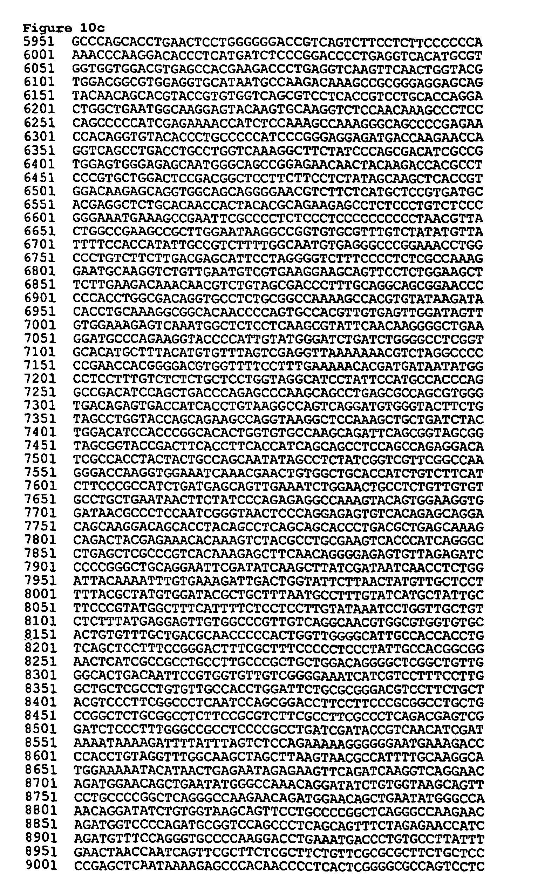

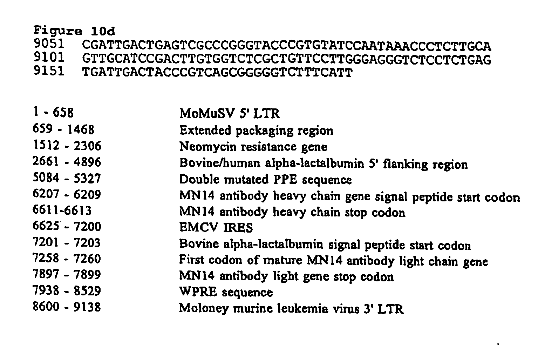

Figures 10a-d provide the sequence for the alpha-lactalbumin MN14 Vector (SEQ ID NO:7).

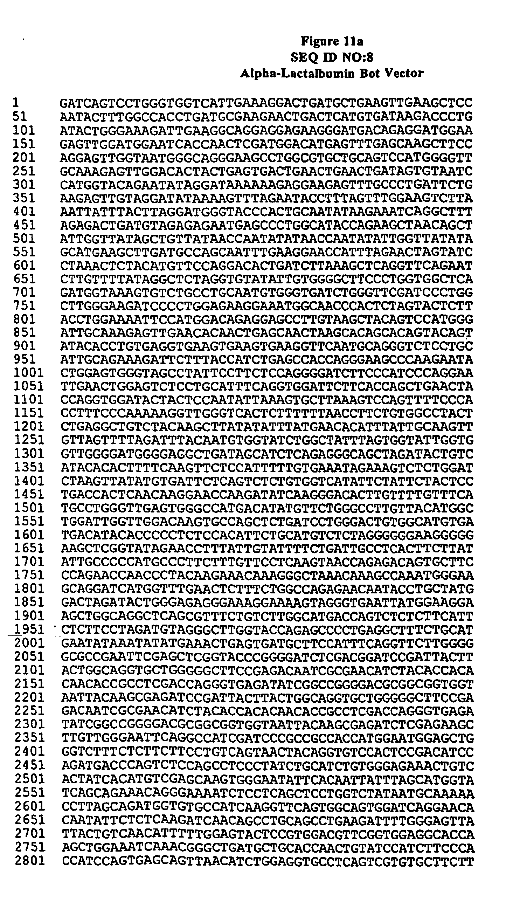

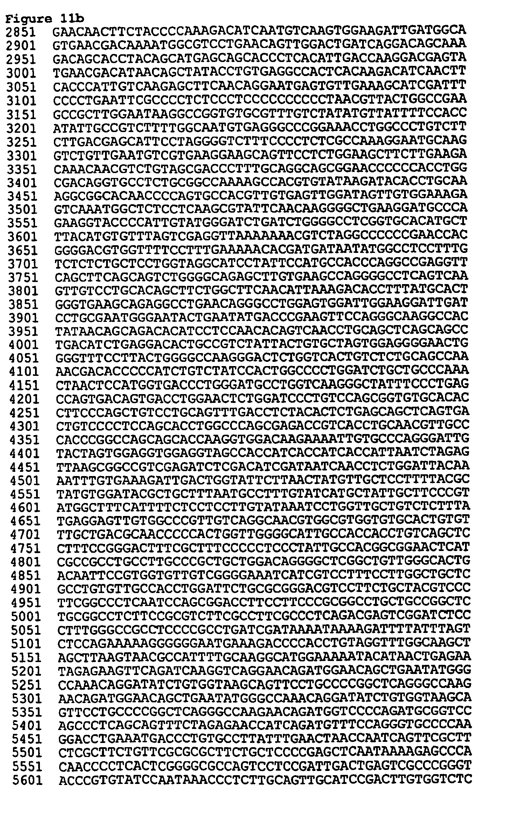

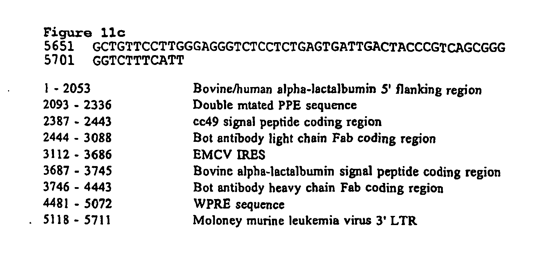

Figures 11a-c provide the sequence for the alpha-lactalbumin Bot vector (SEQ ID NO:8).

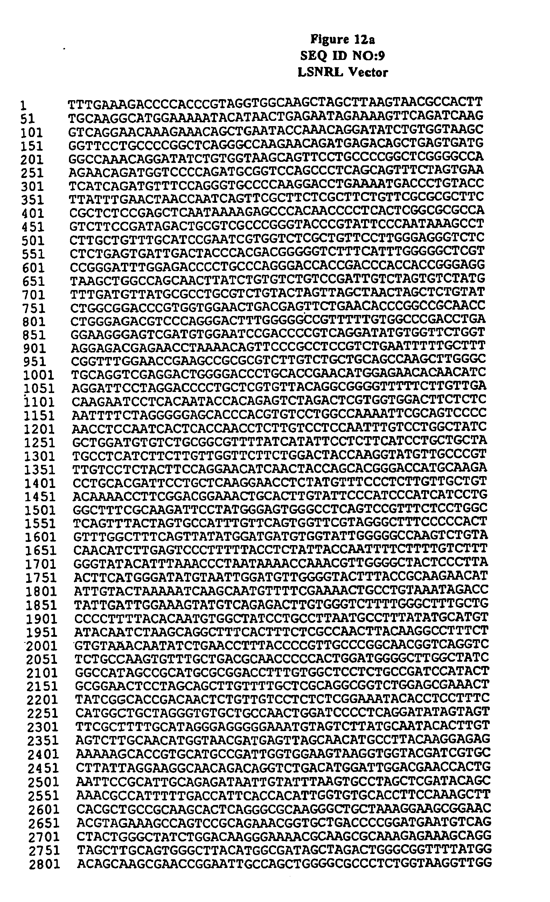

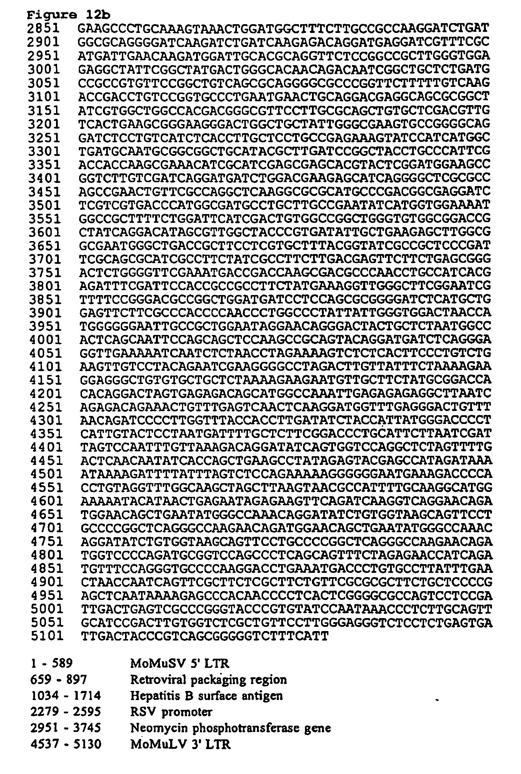

Figures 12a-b provide the sequence for the LSRNL vector (SEQ ID NO:9).

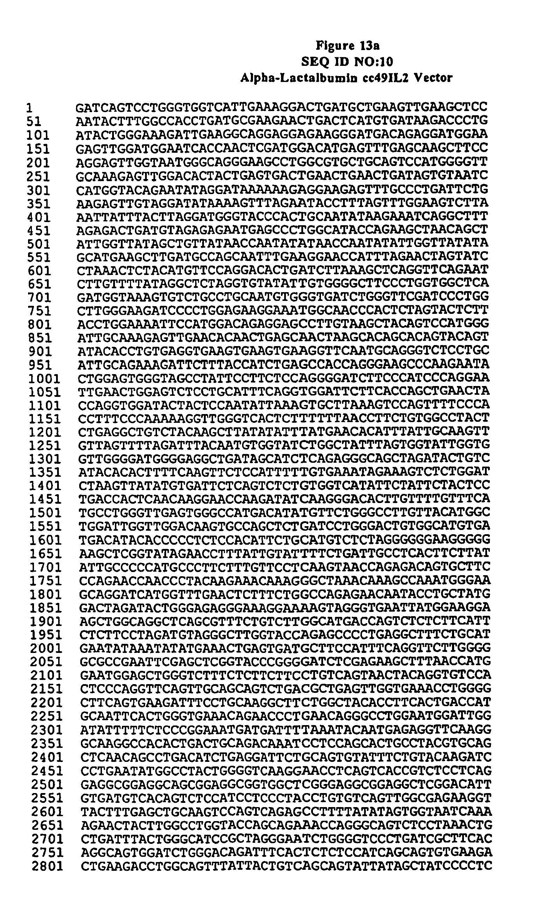

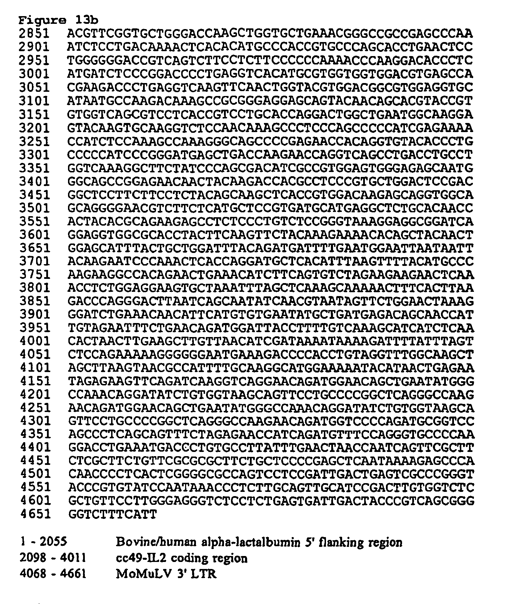

Figures 13a-b provide the sequence for the alpha-lactalbumin cc49IL2 vector (SEQ ID NO:10).

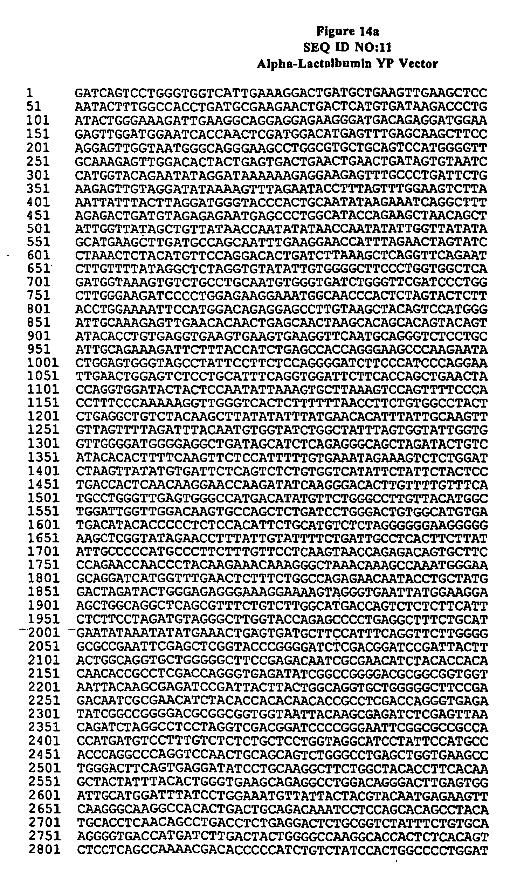

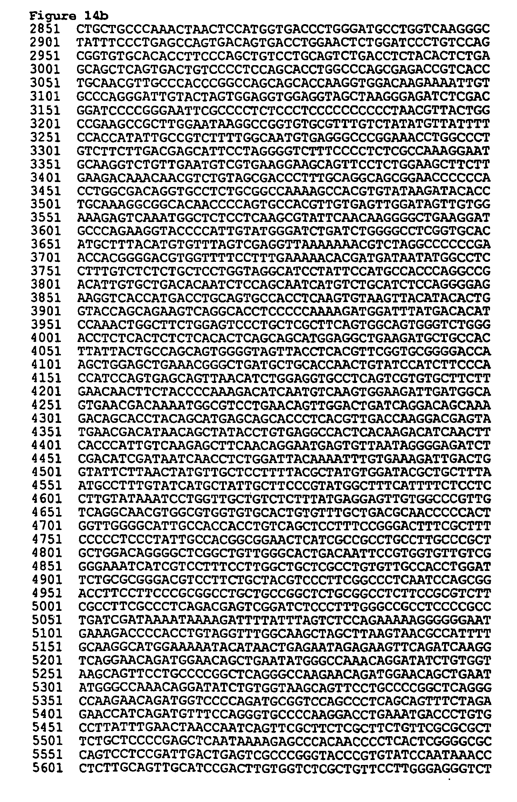

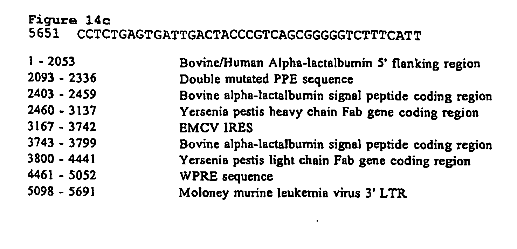

Figures 14a-c provides the sequence for the alpha-lactalbumin YP vector (SEQ ID NO:11).

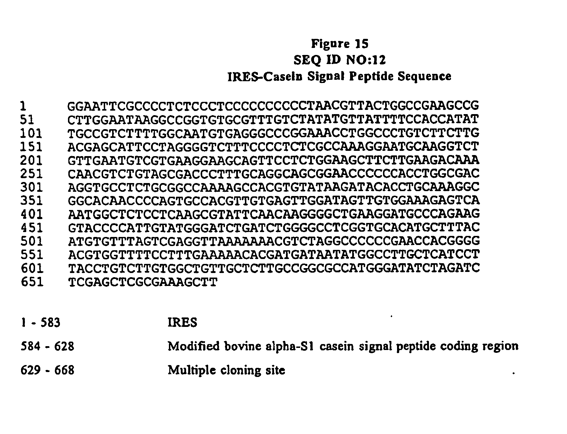

Figure 15 provides the sequence for the IRES-Casein signal peptide sequence (SEQ ID NO:12).

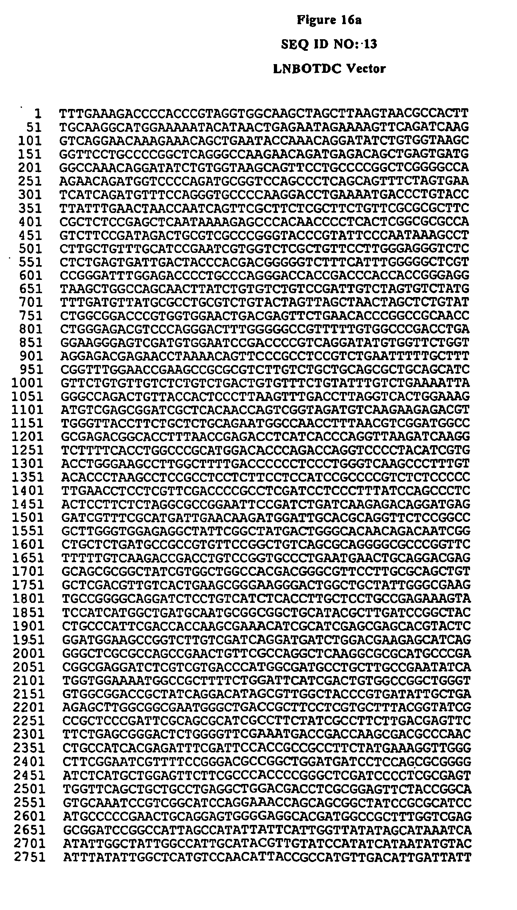

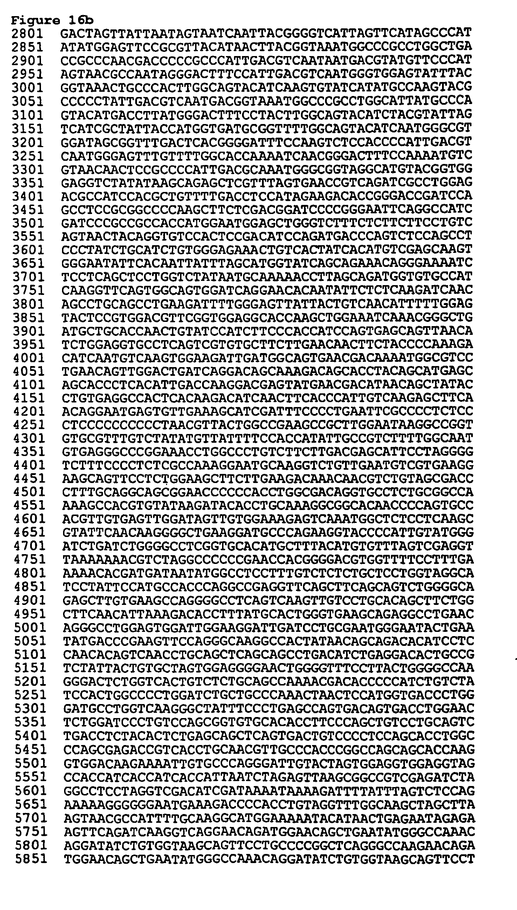

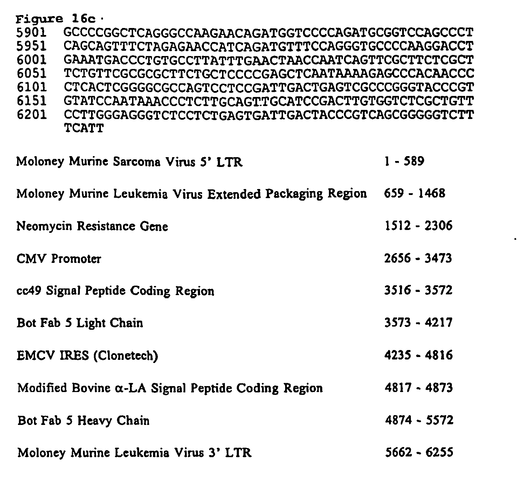

Figures 16a-c provide the sequence for the LNBOTDC vector (SEQ ID NO:13).
Figure 17 provides a graph depicting the INVADER Assay gene ratio in CMV promoter cell lines.
Figure 18 provides a graph depicting the INVADER Assay gene ratio in α-lactalbumin promotor cell lines.
Figures 19a-d provide the sequence of a retroviral vector that expresses a G-Protein coupled receptor and antibody light chain.

### DEFINITIONS

To facilitate understanding of the invention, a number of terms are defined below.

As used herein, the term "host cell" refers to any eukaryotic cell (*e.g.,* mammalian cells, avian cells, amphibian cells, plant cells, fish cells, and insect cells), whether located *in vitro* or in *vivo.*

As used herein, the term "cell culture" refers to any *in vitro* culture of cells. Included within this term are continuous cell lines (*e.g*., with an immortal phenotype), primary cell cultures, finite cell lines (*e.g*., non-transformed cells), and any other cell population maintained in vitro, including oocytes and embryos.

As used herein, the term "vector" refers to any genetic element, such as a plasmid, phage, transposon, cosmid, chromosome, virus, virion, etc., which is capable of replication when associated with the proper control elements and which can transfer gene sequences between cells. Thus, the term includes cloning and expression vehicles, as well as viral vectors.

As used herein, the term "integrating vector" refers to a vector whose integration or insertion into a nucleic acid (*e.g.,* a chromosome) is accomplished via an integrase. Examples of "integrating vectors" include, but are not limited to, retroviral vectors, transposons, and adeno associated virus vectors.

As used herein, the term "integrated" refers to a vector that is stably inserted into the genome (*i.e.*, into a chromosome) of a host cell.

As used herein, the term "multiplicity of infection" or "MOI" refers to the ratio of integrating vectors:host cells used during transfection or transduction of host cells. For example, if 1,000,000 vectors are used to transduce 100,000 host cells, the multiplicity of infection is 10. The use of this term is not limited to events involving transduction, but instead encompasses introduction of a vector into a host by methods such as lipofection, microinjection, calcium phosphate precipitation, and electroporation.

As used herein, the term "genome" refers to the genetic material (*e.g*., chomosomes) of an organism.

The term "nucleotide sequence of interest" refers to any nucleotide sequence (*e.g*., RNA or DNA), the manipulation of which may be deemed desirable for any reason (*e.g*., treat disease, confer improved qualities, expression of a protein of interest in a host cell, expression of a ribozyme, *etc*.), by one of ordinary skill in the art. Such nucleotide sequences include, but are not limited to, coding sequences of structural genes (*e.g*., reporter genes, selection marker genes, oncogenes, drug resistance genes, growth factors, *etc*.), and non-coding regulatory sequences which do not encode an mRNA or protein product (*e.g*., promoter sequence, polyadenylation sequence, termination sequence, enhancer sequence, *etc.*).

As used herein, the term "protein of interest" refers to a protein encoded by a nucleic acid of interest.

As used herein, the term "signal protein" refers to a protein that is co-expressed with a protein of interest and which, when detected by a suitable assay, provides indirect evidence of expression of the protein of interest. Examples of signal protein useful in the present invention include, but are not limited to, immunoglobulin heavy and light chains, beta-galactosidase, beta-lactamase, green fluorescent protein, and luciferase.

As used herein, the term "exogenous gene" refers to a gene that is not naturally present in a host organism or cell, or is artificially introduced into a host organism or cell.

The term "gene" refers to a nucleic acid (*e.g.,* DNA or RNA) sequence that comprises coding sequences necessary for the production of a polypeptide or precursor (*e.g*., proinsulin). The polypeptide can be encoded by a full length coding sequence or by any portion of the coding sequence so long as the desired activity or functional properties (*e.g*., enzymatic activity, ligand binding, signal transduction, etc.) of the full-length or fragment are retained. The term also encompasses the coding region of a structural gene and includes sequences located adjacent to the coding region on both the 5' and 3' ends for a distance of about 1 kb or more on either end such that the gene corresponds to the length of the full-length mRNA. The sequences that are located 5' of the coding region and which are present on the mRNA are referred to as 5' untranslated sequences. The sequences that are located 3' or downstream of the coding region and which are present on the mRNA are referred to as 3' untranslated sequences. The term "gene" encompasses both cDNA and genomic forms of a gene. A genomic form or clone of a gene contains the coding region interrupted with non-coding sequences termed "introns" or "intervening regions" or "intervening sequences." Introns are segments of a gene which are transcribed into nuclear RNA (hnRNA); introns may contain regulatory elements such as enhancers. Introns are removed or "spliced out" from the nuclear or primary transcript; introns therefore are absent in the messenger RNA (mRNA) transcript. The mRNA functions during translation to specify the sequence or order of amino acids in a nascent polypeptide.

As used herein, the term "gene expression" refers to the process of converting genetic information encoded in a gene into RNA (*e.g.*, mRNA, rRNA, tRNA, or snRNA) through "transcription" of the gene (*i.e*., via the enzymatic action of an RNA polymerase), and for protein encoding genes, into protein through "translation" of mRNA. Gene expression can be regulated at many stages in the process. "Up-regulation" or "activation" refers to regulation that increases the production of gene expression products (*i.e*., RNA or protein), while "down-regulation" or "repression" refers to regulation that decrease production. Molecules (*e.g*., transcription factors) that are involved in up-regulation or down-regulation are often called "activators" and "repressors," respectively.

Where "amino acid sequence" is recited herein to refer to an amino acid sequence of a naturally occurring protein molecule, "amino acid sequence" and like terms, such as "polypeptide" or "protein" are not meant to limit the amino acid sequence to the complete, native amino acid sequence associated with the recited protein molecule.

As used herein, the terms "nucleic acid molecule encoding," "DNA sequence encoding," "DNA encoding," "RNA sequence encoding," and "RNA encoding" refer to the order or sequence of deoxyribonucleotides or ribonucleotides along a strand of deoxyribonucleic acid or ribonucleic acid. The order of these deoxyribonucleotides or ribonucleotides determines the order of amino acids along the polypeptide (protein) chain. The DNA or RNA sequence thus codes for the amino acid sequence.

As used herein, the term "variant," when used in reference to a protein, refers to proteins encoded by partially homologous nucleic acids so that the amino acid sequence of the proteins varies. As used herein, the term "variant" encompasses proteins encoded by homologous genes having both conservative and nonconservative amino acid substitutions that do not result in a change in protein function, as well as proteins encoded by homologous genes having amino acid substitutions that cause decreased (*e.g*., null mutations) protein function or increased protein function.

As used herein, the terms "complementary" or "complementarity" are used in reference to polynucleotides (*i.e*., a sequence of nucleotides) related by the base-pairing rules. For example, for the sequence "A-G-T," is complementary to the sequence "T-C-A." Complementarity may be "partial," in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridization between nucleic acid strands. This is of particular importance in amplification reactions, as well as detection methods that depend upon binding between nucleic acids.

The terms "homology" and "percent identity" when used in relation to nucleic acids refers to a degree of complementarity. There may be partial homology (*i.e*., partial identity) or complete homology (*i.e*., complete identity). A partially complementary sequence is one that at least partially inhibits a completely complementary sequence from hybridizing to a target nucleic acid sequence and is referred to using the functional term "substantially homologous." The inhibition of hybridization of the completely complementary sequence to the target sequence may be examined using a hybridization assay (Southern or Northern blot, solution hybridization and the like) under conditions of low stringency. A substantially homologous sequence or probe (*i.e*., an oligonucleotide which is capable of hybridizing to another oligonucleotide of interest) will compete for and inhibit the binding (*i.e*., the hybridization) of a completely homologous sequence to a target sequence under conditions of low stringency. This is not to say that conditions of low stringency are such that non-specific binding is permitted; low stringency conditions require that the binding of two sequences to one another be a specific (*i.e.*, selective) interaction. The absence of non-specific binding may be tested by the use of a second target which lacks even a partial degree of complementarity (*e.g*., less than about 30% identity); in the absence of non-specific binding the probe will not hybridize to the second non-complementary target.

The art knows well that numerous equivalent conditions may be employed to comprise low stringency conditions; factors such as the length and nature (DNA, RNA, base composition) of the probe and nature of the target (DNA, RNA, base composition, present in solution or immobilized, etc.) and the concentration of the salts and other components (*e.g*., the presence or absence of formamide, dextran sulfate, polyethylene glycol) are considered and the hybridization solution may be varied to generate conditions of low stringency hybridization different from, but equivalent to, the above listed conditions. In addition, the art knows conditions that promote hybridization under conditions of high stringency (*e.g*., increasing the temperature of the hybridization and/or wash steps, the use of formamide in the hybridization solution, etc.).

When used in reference to a double-stranded nucleic acid sequence such as a cDNA or genomic clone, the term "substantially homologous" refers to any probe that can hybridize to either or both strands of the double-stranded nucleic acid sequence under conditions of low stringency as described above.

When used in reference to a single-stranded nucleic acid sequence, the term "substantially homologous" refers to any probe that can hybridize (*i.e*., it is the complement of) the single-stranded nucleic acid sequence under conditions of low stringency as described above.

As used herein, the term "hybridization" is used in reference to the pairing of complementary nucleic acids. Hybridization and the strength of hybridization (*i.e*., the strength of the association between the nucleic acids) is impacted by such factors as the degree of complementary between the nucleic acids, stringency of the conditions involved, the Tₘ of the formed hybrid, and the G:C ratio within the nucleic acids. A single molecule that contains pairing of complementary nucleic acids within its structure is said to be "self-hybridized."

As used herein, the term "Tₘ" is used in reference to the "melting temperature" of a nucleic acid. The melting temperature is the temperature at which a population of double-stranded nucleic acid molecules becomes half dissociated into single strands. The equation for calculating the Tₘ of nucleic acids is well known in the art. As indicated by , standard references, a simple estimate of the Tₘ value may be calculated by the equation: Tₘ = 81.5 + 0.41(% G + C), when a nucleic acid is in aqueous solution at 1 M NaCl (*See e.g.,* Anderson and Young, Quantitative Filter Hybridization, in Nucleic Acid Hybridization [1985]). Other references include more sophisticated computations that take structural as well as sequence characteristics into account for the calculation of Tₘ.

As used herein the term "stringency" is used in reference to the conditions of temperature, ionic strength, and the presence of other compounds such as organic solvents, under which nucleic acid hybridizations are conducted. With "high stringency" conditions, nucleic acid base pairing will occur only between nucleic acid fragments that have a high frequency of complementary base sequences. Thus, conditions of "weak" or "low" stringency are often required with nucleic acids that are derived from organisms that are genetically diverse, as the frequency of complementary sequences is usually less.

"High stringency conditions" when used in reference to nucleic acid hybridization comprise conditions equivalent to binding or hybridization at 42°C in a solution consisting of 5X SSPE (43.8 g/l NaCl, 6.9 g/l NaH₂PO₄·H₂O and 1.85 g/l EDTA, pH adjusted to 7.4 with NaOH), 0.5% SDS, 5X Denhardt's reagent and 100 µg/ml denatured salmon sperm DNA followed by washing in a solution comprising 0.1X SSPE, 1.0% SDS at 42°C when a probe of about 500 nucleotides in length is employed.

"Medium stringency conditions" when used in reference to nucleic acid hybridization comprise conditions equivalent to binding or hybridization at 42°C in a solution consisting of 5X SSPE (43.8 g/l NaCl, 6.9 g/l NaH₂PO₄·H₂O and 1.85 g/l EDTA, pH adjusted to 7.4 with NaOH), 0.5% SDS, 5X Denhardt's reagent and 100 µg/ml denatured salmon sperm DNA followed by washing in a solution comprising 1.0X SSPE, 1.0% SDS at 42°C when a probe of about 500 nucleotides in length is employed.

"Low stringency conditions" comprise conditions equivalent to binding or hybridization at 42°C in a solution consisting of 5X SSPE (43.8 g/l NaCl, 6.9 g/l NaH₂PO₄·H₂O and 1.85 g/l EDTA, pH adjusted to 7.4 with NaOH), 0.1% SDS, 5X Denhardt's reagent [50X Denhardt's contains per 500 ml: 5 g Ficoll (Type 400, Pharamcia), 5 g BSA (Fraction V; Sigma)] and 100 µg/ml denatured salmon sperm DNA followed by washing in a solution comprising 5X SSPE, 0.1% SDS at 42°C when a probe of about 500 nucleotides in length is employed.

A gene may produce multiple RNA species that are generated by differential splicing of the primary RNA transcript. cDNAs that are splice variants of the same gene will contain regions of sequence identity or complete homology (representing the presence of the same exon or portion of the same exon on both cDNAs) and regions of complete non-identity (for example, representing the presence of exon "A" on cDNA 1 wherein cDNA 2 contains exon "B" instead). Because the two cDNAs contain regions of sequence identity they will both hybridize to a probe derived from the entire gene or portions of the gene containing sequences found on both cDNAs; the two splice variants are therefore substantially homologous to such a probe and to each other.

The terms "in operable combination," "in operable order," and "operably linked" as used herein refer to the linkage of nucleic acid sequences in such a manner that a nucleic acid molecule capable of directing the transcription of a given gene and/or the synthesis of a desired protein molecule is produced. The term also refers to the linkage of amino acid sequences in such a manner so that a functional protein is produced.

As used herein, the term "selectable marker" refers to a gene that encodes an enzymatic activity that confers the ability to grow in medium lacking what would otherwise be an essential nutrient (*e.g*. the *HIS3* gene in yeast cells); in addition, a selectable marker may confer resistance to an antibiotic or drug upon the cell in which the selectable marker is expressed. Selectable markers may be "dominant"; a dominant selectable marker encodes an enzymatic activity that can be detected in any eukaryotic cell line. Examples of dominant selectable markers include the bacterial aminoglycoside 3' phosphotransferase gene (also referred to as the *neo* gene) that confers resistance to the drug G418 in mammalian cells, the bacterial hygromycin G phosphotransferase *(hyg)* gene that confers resistance to the antibiotic hygromycin and the bacterial xanthine-guanine phosphoribosyl transferase gene (also referred to as the *gpt* gene) that confers the ability to grow in the presence of mycophenolic acid. Other selectable markers are not dominant in that their use must be in conjunction with a cell line that lacks the relevant enzyme activity. Examples of non-dominant selectable markers include the thymidine kinase (*tk*) gene that is used in conjunction with *tk⁻* cell lines, the CAD gene which is used in conjunction with CAD-deficient cells and the mammalian hypoxanthine-guanine phosphoribosyl transferase (*hprt*) gene which is used in conjunction with *hprt⁻* cell lines. A review of the use of selectable markers in mammalian cell lines is provided in Sambrook, J. et al., Molecular Cloning: A Laboratory Manual, 2nd ed., Cold Spring Harbor Laboratory Press, New York (1989) pp.16.9-16.15.

As used herein, the term "regulatory element" refers to a genetic element which controls some aspect of the expression of nucleic acid sequences. For example, a promoter is a regulatory element that facilitates the initiation of transcription of an operably linked coding region. Other regulatory elements are splicing signals, polyadenylation signals, termination signals, RNA export elements, internal ribosome entry sites, etc. (defined *infra*).

Transcriptional control signals in eukaryotes comprise "promoter" and "enhancer" elements. Promoters and enhancers consist of short arrays of DNA sequences that interact specifically with cellular proteins involved in transcription (Maniatis et al., Science 236:1237 [1987]). Promoter and enhancer elements have been isolated from a variety of eukaryotic sources including genes in yeast, insect and mammalian cells, and viruses (analogous control elements, i.e., promoters, are also found in prokaryotes). The selection of a particular promoter and enhancer depends on what cell type is to be used to express the protein of interest. Some eukaryotic promoters and enhancers have a broad host range while others are functional in a limited subset of cell types (for review see, Voss et al., Trends Biochem. Sci., 11:287 [1986]; and Maniatis *et al., supra*). For example, the SV40 early gene enhancer is very active in a wide variety of cell types from many mammalian species and has been widely used for the expression of proteins in mammalian cells (Dijkema et al., EMBO J. 4:761 [1985]). Two other examples of promoter/enhancer elements active in a broad range of mammalian cell types are those from the human elongation factor 1α gene (Uetsuki et al., J. Biol. Chem., 264:5791 [1989]; Kim et al., Gene 91:217 [1990]; and Mizushima and Nagata, Nuc. Acids. Res., 18:5322 [1990]) and the long terminal repeats of the Rous sarcoma virus (Gorman et al., Proc. Natl. Acad. Sci. USA 79:6777 [1982]) and the human cytomegalovirus (Boshart et al., Cell 41:521 [1985]).

As used herein, the term "promoter/enhancer" denotes a segment of DNA which contains sequences capable of providing both promoter and enhancer functions (*i.e*., the functions provided by a promoter element and an enhancer element, see above for a discussion of these functions). For example, the long terminal repeats of retroviruses contain both promoter and enhancer functions. The enhancer/promoter may be "endogenous" or "exogenous" or "heterologous." An "endogenous" enhancer/promoter is one which is naturally linked with a given gene in the genome. An "exogenous" or "heterologous" enhancer/promoter is one which is placed in juxtaposition to a gene by means of genetic manipulation (*i.e*., molecular biological techniques such as cloning and recombination) such that transcription of that gene is directed by the linked enhancer/promoter.

Regulatory elements may be tissue specific or cell specific. The term "tissue specific" as it applies to a regulatory element refers to a regulatory element that is capable of directing selective expression of a nucleotide sequence of interest to a specific type of tissue (*e.g*., liver) in the relative absence of expression of the same nucleotide sequence of interest in a different type of tissue (*e.g.*, lung).

Tissue specificity of a regulatory element may be evaluated by, for example, operably linking a reporter gene to a promoter sequence (which is not tissue-specific) and to the regulatory element to generate a reporter construct, introducing the reporter construct into the genome of an animal such that the reporter construct is integrated into every tissue of the resulting transgenic animal, and detecting the expression of the reporter gene (*e.g.*, detecting mRNA, protein, or the activity of a protein encoded by the reporter gene) in different tissues of the transgenic animal. The detection of a greater level of expression of the reporter gene in one or more tissues relative to the level of expression of the reporter gene in other tissues shows that the regulatory element is "specific" for the tissues in which greater levels of expression are detected. Thus, the term "tissue-specific" (*e.g*., liver-specific) as used herein is a relative term that does not require absolute specificity of expression. In other words, the term "tissue-specific" does not require that one tissue have extremely high levels of expression and another tissue have no expression. It is sufficient that expression is greater in one tissue than another. By contrast, "strict" or "absolute" tissue-specific expression is meant to indicate expression in a single tissue type (*e.g.*, liver) with no detectable expression in other tissues.

The term "cell type specific" as applied to a regulatory element refers to a regulatory element which is capable of directing selective expression of a nucleotide sequence of interest in a specific type of cell in the relative absence of expression of the same nucleotide sequence of interest in a different type of cell within the same tissue. The term "cell type specific" when applied to a regulatory element also means a regulatory element capable of promoting selective expression of a nucleotide sequence of interest in a region within a single tissue.

Cell type specificity of a regulatory element may be assessed using methods well known in the art (*e.g.*, immunohistochemical staining and/or Northern blot analysis). Briefly, for immunohistochemical staining, tissue sections are embedded in paraffin, and paraffin sections are reacted with a primary antibody specific for the polypeptide product encoded by the nucleotide sequence of interest whose expression is regulated by the regulatory element. A labeled (*e.g*., peroxidase conjugated) secondary antibody specific for the primary antibody is allowed to bind to the sectioned tissue and specific binding detected (*e.g*., with avidin/biotin) by microscopy. Briefly, for Northern blot analysis, RNA is isolated from cells and electrophoresed on agarose gels to fractionate the RNA according to size followed by transfer of the RNA from the gel to a solid support (*e.g*., nitrocellulose or a nylon membrane). The immobilized RNA is then probed with a labeled oligo-deoxyribonucleotide probe or DNA probe to detect RNA species complementary to the probe used. Northern blots are a standard tool of molecular biologists.

The term "promoter," "promoter element," or "promoter sequence" as used herein, refers to a DNA sequence which when ligated to a nucleotide sequence of interest is capable of controlling the transcription of the nucleotide sequence of interest into mRNA. A promoter is typically, though not necessarily, located 5' (*i.e*., upstream) of a nucleotide sequence of interest whose transcription into mRNA it controls, and provides a site for specific binding by RNA polymerase and other transcription factors for initiation of transcription.

Promoters may be constitutive or regulatable. The term "constitutive" when made in reference to a promoter means that the promoter is capable of directing transcription of an operably linked nucleic acid sequence in the absence of a stimulus (*e.g.,* heat shock, chemicals, *etc*.). In contrast, a "regulatable" promoter is one which is capable of directing a level of transcription of an operably linked nucleic acid sequence in the presence of a stimulus (*e.g.,* heat shock, chemicals, *etc*.) which is different from the level of transcription of the operably linked nucleic acid sequence in the absence of the stimulus.

The presence of "splicing signals" on an expression vector often results in higher levels of expression of the recombinant transcript. Splicing signals mediate the removal of introns from the primary RNA transcript and consist of a splice donor and acceptor site (Sambrook et al., Molecular Cloning: A Laboratory Manual, 2nd ed., Cold Spring Harbor Laboratory Press, New York [1989], pp. 16.7-16.8). A commonly used splice donor and acceptor site is the splice junction from the 16S RNA of SV40.

Efficient expression of recombinant DNA sequences in eukaryotic cells requires expression of signals directing the efficient termination and polyadenylation of the resulting transcript. Transcription termination signals are generally found downstream of the polyadenylation signal and are a few hundred nucleotides in length. The term "poly A site" or "poly A sequence" as used herein denotes a DNA sequence that directs both the termination and polyadenylation of the nascent RNA transcript. Efficient polyadenylation of the recombinant transcript is desirable as transcripts lacking a poly A tail are unstable and are rapidly degraded. The poly A signal utilized in an expression vector may be "heterologous" or "endogenous." An endogenous poly A signal is one that is found naturally at the 3' end of the coding region of a given gene in the genome. A heterologous poly A signal is one that is isolated from one gene and placed 3' of another gene. A commonly used heterologous poly A signal is the SV40 poly A signal. The SV40 poly A signal is contained on a 237 bp *Bam*HI/*Bcl*I restriction fragment and directs both termination and polyadenylation (Sambrook, *supra,* at 16.6-16.7).

Eukaryotic expression vectors may also contain "viral replicons "or "viral origins of replication." Viral replicons are viral DNA sequences that allow for the extrachromosomal replication of a vector in a host cell expressing the appropriate replication factors. Vectors that contain either the SV40 or polyoma virus origin of replication replicate to high "copy number" (up to 10⁴ copies/cell) in cells that express the appropriate viral T antigen. Vectors that contain the replicons from bovine papillomavirus or Epstein-Barr virus replicate extrachromosomally at "low copy number" (-100 copies/cell). However, it is not intended that expression vectors be limited to any particular viral origin of replication.

As used herein, the term "long terminal repeat" of "LTR" refers to transcriptional control elements located in or isolated from the U3 region 5' and 3' of a retroviral genome. As is known in the art, long terminal repeats may be used as control elements in retroviral vectors, or isolated from the retroviral genome and used to control expression from other types of vectors.

As used herein; the term "secretion signal" refers to any DNA sequence which when operably linked to a recombinant DNA sequence encodes a signal peptide which is capable of causing the secretion of the recombinant polypeptide. In general, the signal peptides comprise a series of about 15 to 30 hydrophobic amino acid residues *(See, e.g.,* Zwizinski et al., J. Biol. Chem. 255(16): 7973-77 [1980], Gray et al., Gene 39(2): 247-54 [1985], and Martial et al., Science 205: 602-607 [1979]). Such secretion signal sequences are preferably derived from genes encoding polypeptides secreted from the cell type targeted for tissue-specific expression (*e.g*., secreted milk proteins for expression in and secretion from mammary secretory cells). Secretory DNA sequences, however, are not limited to such sequences. Secretory DNA sequences from proteins secreted from many cell types and organisms may also be used (*e.g*., the secretion signals for t-PA, serum albumin, lactoferrin, and growth hormone, and secretion signals from microbial genes encoding secreted polypeptides such as from yeast, filamentous fungi, and bacteria).

As used herein, the terms "RNA export element" or "Pre-mRNA Processing Enhancer (PPE)" refer to 3' and 5' cis-acting post-transcriptional regulatory elements that enhance export of RNA from the nucleus. "PPE" elements include, but are not limited to Mertz sequences (described in U.S. Pat. Nos. 5,914,267 and 5,686,120) and woodchuck mRNA processing enhancer (WPRE; WO99/14310 and U.S. Pat. No. 6,136,597).

As used herein, the term "polycistronic" refers to an mRNA encoding more than polypeptide chain (*See, e.g*., WO 93/03143, WO 88/05486, and European Pat. No. 117058). Likewise, the term "arranged in polycistronic sequence" refers to the arrangement of genes encoding two different polypeptide chains in a single mRNA.

As used herein, the term "internal ribosome entry site" or "IRES" refers to a sequence located between polycistronic genes that permits the production of the expression product originating from the second gene by internal initiation of the translation of the dicistronic mRNA. Examples of internal ribosome entry sites include, but are not limited to, those derived from foot and mouth disease virus (FDV), encephalomyocarditis virus, poliovirus and RDV (Scheper et al., Biochem. 76: 801-809 [1994]; Meyer et al., J. Virol. 69: 2819-2824 [1995]; Jang et al., 1988, J. Virol. 62: 2636-2643 [1998]; Haller et al., J. Virol. 66: 5075-5086 [1995]). Vectors incorporating IRES's may be assembled as is known in the art. For example, a retroviral vector containing a polycistronic sequence may contain the following elements in operable association: nucleotide polylinker, gene of interest, an internal ribosome entry site and a mammalian selectable marker or another gene of interest. The polycistronic cassette is situated within the retroviral vector between the 5' LTR and the 3' LTR at a position such that transcription from the 5' LTR promoter transcribes the polycistronic message cassette. The transcription of the polycistronic message cassette may also be driven by an internal promoter (*e.g*., cytomegalovirus promoter) or an inducible promoter, which may be preferable depending on the use. The polycistronic message cassette can further comprise a cDNA or genomic DNA (gDNA) sequence operatively associated within the polylinker. Any mammalian selectable marker can be utilized as the polycistronic message cassette mammalian selectable marker. Such mammalian selectable markers are well known to those of skill in the art and can include, but are not limited to, kanamycin/G418, hygromycin B or mycophenolic acid resistance markers.

As used herein, the term "retrovirus" refers to a retroviral particle which is capable of entering a cell (*i.e.,* the particle contains a membrane-associated protein such as an envelope protein or a viral G glycoprotein which can bind to the host cell surface and facilitate entry of the viral particle into the cytoplasm of the host cell) and integrating the retroviral genome (as a double-stranded provirus) into the genome of the host cell. The term "retrovirus" encompasses Oncovirinae (*e.g*., Moloney murine leukemia virus (MoMOLV), Moloney murine sarcoma virus (MoMSV), and Mouse mammary tumor virus (MMTV), Spumavirinae, amd Lentivirinae (*e.g*., Human immunodeficiency virus, Simian immunodeficiency virus, Equine infection anemia virus, and Caprine arthritis-encephalitis virus; *See, e.g.,* U.S. Pat. Nos. 5,994,136 and 6,013,516).

As used herein, the term "retroviral vector" refers to a retrovirus that has been modified to express a gene of interest. Retroviral vectors can be used to transfer genes efficiently into host cells by exploiting the viral infectious process. Foreign or heterologous genes cloned (*i.e.*, inserted using molecular biological techniques) into the retroviral genome can be delivered efficiently to host cells which are susceptible to infection by the retrovirus. Through well known genetic manipulations, the replicative capacity of the retroviral genome can be destroyed. The resulting replication-defective vectors can be used to introduce new genetic material to a cell but they are unable to replicate. A helper virus or packaging cell line can be used to permit vector particle assembly and egress from the cell. Such retroviral vectors comprise a replication-deficient retroviral genome containing a nucleic acid sequence encoding at least one gene of interest (*i.e*, a polycistronic nucleic acid sequence can encode more than one gene of interest), a 5' retroviral long terminal repeat (5' LTR); and a 3' retroviral long terminal repeat (3' LTR).

The term "pseudotyped retroviral vector" refers to a retroviral vector containing a heterologous membrane protein. The term "membrane-associated protein" refers to a protein (*e.g*., a viral envelope glycoprotein or the G proteins of viruses in the Rhabdoviridae family such as VSV, Piry, Chandipura and Mokola) which are associated with the membrane surrounding a viral particle; these membrane-associated proteins mediate the entry of the viral particle into the host cell. The membrane associated protein may bind to specific cell surface protein receptors, as is the case for retroviral envelope proteins or the membrane-associated protein may interact with a phospholipid component of the plasma membrane of the host cell, as is the case for the G proteins derived from members of the Rhabdoviridae family.

The term "heterologous membrane-associated protein" refers to a membrane-associated protein which is derived from a virus which is not a member of the same viral class or family as that from which the nucleocapsid protein of the vector particle is derived. "Viral class or family" refers to the taxonomic rank of class or family, as assigned by the International Committee on Taxonomy of Viruses.

The term "Rhabdoviridae" refers to a family of enveloped RNA viruses that infect animals, including humans, and plants. The Rhabdoviridae family encompasses the genus Vesiculovirus which includes vesicular stomatitis virus (VSV), Cocal virus, Piry virus, Chandipura virus, and Spring viremia of carp virus (sequences encoding the Spring viremia of carp virus are available under GenBank accession number U18101). The G proteins of viruses in the Vesiculovirus genera are virally-encoded integral membrane proteins that form externally projecting homotrimeric spike glycoproteins complexes that are required for receptor binding and membrane fusion. The G proteins of viruses in the Vesiculovirus genera have a covalently bound palmititic acid (C₁₆) moiety. The amino acid sequences of the G proteins from the Vesiculoviruses are fairly well conserved. For example, the Piry virus G protein share about 38% identity and about 55% similarity with the VSV G proteins (several strains of VSV are known, *e.g*., Indiana, New Jersey, Orsay, San Juan, etc., and their G proteins are highly homologous). The Chandipura virus G protein and the VSV G proteins share about 37% identity and 52% similarity. Given the high degree of conservation (amino acid sequence) and the related functional characteristics (*e.g.,* binding of the virus to the host cell and fusion of membranes, including syncytia formation) of the G proteins of the Vesiculoviruses, the G proteins from non-VSV Vesiculoviruses may be used in place of the VSV G protein for the pseudotyping of viral particles. The G proteins of the Lyssa viruses (another genera within the Rhabdoviridae family) also share a fair degree of conservation with the VSV G proteins and function in a similar manner (*e.g*., mediate fusion of membranes) and therefore may be used in place of the VSV G protein for the pseudotyping of viral particles. The Lyssa viruses include the Mokola virus and the Rabies viruses (several strains of Rabies virus are known and their G proteins have been cloned and sequenced). The Mokola virus G protein shares stretches of homology (particularly over the extracellular and transmembrane domains) with the VSV G proteins which show about 31% identity and 48% similarity with the VSV G proteins. Preferred G proteins share at least 25% identity, preferably at least 30% identity and most preferably at least 35% identity with the VSV G proteins. The.VSV G protein from which New Jersey strain (the sequence of this G protein is provided in GenBank accession numbers M27165 and M21557) is employed as the reference VSV G protein.

As used herein, the term "lentivirus vector" refers to retroviral vectors derived from the Lentiviridae family (*e.g*., human immunodeficiency virus, simian immunodeficiency virus, equine infectious anemia virus, and caprine arthritis-encephalitis virus) that are capable of integrating into non-dividing cells (*See, e.g.,* U.S. Pat. Nos. 5,994,136 and 6,013,516, both of which are incorporated herein by reference).

The term "pseudotyped lentivirus vector" refers to lentivirus vector containing a heterologous membrane protein (*e.g*., a viral envelope glycoprotein or the G proteins of viruses in the Rhabdoviridae family such as VSV, Piry, Chandipura and Mokola).

As used herein the term, the term *"in vitro"* refers to an artificial environment and to processes or reactions that occur within an artificial environment. *In vitro* environments can consist of, but are not limited to, test tubes and cell cultures. The term *"in vivo"* refers to the natural environment (*e.g*., an animal or a cell) and to processes or reaction that occur within a natural environment.

As used herein, the term "clonally derived" refers to a cell line that it derived from a single cell.

As used herein, the term "non-clonally derived" refers to a cell line that is derived from more than one cell.

As used herein, the term "passage" refers to the process of diluting a culture of cells that has grown to a particular density or confluency (*e.g*., 70% or 80% confluent), and then allowing the diluted cells to regrow to the particular density or confluency desired (*e.g*., by replating the cells or establishing a new roller bottle culture with the cells.

As used herein, the term "stable," when used in reference to genome, refers to the stable maintenance of the information content of the genome from one generation to the next, or, in the particular case of a cell line, from one passage to the next. Accordingly, a genome is considered to be stable if no gross changes occur in the genome (*e.g*., a gene is deleted or a chromosomal translocation occurs). The term "stable" does not exclude subtle changes that may occur to the genome such as point mutations.

As used herein, the term "response," when used in reference to an assay, refers to the generation of a detectable signal (*e.g*., accumulation of reporter protein, increase in ion concentration, accumulation of a detectable chemical product).

As used herein, the term "membrane receptor protein" refers to membrane spanning proteins that bind a ligand (*e.g*., a hormone or neurotransmitter). As is known in the art, protein phosphorylation is a common regulatory mechanism used by cells to selectively modify proteins carrying regulatory signals from outside the cell to the nucleus. The proteins that execute these biochemical modifications are a group of enzymes known as protein kinases. They may further be defined by the substrate residue that they target for phosphorylation. One group of protein kinases are the tyrosine kinases (TKs) which selectively phosphorylate a target protein on its tyrosine residues. Some tyrosine kinases are membrane-bound receptors (RTKs), and, upon activation by a ligand, can autophosphorylate as well as modify substrates. The initiation of sequential phosphorylation by ligand stimulation is a paradigm that underlies the action of such effectors as, for example, epidermal growth factor (EGF), insulin, platelet-derived growth factor (PDGF), and fibroblast growth factor (FGF). The receptors for these ligands are tyrosine kinases and provide the interface between the binding of a ligand (hormone, growth factor) to a target cell and the transmission of a signal into the cell by the activation of one or more biochemical pathways. Ligand binding to a receptor tyrosine kinase activates its intrinsic enzymatic activity *(See, e.g.,* Ullrich and Schlessinger, Cell 61:203-212 [1990]). Tyrosine kinases can also be cytoplasmic, non-receptor-type enzymes and act as a downstream component of a signal transduction pathway.

As used herein, the term "signal transduction protein" refers to a proteins that are activated or otherwise effected by ligand binding to a membrane receptor protein or some other stimulus. Examples of signal transduction protein include adenyl cyclase, phospholipase C, and G-proteins. Many membrane receptor proteins are coupled to G-proteins (*i.e*., G-protein coupled receptors (GPCRs); for a review, *see* Neer, 1995, Cell 80:249-257 [1995]). Typically, GPCRs contain seven transmembrane domains. Putative GPCRs can be identified on the basis of sequence homology to known GPCRs.

GPCRs mediate signal transduction across a cell membrane upon the binding of a ligand to an extracellular portion of a GPCR. The intracellular portion of a GPCR interacts with a G-protein to modulate signal transduction from outside to inside a cell. A GPCR is therefore said to be "coupled" to a G-protein. G-proteins are composed of three polypeptide subunits: an α subunit, which binds and hydrolyses GTP, and a dimeric βγ subunit. In the basal, inactive state, the G-protein exists as a heterotrimer of the α and βγ subunits. When the G-protein is inactive, guanosine diphosphate (GDP) is associated with the α subunit of the G-protein. When a GPCR is bound and activated by a ligand, the GPCR binds to the G-protein heterotrimer and decreases the affinity of the Gα subunit for GDP. In its active state, the G subunit exchanges GDP for guanine triphosphate (GTP) and active Gα subunit disassociates from both the receptor and the dimeric βγ subunit. The disassociated, active Gα subunit transduces signals to effectors that are "downstream" in the G-protein signalling pathway within the cell. Eventually, the G-protein's endogenous GTPase activity returns active G subunit to its inactive state, in which it is associated with GDP and the dimeric βγ subunit.

Numerous members of the heterotrimeric G-protein family have been cloned, including more than 20 genes encoding various Gα subunits. The various G subunits have been categorized into four families, on the basis of amino acid sequences and functional homology. These four families are termed Gαₛ, Gaᵢ, Ga_{q}, and Ga₁₂. Functionally, these four families differ with respect to the intracellular signaling pathways that they activate and the GPCR to which they couple.

For example, certain GPCRs normally couple with Gαₛ and, through Gαₛ, these GPCRs stimulate adenylyl cyclase activity. Other GPCRs normally couple with GGα_{q}, and through GGα_{q}, these GPCRs can activate phospholipase C (PLC), such as the β isoform of phospholipase C (*i.e*., PLCβ, Stermweis and Smrcka, Trends in Biochem. Sci. 17:502-506 [1992]).

As used herein, the term "nucleic acid binding protein" refers to proteins that bind to nucleic acid, and in particular to proteins that cause increased *(i.e.,* activators or transcription factors) or decreased (*i.e*., inhibitors) transcription from a gene.

As used herein, the term "ion channel protein" refers to proteins that control the ingress or egress of ions across cell membranes. Examples of ion channel proteins include, but are not limited to, the Na⁺-K⁺ ATPase pump, the Ca²⁺ pump, and the K⁺ leak channel.

As used herein, the term "protein kinase" refers to proteins that catalyze the addition of a phosphate group from a nucleoside triphosphate to an amino acid side chain in a protein. Kinases comprise the largest known enzyme superfamily and vary widely in their target proteins. Kinases may be categorized as protein tyrosine kinases (PTKs), which phosphorylate tyrosine residues, and protein serine/threonine kinases (STKs), which phosphorylate serine and/or threonine residues. Some kinases have dual specificity for both serine/threonine and tyrosine residues. Almost all kinases contain a conserved 250-300 amino acid catalytic domain. This domain can be further divided into 11 subdomains. N-terminal subdomains I-IV fold into a two-lobed structure which binds and orients the ATP donor molecule, and subdomain V spans the two lobes. C-terminal subdomains VI-XI bind the protein substrate and transfer the gamma phosphate from ATP to the hydroxyl group of a serine, threonine, or tyrosine residue. Each of the 11 subdomains contains specific catalytic residues or amino acid motifs characteristic of that subdomain. For example, subdomain I contains an 8-amino acid glycine-rich ATP binding consensus motif, subdomain II contains a critical lysine residue required for maximal catalytic activity, and subdomains VI through IX comprise the highly conserved catalytic core. STKs and PTKs also contain distinct sequence motifs in subdomains VI and VIII which may confer hydroxyamino acid specificity. Some STKs and PTKs possess structural characteristics of both families. In addition, kinases may also be classified by additional amino acid sequences, generally between 5 and 100 residues, which either flank or occur within the kinase domain.

Non-transmembrane PTKs form signaling complexes with the cytosolic domains of plasma membrane receptors. Receptors that signal through non-transmembrane PTKs include cytokine, hormone, and antigen-specific lymphocytic receptors. Many PTKs were first identified as oncogene products in cancer cells in which PTK activation was no longer subject to normal cellular controls. In fact, about one third of the known oncogenes encode PTKs. Furthermore, cellular transformation (oncogenesis) is often accompanied by increased tyrosine phosphorylation activity *(See, e.g.,* Carbonneau, H. and Tonics, Annu. Rev. Cell Biol. 8:463-93 [1992]). Regulation of PTIC activity may therefore be an important strategy in controlling some types of cancer.

Examples of protein kinases include, but are not limited to, cAMP-dependent protein kinase, protein kinase C, and cyclin-dependent protein kinases, *(See, e.g.,* U.S. Pat. Nos. 6,034,228; 6,030,822; 6,030,788; 6,020,306; 6,013,455; 6,013,464; and 6,015,807).

As used herein, the term "protein phosphatase" refers to proteins that remove a phosphate group from a protein. Protein phosphatases are generally divided into two groups, receptor and non-receptor type proteins. Most receptor-type protein tyrosine phosphatases contain two conserved catalytic domains, each of which encompasses a segment of 240 amino acid residues. *(See, e.g.,* Saito et al., Cell Growth and Diff. 2:59-65 [1991]). Receptor protein tyrosine phosphatases can be subclassified further based upon the amino acid sequence diversity of their extracellular domains. *(See, e.g.,* Krueger et al., Proc. Natl. Acad. Sci. USA 89:7417-7421 [1992]). Examples of protein phosphatases include, but are not limited to, cdc25 a, b, and c, PTP20, PTP1D, and PTPλ *(See, e.g.,* U.S. Pat. Nos. 5,976,853; 5,994,074; 6,004,791; 5,981,251; 5,976,852; 5,958,719; 5,955,592; and 5,952,212).

As used herein, the term "protein encoded by an oncogene" refers to proteins that cause, either directly or indirectly, the neoplastic transformation of a host cell. Examples of oncogenes include, but are not limited to, the following genes: *src, fps, fes, fgr, ros, H-ras, abl, ski, erbA, erbB, fms, fos, mos, sis, myc, myb, rel, kit, raf, K-ras, and ets.*

As used herein, the term "immunoglobulin" refers to proteins which bind a specific antigen. Immunoglobulins include, but are not limited to, polyclonal, monoclonal, chimeric, and humanized antibodies, Fab fragments, F(ab')2 fragments, and includes immunoglobulins of the following classes: IgG, IgA, IgM, IgD, IbE, and secreted immunoglobulins (sIg). Immunoglobulins generally comprise two identical heavy chains (γ, α, µ, δ, or ε) and two light chains (κ or λ).

As used herein, the term "antigen binding protein" refers to proteins which bind to a specific antigen. "Antigen binding proteins" include, but are not limited to, immunoglobulins, including polyclonal, monoclonal, chimeric, and humanized antibodies; Fab fragments, F(ab')2 fragments, and Fab expression libraries; and single chain antibodies. Various procedures known in the art are used for the production of polyclonal antibodies. For the production of an antibody, various host animals can be immunized by injection with the peptide corresponding to the desired epitope including but not limited to rabbits, mice, rats, sheep, goats, etc. In a preferred embodiment, the peptide is conjugated to an immunogenic carrier (*e.g*., diphtheria toxoid, bovine serum albumin (BSA), or keyhole limpet hemocyanin (KLH)). Various adjuvants are used to increase the immunological response, depending on the host species, including but not limited to Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (Bacille Calmette-Guerin) and *Corynebacterium parvum.*

For preparation of monoclonal antibodies, any technique that provides for the production of antibody molecules by continuous cell lines in culture may be used *(See, e.g.,* Harlow and Lane, Antibodies: A Laboratory Manual, Cold Spring Harbor Laboratory Press, Cold Spring Harbor, NY). These include, but are not limited to, the hybridoma technique originally developed by Köhler and Milstein (Köhler and Milstein, Nature 256:495-497 [1975]), as well as the trioma technique, the human B-cell hybridoma technique *(See e.g.,* Kozbor et al. Immunol. Today 4:72 [1983]), and the EBV-hybridoma technique to produce human monoclonal antibodies *(*Cole et al., in Monoclonal Antibodies and Cancer Therapy, Alan R Liss, Inc., pp. 77-96 [1985]).

According to the invention, techniques described for the production of single chain antibodies (U.S. Patent 4,946,778; herein-incorporated by reference) can be adapted to produce specific single chain antibodies as desired. An additional embodiment of the invention utilizes the techniques known in the art for the construction of Fab expression libraries (Huse et al., Science 246:1275-1281 [1989]) to allow rapid and easy identification of monoclonal Fab fragments with the desired specificity.

Antibody fragments that contain the idiotype (antigen binding region) of the antibody molecule can be generated by known techniques. For example, such fragments include but are not limited to: the F(ab')2 fragment that can be produced by pepsin digestion of an antibody molecule; the Fab' fragments that can be generated by reducing the disulfide bridges of an F(ab')2 fragment, and the Fab fragments that can be generated by treating an antibody molecule with papain and a reducing agent.

Genes encoding antigen binding proteins can be isolated by methods known in the art. In the production of antibodies, screening for the desired antibody can be accomplished by techniques known in the art (*e.g*., radioimmunoassay, ELISA (enzyme-linked immunosorbant assay), "sandwich" immunoassays, immunoradiometric assays, gel diffusion precipitin reactions, immunodiffusion assays, *in situ* immunoassays (using colloidal gold, enzyme or radioisotope labels, for example), Western Blots, precipitation reactions, agglutination assays (*e.g*., gel agglutination assays, hemagglutination assays, etc.), complement fixation assays, immunofluorescence assays, protein A assays, and immunoelectrophoresis assays, etc.) etc.

As used herein, the term "reporter gene" refers to a gene encoding a protein that may be assayed. Examples of reporter genes include, but are not limited to, luciferase *(See, e.g.,* de Wet et al., Mol. Cell. Biol. 7:725 [1987] and U.S. Pat Nos.,6,074,859; 5,976,796; 5,674,713; and 5,618,682) green fluorescent protein (*e.g*., GenBank Accession Number U43284; a number of GFP variants are commercially available from CLONTECH Laboratories, Palo Alto, CA), chloramphenicol acetyltransferase, β-galactosidase, alkaline phosphatase, and horse radish peroxidase.

As used herein, the term "purified" refers to molecules, either nucleic or amino acid sequences, that are removed from their natural environment, isolated or separated. An "isolated nucleic acid sequence" is therefore a purified nucleic acid sequence. "Substantially purified" molecules are at least 60% free, preferably at least 75% free, and more preferably at least 90% free from other components with which they are naturally associated.

The term "test compound" refers to any chemical entity, pharmaceutical, drug, and the like contemplated to be useful in the treatment and/or prevention of a disease, illness, sickness, or disorder of bodily function, or otherwise alter the physiological or cellular status of a sample. Test compounds comprise both known and potential therapeutic compound. A test compound can be determined to be therapeutic by screening using the screening methods of the present invention. A "known therapeutic compound" refers to a therapeutic compound that has been shown (*e.g*., through animal trials or prior experience with administration to humans) to be effective in such treatment or prevention.

### DETAILED DESCRIPTION OF THE INVENTION

The present invention is defined in the claims and relates to the production of proteins in host cells, and more particularly to host cells containing multiple integrated copies of an integrating vector. Integrating vectors (*i.e*., vectors that integrate via an integrase or transposase) are used to create cell lines containing a high copy number of a nucleic acid encoding a gene of interest. The transfected genomes of the high copy number cells are stable through repeated passages (*e.g*., at least 10 passages, preferably at least 50 passages, and most preferably at least 100 passages). Furthermore, the host cells of the present disclosure are capable of producing high levels of protein (*e.g*., more than 1 pg/cell/day, preferably more than 10 pg/cell/day, more preferably more than 50 pg/cell/day, and most preferably more than 100 pg/cell/day.)

The genomic stability and high expression levels of the host cells of the present disclosure provide distinct advantages over previously described methods of cell culture. For example, mammalian cell lines containing multiple copies of genes are known in the art to be intrinsically unstable. Indeed, this instability is a recognized problem facing researchers desiring to use mammalian cell lines for various purposes, including high throughout screening assays (*See*, *e.g*.,. Sittampalam et al., Curr. Opin. Chem. Biol. 1(3):384-91 [1997]).

It is not intended that the present invention be limited to particular mechanism of action. Indeed, an understanding of the mechanism is not necessary to make and use the present invention. However, the high genomic stability and protein expression levels of the host cells of the present disclosure are thought to be due to unique properties of the integrating vectors (*e.g*., retroviral vectors). For example, it is known that retroviruses are inherited elements in the germ line of many organisms. Indeed, as much as 5-10% of the mammalian genome may consist of elements contributed by reverse transcription, indicating a high degree of stability. Likewise, many of these types of vectors target active (*e.g*., DNase I hypersensitive sites) transcriptional sites in the genome.

Many investigations have focused on the deleterious effects of retroviral and transposon integration. The property of targeting active regions of the genome has led to the use of retroviral vectors and transposon vectors in promoter trap schemes and for saturation mutagenesis *(See, e.g.,* U.S. Pat. Nos. 5,627,058 and 5,922,601). In promoter trap schemes, the cells are infected with a promoterless reporter vector. If the promoterless vector integrates downstream of a promoter (*i.e*., into a gene), the reporter gene encoded by the vector is activated. The promoter can then be cloned and further characterized.

As can be seen, these schemes rely on the disruption of an endogenous gene. Therefore, it is surprising that the methods of the present disclosure, which utilize integrating vectors at high multiplicities of infection that would normally be thought to lead to gene disruption, led to the development of stable cell lines that express high quantities of a protein of interest. The development of these cell lines is described more fully below. The description is divided into the following sections: I) Host Cells; II) Vectors and Methods of Transfection; and III) Uses of Transfected Host Cells.

### I. Host Cells

The present disclosure contemplates the transfection of a variety of host cells with integrating vectors. A number of mammalian host cell lines are known in the art. In general, these host cells are capable of growth and survival when placed in either monolayer culture or in suspension culture in a medium containing the appropriate nutrients and growth factors, as is described in more detail below. Typically, the cells are capable of expressing and secreting large quantities of a particular protein of interest into the culture medium. Examples of suitable mammalian host cells include, but are not limited to Chinese hamster ovary cells (CHO-K1, ATCC CCl-61); bovine mammary epithelial cells (ATCC CRL 10274; bovine mammary epithelial cells); monkey kidney CV1 line transformed by SV40 (COS-7, ATCC CRL 1651); human embryonic kidney line (293 or 293 cells subcloned for growth in suspension culture; *see, e.g.,* Graham et al., J. Gen Virol., 36:59 [1977]); baby hamster kidney cells (BHK, ATCC CCL 10); mouse sertoli cells (TM4, Mather, Biol. Reprod. 23:243-251 [1980]); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci., 383:44-68 [1982]); MRC 5 cells; FS4 cells; rat fibroblasts (208F cells); MDBK cells (bovine kidney cells); and a human hepatoma line (Hep G2).

### II. Vectors and Methods for Transfection

According to the present disclosure, host cells such as those described above are transduced or transfected with integrating vectors particularly retroviral vectors and lentiviral vectors. The design, production, and use of these vectors in the present invention is described below.

### A. Retroviral Vectors

Retroviruses (family Retroviridae) are divided into three groups: the spumaviruses (*e.g*., human foamy virus); the lentiviruses (*e.g*., human immunodeficiency virus and sheep visna virus) and the oncoviruses (*e.g*., MLV, Rous sarcoma virus).

Retroviruses are enveloped (*i.e*., surrounded by a host cell-derived lipid bilayer membrane) single-stranded RNA viruses which infect animal cells. When a retrovirus infects a cell, its RNA genome is converted into a double-stranded linear DNA form (*i.e*., it is reverse transcribed). The DNA form of the virus is then integrated into the host cell genome as a provirus. The provirus serves as a template for the production of additional viral genomes and viral mRNAs. Mature viral particles containing two copies of genomic RNA bud from the surface of the infected cell. The viral particle comprises the genomic RNA, reverse transcriptase and other *pol* gene products inside the viral capsid (which contains the viral gag gene products) which is surrounded by a lipid bilayer membrane derived from the host cell containing the viral envelope glycoproteins (also referred to as membrane-associated proteins).

The organization of the genomes of numerous retroviruses is well known to the art and this has allowed the adaptation of the retroviral genome to produce retroviral vectors. The production of a recombinant retroviral vector_carrying a gene of interest is typically achieved in two stages.

First, the gene of interest is inserted into a retroviral vector which contains the sequences necessary for the efficient expression of the gene of interest (including promoter and/or enhancer elements which may be provided by the viral long terminal repeats (LTRs) or by an internal promoter/enhancer and relevant splicing signals), sequences required for the efficient packaging of the viral RNA into infectious virions (*e.g*., the packaging signal (Psi), the tRNA primer binding site (-PBS), the 3' regulatory sequences required for reverse transcription (+PBS)) and the viral LTRs. The LTRs contain sequences required for the association of viral genomic RNA, reverse transcriptase and integrase functions, and sequences involved in directing the expression of the genomic RNA to be packaged in viral particles. For safety reasons, many recombinant retroviral vectors lack functional copies of the genes which are essential for viral replication (these essential genes are either deleted or disabled); therefore, the resulting virus is said to be replication defective.

Second, following the construction of the recombinant vector, the vector DNA is introduced into a packaging cell line. Packaging cell lines provide proteins required in *trans* for the packaging of the viral genomic RNA into viral particles having the desired host range (*i.e*., the viral-encoded gag, pol and env proteins). The host range is controlled, in part, by the type of envelope gene product expressed on the surface of the viral particle. Packaging cell lines may express .ecotrophic, amphotropic or xenotropic envelope gene products. Alternatively, the packaging cell line may lack sequences encoding a viral envelope (env) protein. In this case the packaging cell line will package the viral genome into particles which lack a membrane-associated protein (*e.g*., an env protein). In order to produce viral particles containing a membrane associated protein which will permit entry of the virus into a cell, the packaging cell line containing the retroviral sequences is transfected with sequences encoding a membrane-associated protein (*e.g*., the G protein of vesicular stomatitis virus (VSV)). The transfected packaging cell will then produce viral particles which contain the membrane-associated protein expressed by the transfected packaging cell line; these viral particles which contain viral genomic RNA derived from one virus encapsidated by the envelope proteins of another virus are said to be pseudotyped virus particles.

The retroviral vectors of the present disclosure can be further modified to include additional regulatory sequences. As described above, the retroviral vectors of the present invention include the following elements in operable association: a) a 5' LTR; b) a packaging signal; c) a 3' LTR and d) a nucleic acid encoding a protein of interest located between the 5' and 3' LTRs. In some embodiments, the nucleic acid of interest may be arranged in opposite orientation to the 5' LTR when transcription from an internal promoter is desired. Suitable internal promoters include, but are not limited to, the alpha-lactalbumin promoter, the CMV promoter (human or ape), and the thymidine kinase promoter.

In other embodiments, where secretion of the protein of interest is desired, the vectors are modified by including a signal peptide sequence in operable association with the protein of interest. The sequences of several suitable signal peptides are known to those in the art, including, but not limited to, those derived from tissue plasminogen activator; human growth hormone, lactoferrin, alpha-casein, and alpha-lactalbumin.

In other embodiments the vectors are modified by incorporating an RNA export element *(See, e.g.,* U.S. Pat. Nos. 5,914,267; 6,136,597; and 5,686,120; and WO99/14310) either 3' or 5' to the nucleic acid sequence encoding the protein of interest. It is contemplated that the use of RNA export elements allows high levels of expression of the protein of interest without incorporating splice signals or introns in the nucleic acid sequence encoding the protein of interest.

In still other embodiments, the vector further comprises at least one internal ribosome entry site (IRES) sequence. The sequences of several suitable IRES's are available, including, but not limited to, those derived from foot and mouth disease virus (FDV), encephalomyocarditis virus, and poliovirus. The IRES sequence can be interposed between two transcriptional units (*e.g*., nucleic acids encoding different proteins of interest or subunits of a multisubunit protein such as an antibody) to form a polycistronic sequence so that the two transcriptional units are transcribed from the same promoter.

The retroviral vectors of the present disclosure may also further comprise a selectable marker allowing selection of transformed cells. A number of selectable markers can be used, including, but not limited to the bacterial aminoglycoside 3' phosphotransferase gene (also referred to as the neo gene) that confers resistance to the drug G418 in mammalian cells, the bacterial hygromycin G phosphotransferase (*hyg*) gene that confers resistance to the antibiotic hygromycin and the bacterial xanthine-guanine phosphoribosyl transferase gene (also referred to as the *gpt* gene) that confers the ability to grow in the presence of mycophenolic acid. In some embodiments, the selectable marker gene is provided as part of polycistronic sequence that also encodes the protein of interest.

In still other embodiments, the retroviral vectors may comprise recombination elements recognized by a recombination system (*e.g*., the cre/loxP or flp recombinase systems, *see, e.g.,* Hoess et al., Nucleic Acids Res. 14:2287-2300 [1986], O'Gorman et al., Science 251:1351-55 [1991], van Deursen et al., Proc. Natl. Acad. Sci. USA 92:7376-80 [1995], and U.S. Pat. No. 6,025,192). After integration of the vectors into the genome of the host cell, the host cell can be transiently transfected (*e.g*., by electroporation, lipofection, or microinjection) with either a recombinase enzyme (*e.g*., Cre recombinase) or a nucleic acid sequence encoding the recombinase enzyme and one or more nucleic acid sequences encoding a protein of interest flanked by sequences recognized by the recombination enzyme so that the nucleic acid sequence is inserted into the integrated vector.

Viral vectors, including recombinant retroviral vectors, provide a more efficient means of transferring genes into cells as compared to other techniques such as calcium phosphate-DNA co-precipitation or DEAE-dextran-mediated transfection, electroporation or microinjection of nucleic acids. It is believed that the efficiency of viral transfer is due in part to the fact that the transfer of nucleic acid is a receptor-mediated process (*i.e.,* the virus binds to a specific receptor protein on the surface of the cell to be infected). In addition, the virally transferred nucleic acid once inside a cell integrates in controlled manner in contrast to the integration of nucleic acids which are not virally transferred; nucleic acids transferred by other means such as calcium phosphate-DNA co-precipitation are subject to rearrangement and degradation.

The most commonly used recombinant retroviral vectors are derived from the amphotropic Moloney murine leukemia virus (MoMLV) (*See e.g.,* Miller and Baltimore Mol. Cell. Biol. 6:2895 [1986]). The MoMLV system has several advantages: 1) this specific retrovirus can infect many different cell types, 2) established packaging cell lines are available for the production of recombinant MoMLV viral particles and 3) the transferred genes are permanently integrated into the target cell chromosome. The established MoMLV vector systems comprise a DNA vector containing a small portion of the retroviral sequence (*e.g*., the viral long terminal repeat or "LTR" and the packaging or "psi" signal) and a packaging cell line. The gene to be transferred is inserted into the DNA vector. The viral sequences present on the DNA vector provide the signals necessary for the insertion or packaging of the vector RNA into the viral particle and for the expression of the inserted gene. The packaging cell line provides the proteins required for particle assembly (Markowitz et al., J. Virol. 62:1120 [1988]).

Despite these advantages, existing retroviral vectors based upon MoMLV are limited by several intrinsic problems: 1) they do not infect non-dividing cells (Miller et al., Mol. Cell. Biol. 10:4239 [1990]), except, perhaps, oocytes; 2) they produce low titers of the recombinant virus (Miller and Rosman, BioTechniques 7: 980 [1980] and Miller, Nature 357: 455 [1990]); and 3) they infect certain cell types (*e.g*., human lymphocytes) with low efficiency (Adams et al., Proc. Natl. Acad. Sci. USA 89:8981 [1992]). The low titers associated with MoMLV-based vectors have been attributed, at least in part, to the instability of the virus-encoded envelope protein. Concentration of retrovirus stocks by physical means (*e.g*., ultracentrifugation and ultrafiltration) leads to a severe loss of infectious virus.

The low titer and inefficient infection of certain cell types by MoMLV-based vectors has been overcome by the use of pseudotyped retroviral vectors which contain the G protein of VSV as the membrane associated protein. Unlike retroviral envelope proteins which bind to a specific cell surface protein receptor to gain entry into a cell, the VSV G protein interacts with a phospholipid component of the plasma membrane (Mastromarino et al., J. Gen. Virol. 68:2359 [1977]). Because entry of VSV into a cell is not dependent upon the presence of specific protein receptors, VSV has an extremely broad host range. Pseudotyped retroviral vectors bearing the VSV G protein have an altered host range characteristic of VSV (*i.e.,* they can infect almost all species of vertebrate, invertebrate and insect cells). Importantly, VSV G-pseudotyped retroviral vectors can be concentrated 2000-fold or more by ultracentrifugation without significant loss of infectivity (Burns et al. Proc. Natl. Acad. Sci. USA 90:8033 [1993]).

The present disclosure is not limited to the use of the VSV G protein when a viral G protein is employed as the heterologous membrane-associated protein within a viral particle (*See*, *e.g.,* U.S. Pat. No. 5,512,421). The G proteins of viruses in the Vesiculovirus genera other than VSV, such as the Piry and Chandipura viruses, that are highly homologous to the VSV G protein and, like the VSV G protein, contain covalently linked palmitic acid (Brun et al. Intervirol. 38:274 [1995] and Masters et al., Virol. 171:285 (1990]). Thus, the G protein of the Piry and Chandipura viruses can be used in place of the VSV G protein for the pseudotyping of viral particles. In addition, the VSV G proteins of viruses within the Lyssa virus genera such as Rabies and Mokola viruses show a high degree of conservation (amino acid sequence as well as functional conservation) with the VSV G proteins. For example, the Mokola virus G protein has been shown to function in a manner similar to the VSV G protein (*i.e*., to mediate membrane fusion) and therefore may be used in place of the VSV G protein for the pseudotyping of viral particles (Mebatsion et al., J. Virol. 69:1444 [1995]). Viral particles may be pseudotyped using either the Piry, Chandipura or Mokola G protein as described in Example 2, with the exception that a plasmid containing sequences encoding either the Piry, Chandipura or Mokola G protein under the transcriptional control of a suitable promoter element (*e.g*., the CMV intermediate-early promoter; numerous expression vectors containing the CMV IE promoter are available, such as the pcDNA3.1 vectors (Invitrogen)) is used in place of pHCMV-G. Sequences encoding other G proteins derived from other members of the Rhabdoviridae family may be used; sequences encoding numerous rhabdoviral G proteins are available from the GenBank database.
The majority of retroviruses can transfer or integrate a double-stranded linear form of the virus (the provirus) into the genome of the recipient cell only if the recipient cell is cycling (*i.e*., dividing) at the time of infection. Retroviruses which have been shown to infect dividing cells exclusively, or more efficiently, include MLV, spleen necrosis virus, Rous sarcoma virus and human immunodeficiency virus (HIV; while HIV infects dividing cells more efficiently, HIV can infect non-dividing cells).
It has been shown that the integration of MLV virus DNA depends upon the host cell's progression through mitosis and it has been postulated that the dependence upon mitosis reflects a requirement for the breakdown of the nuclear envelope in order for the viral integration complex to gain entry into the nucleus (Roe et al., EMBO J. 12:2099 [1993]). However, as integration does not occur in cells arrested in metaphase, the breakdown of the nuclear envelope alone may not be sufficient to permit viral integration; there may be additional requirements such as the state of condensation of the genomic DNA (Roe *et al., supra*).

### B. Lentiviral Vectors

The present disclosure also contemplates the use of lentiviral vectors to generate high copy number cell lines. The lentiviruses (*e.g*., equine infectious anemia virus, caprine arthritis-encephalitis virus, human immunodeficiency virus) are a subfamily of retroviruses that are able to integrate into non-dividing cells. The lentiviral genome and the proviral DNA have the three genes found in all retroviruses: *gag, pol,* and *env,* which are flanked by two LTR sequences. The gag gene encodes the internal structural proteins (*e.g*., matrix, capsid, and nucleocapsid proteins); the pol gene encodes the reverse transcriptase, protease, and integrase proteins; and the pol gene encodes the viral envelope glycoproteins. The 5' and 3' LTRs control transcription and polyadenylation of the viral RNAs. Additional genes in the lentiviral genome include the *vif, vpr, tat, rev, vpu, nef,* and *vpx* genes.

A variety of lentiviral vectors and packaging cell lines are known in the art and find use in the present disclosure *(See, e.g.,* U.S. Pat. Nos. 5,994,136 and 6,013,516). Furthermore, the VSV G protein has also been used to pseudotype retroviral vectors based upon the human immunodeficiency virus (HIV) (Naldini et al., Science 272:263 [1996]). Thus, the VSV G protein may be used to generate a variety of pseudotyped retroviral vectors and is not limited to vectors based on MoMLV. The lentiviral vectors may also be modified as described above to contain various regulatory sequences (*e.g*., signal peptide sequences, RNA export elements, and IRES's). After the lentiviral vectors are produced, they may be used to transfect host cells as described above for retroviral vectors.

### C. Transfection at High Multiplicities of Infection

Once integrating vectors (*e.g*., retroviral vectors) encoding a protein of interest have been produced, they may be used to transfect or transduce host cells (examples of which are described above in Section I). Preferably, host cells are transfected or transduced with integrating vectors at a multiplicity of infection sufficient to result in the integration of at least 1, and preferably at least 2 or more retroviral vectors. In some other cases, multiplicities of infection of from 10 to 1,000,000 may be utilized, so that the genomes of the infected host cells contain from 2 to 100 copies of the integrated vectors, and preferably from 5 to 50 copies of the integrated vectors. In other cases, a multiplicity of infection of from 10 to 10,000 is utilized. When non-pseudotyped retroviral vectors are utilized for infection, the host cells are incubated with the culture medium from the retroviral producers cells containing the desired titer *(i.e.,* colony forming units, CFUs) of infectious vectors. When pseudotyped retroviral vectors are utilized, the vectors are concentrated to the appropriate titer by ultracentrifugation and then added to the host cell culture. Alternatively, the concentrated vectors can be diluted in a culture medium appropriate for the cell type. Additionally, when expression of more than one protein of interest by the host cell is desired, the host cells can be transfected with multiple vectors each containing a nucleic acid encoding a different protein of interest.

In each case, the host cells are exposed to medium containing the infectious retroviral vectors for a sufficient period of time to allow infection and subsequent integration of the vectors. In general, the amount of medium used to overlay the cells should be kept to as small a volume as possible so as to encourage the maximum amount of integration events per cell. As a general guideline, the number of colony forming units (cfu) per milliliter should be about 10⁵ to 10⁷ cfu/ml, depending upon the number of integration events desired.

The present disclosure is not limited to any particular mechanism of action. Indeed, an understanding of the mechanism of action is not necessary for practicing the present invention. However, the diffusion rate of the vectors is known to be very limited (*See*, *e.g*., U.S. Pat. No. 5,866,400). Therefore, it is expected that the actual integration rate will be lower (and in some cases much lower) than the multiplicity of infection. Applying the equations from U.S Pat. No. 5,866,400, a titer of 10⁶ cfu/ml has an average vector-vector spacing of 1 micron. The diffusion time of a MMLV vector across 100 microns is approximately 20 minutes. Accordingly, the vector can travel approximately 300 microns in one hour. If 1000 cells are plated in a T25 flask, the cells are spaced 2.5 mm apart on average. Using these values, the only 56 viral particles would be expected to contact a given cell within an hour. The Table below provides the expected contact rate for a given number of cells in a T25 flask with a particular vector titer. However, as shown below in the examples, the actual number of integrations obtained is much lower than may be predicted by these equations.

| **Vector Contact Frequency As A Function of Time and Cell Spacing** | | | |
|---|---|---|---|
| **Vector Titer** | **Cells/T25 Flask** | **MOI** | **Contacts/Hour** |
| 10⁶ | 1000 | 1,000 | 56 |
| 10⁶ | 100 | 10,000 | <56 |
| 10⁵ | 1000 | 100 | 5.6 |
| 10⁴ | 1000 | 10 | 0.6 |

Accordingly, it is contemplated that the actual integration rate is dependent not only on the multiplicity of infection, but also on the contact time (*i.e*., the length of time the host cells are exposed to infectious vector), the confluency or geometry of the host cells being transfected, and the volume of media that the vectors are contained in. It is contemplated that these conditions can be varied as taught herein to produce host cell lines containing multiple integrated copies of integrating vectors. As demonstrated in Examples 8 and 9, MOI can be varied by either holding the number of cells constant and varying CFU's (Example 9), or by holding CFU's constant and varying cell number (Example 8).

In some embodiments, after transfection or transduction, the cells are allowed to multiply, and are then trypsinized and replated. Individual colonies are then selected to provide clonally selected cell lines. In still further embodiments, the clonally selected cell lines are screened by Southern blotting or INVADER assay to verify that the desired number of integration events has occurred. It is also contemplated that clonal selection allows the identification of superior protein producing cell lines. In other embodiments, the cells are not clonally selected following transfection.

In some embodiments, the host cells are transfected with vectors encoding different proteins of interest. The vectors encoding different proteins of interest can be used to transfect the cells at the same time (*e.g*., the host cells are exposed to a solution containing vectors encoding different proteins of interest) or the transfection can be serial (*e.g*., the host cells are first transfected with a vector encoding a first protein of interest, a period of time is allowed to pass, and the host cells are then transfected with a vector encoding a second protein of interest). In some other embodiments, the host cells are transfected with an integrating vector encoding a first protein of interest, high expressing cell lines containing multiple integrated copies of the integrating vector are selected (*e.g*., clonally selected), and the selected cell line is transfected with an integrating vector encoding a second protein of interest. This process may be repeated to introduce multiple proteins of interest. In some embodiments, the multiplicities of infection may be manipulated (*e.g*., increased or decreased) to increase or decrease the expression of the protein of interest. Likewise, the different promoters may be utilized to vary the expression of the proteins of interest. It is contemplated that these transfection methods can be used to construct host cell lines containing an entire exogenous metabolic pathway or to provide host cells with an increased capability to process proteins (*e.g*., the host cells can be provided with enzymes necessary for post-translational modification).

In still further embodiments, cell lines are serially transfected with vectors encoding the same gene. In some other embodiments, the host cells are transfected (*e.g*., at an MOI of about 10 to 100,000, preferably 100 to 10,000) with an integrating vector encoding a protein of interest, cell lines containing single or multiple integrated copies of the integrating vector or expressing high levels of the desired protein are selected (*e.g*., clonally selected), and the selected cell line is retransfected with the vector (*e.g*., at an MOI of about 10 to 100,000, preferably 100 to 10,000). In some embodiments, cell lines comprising at least two integrated copies of the vector are identified and selected. This process may be repeated multiple times until the desired level of protein expression is obtained and may also be repeated to introduce vectors encoding multiple proteins of interest. Unexpectedly, serial transfection with the same gene results in increases in protein production from the resulting cells that are not merely additive.

### III. Uses of Transfected Host Cells

The host cells transfected at a high multiplicity of infection can be used for a variety of purposes. First, the host cells find use in the production of proteins for pharmaceutical, industrial, diagnostic, and other purposes. Second, host cells expressing a particular protein or proteins find use in screening assays (*e.g*., high throughput screening). Third, the host cells find use in the production of multiple variants of proteins, followed by analysis of the activity of the protein variants. Each of these uses is explained in more detail below.

### A. Production of Proteins

It is contemplated that the host cells of the present disclosure find use in the production of proteins for pharmaceutical, industrial, diagnostic, and other uses. The present disclosure is not limited to the production of any particular protein. Indeed, the production of a wide variety of proteins is contemplated, including, but not limited to, erythropoietin, alpha-interferon, alpha-1 proteinase inhibitor, angiogenin, antithrombin III, beta-acid decarboxylase, human growth hormone, bovine growth hormone, porcine growth hormone, human serum albumin, beta-interferon, calf intestine alkaline phosphatase, cystic fibrosis transmembrane regulator, Factor VIII, Factor IX, Factor X, insulin, lactoferrin, tissue plasminogen activator, myelin basic protein, insulin, proinsulin, prolactin, hepatitis B antigen, immunoglobulins, monoclonal antibody CTLA4 Ig, Tag 72 monoclonal antibody, Tag 72 single chain antigen binding protein, protein C, cytokines and their receptors, including, for instance tumor necrosis factors alpha and beta, their receptors and their derivatives; renin; growth hormone releasing factor; parathyroid hormone; thyroid stimulating hormone; lipoproteins; alpha-1-antitrypsin; follicle stimulating hormone; calcitonin; luteinizing hormone; glucagon; von Willebrands factor; atrial natriuretic factor; lung surfactant; urokinase; bombesin; thrombin; hemopoietic growth factor; enkephalinase; human macrophage inflammatory protein (MIP-1-alpha); a serum albumin such mullerian-inhibiting substance; relaxin A-chain; relaxin B-chain; prorelaxin; mouse gonadotropin-associated peptide; beta-lactamase; DNase; inhibin; activin; vascular endothelial growth factor (VEGF); receptors for hormones or growth factors; integrin; protein A or D; rheumatoid factors; a neurotrophic factor such as bone-derived neurotrophic factor (BDNF), neurotrophin-3, -4, -5, or -6 (NT-3, NT-4, NT-5, or NT-6), or a nerve growth factor such as NGF-beta; platelet-derived growth factor (PDGF); fibroblast growth factor such as aFGF and bFGF; epidermal growth factor (EGF); transforming growth factor (TGF) such as TGF-alpha and TGF-beta, including TGF-β1, TGF-β2, TGF-β3, TGF-β4, or TGF-β5; insulin-like growth factor-I and -II (IGF-I and IGF-II); des(1-3)-IGF-I (brain IGF-I), insulinslike growth factor binding proteins; CD proteins such as CD-3, CD-4, CD-8, and CD-19; osteoinductive factors; immunotoxins; a bone morphogenetic protein (BMP); an interferon such as interferon-alpha, -beta, and -gamma; colony stimulating factors (CSFs), *e.g.,* M-CSF, GM-CSF, and G-CSF; interleukins (ILs), *e.g.,* IL-1 to IL-10; superoxide dismutase; T-cell receptors; surface membrane proteins; decay accelerating factor; viral antigen such as, for example, a portion of the AIDS envelope; transport proteins; homing receptors; addressins; regulatory proteins; antibodies; chimeric proteins, such as immunoadhesins, and fragments of any of the above-listed polypeptides. Nucleic acid and protein sequences for these proteins are available in public databases such as GenBank.

In some embodiments, the host cells express more than one exogenous protein. For example, the host cells may be transfected vectors encoding different proteins of interest (*e.g*., cotransfection or infection at a multiplicity of infection of 1000 with one vector encoding a first protein of interest and a second vector encoding a second protein of interest or serial transfection or infection) so that the host cell contains at least one integrated copy of a first vector encoding a first protein of interest and at least one integrated copy of second integrating vector encoding a second protein of interest. In other embodiments, more than one protein is expressed by arranging the nucleic acids encoding the different proteins of interest in a polycistronic sequence (*e.g*., bicistronic or tricistronic sequences). This arrangement is especially useful when expression of the different proteins of interest in about a 1:1 molar ratio is desired (*e.g*., expressing the light and heavy chains of an antibody molecule).

In still further embodiments, ribozymes are expressed in the host cells. It is contemplated that the ribozyme can be utilized for down-regulating expression of a particular gene or used in conjunction with gene switches such as TET, ecdysone, glucocorticoid enhancer, etc. to provide host cells with various phenotypes.

The transfected host cells are cultured according to methods known in the art. Suitable culture conditions for mammalian cells are well known in the art *(See e.g.,* J. Immunol. Methods (1983)56:221-234 [1983], Animal Cell Culture: A Practical Approach 2nd Ed., Rickwood, D. and Hames, B. D., eds. Oxford University Press, New York [1992]).

The host cell cultures of the present disclosure are prepared in a media suitable for the particular cell being cultured. Commercially available media such as Ham's F10 (Sigma, St. Louis, MO), Minimal Essential Medium (MEM, Sigma), RPMI-1640 (Sigma), and Dulbecco's Modified Eagle's Medium (DMEM, Sigma) are exemplary nutrient solutions. Suitable media are also described in U.S. Pat. Nos. 4,767,704; 4,657,866; 4,927,762; 5,122,469; 4,560,655; and WO 90/03430 and WO 87/00195. Any of these media may be supplemented as necessary with serum, hormones and/or other growth factors (such as insulin, transferrin, or epidermal growth factor), salts (such as sodium chloride, calcium, magnesium, and phosphate), buffers (such as HEPES), nucleosides (such as adenosine and thymidine), antibiotics (such as gentamycin (gentamicin), trace elements (defined as inorganic compounds usually present at final concentrations in the micromolar range) lipids (such as linoleic or other fatty acids) and their suitable carriers, and glucose or an equivalent energy source. Any other necessary supplements may also be included at appropriate concentrations that would be known to those skilled in the art. For mammalian cell culture, the osmolality of the culture medium is generally about 290-330 mOsm.

The present disclosure also contemplates the use of a variety of culture systems (*e.g*., petri dishes, 96 well plates; roller bottles, and bioreactors) for the transfected host cells. For example, the transfected host cells can be cultured in a perfusion system. Perfusion culture refers to providing a continuous flow of culture medium through a culture maintained at high cell density. The cells are suspended and do not require a solid support to grow on. Generally, fresh nutrients must be supplied continuously with concomitant removal of toxic metabolites and, ideally, selective removal of dead cells. Filtering, entrapment and micro-capsulation methods are all suitable for refreshing the culture environment at sufficient rates.

As another example, in some embodiments a fed batch culture procedure can be employed. In the preferred fed batch culture the mammalian host, cells and culture medium are supplied to a culturing vessel initially and additional culture nutrients are fed, continuously or in discrete increments, to the culture during culturing, with or without periodic cell and/or product harvest before termination of culture. The fed batch culture can include, for example, a semi-continuous fed batch culture, wherein periodically whole culture (including cells and medium) is removed and replaced by fresh medium. Fed batch culture is distinguished from simple batch culture in which all components for cell culturing (including the cells and all culture nutrients) are supplied to the culturing vessel at the start of the culturing process. Fed batch culture can be further distinguished from perfusion culturing insofar as the supernate is not removed from the culturing vessel during the process (in perfusion culturing, the cells are restrained in the culture by, *e.g*., filtration, encapsulation, anchoring to microcarriers etc. and the culture medium is continuously or intermittently introduced and removed from the culturing vessel). In some particularly preferred embodiments, the batch cultures are performed in roller bottles.

Further, the cells of the culture may be propagated according to any scheme or routine that may be suitable for the particular host cell and the particular production plan contemplated. Therefore, the present disclosure contemplates a single step or multiple step culture procedure. In a single step culture the host cells are inoculated into a culture environment and the processes of the instant invention are employed during a single production phase of the cell culture. Alternatively, a multi-stage culture is envisioned. In the multi-stage culture cells may be cultivated in a number of steps or phases. For instance, cells may be grown in a first step or growth phase culture wherein cells, possibly removed from storage, are inoculated into a medium suitable for promoting growth and high viability. The cells may be maintained in the growth phase for a suitable period of time by the addition of fresh medium to the host cell culture.

Fed batch or continuous cell culture conditions are devised to enhance growth of the mammalian cells in the growth phase of the cell culture. In the growth phase cells are grown under conditions and for a period of time that is maximized for growth. Culture conditions, such as temperature, pH, dissolved oxygen (dO₂) and the like, are those used with the particular host and will be apparent to the ordinarily skilled artisan. Generally, the pH is adjusted to a level between about 6.5 and 7.5 using either an acid (*e.g*., CO₂) or a base (*e.g*., Na₂CO₃ or NaOH). A suitable temperature range for culturing mammalian cells such as CHO cells is between about 30° to 38° C and a suitable dO₂ is between 5-90% of air saturation.

Following the polypeptide production phase, the polypeptide of interest is recovered from the culture medium using techniques which are well established in the art. The protein of interest preferably is recovered from the culture medium as a secreted polypeptide (*e.g*., the secretion of the protein of interest is directed by a signal peptide sequence), although it also may be recovered from host cell lysates. As a first step, the culture medium or lysate is centrifuged to remove particulate cell debris. The polypeptide thereafter is purified from contaminant soluble proteins and polypeptides, with the following procedures being exemplary of suitable purification procedures: by fractionation on immunoaffinity or ion-exchange columns; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; and protein A Sepharose columns to remove contaminants such as IgG. A protease inhibitor such as phenyl methyl sulfonyl fluoride (PMSF) also may be useful to inhibit proteolytic degradation during purification. Additionally, the protein of interest can be fused in frame to a marker sequence which allows for purification of the protein of interest. Non-limiting examples of marker sequences include a hexahistidine tag which may be supplied by a vector, preferably a pQE-9 vector, and a hemagglutinin (HA) tag. The HA tag corresponds to an epitope derived from the influenza hemagglutinin protein *(See* e.g*.,* Wilson et al., Cell, 37:767 [1984]). One skilled in the art will appreciate that purification methods suitable for the polypeptide of interest may require modification to account for changes in the character of the polypeptide upon expression in recombinant cell culture.

The host cells of the present disclosure are also useful for expressing G-protein coupled receptors (GPCRs) and other transmembrane proteins. It is contemplated that when these proteins are expressed, they are correctly inserted into the membrane in their native conformation. Thus, GPCRs and other transmembrane proteins may be purified as part of a membrane fraction or purified from the membranes by methods known in the art.

Furthermore, the vectors of the present disclosure are useful for co-expressing a protein of interest for which there is no assay or for which assays are difficult. In this system, a protein of interest and a signal protein are arranged in a polycistronic sequence. Preferably, an IRES sequence separates the signal protein and protein of interest (*e.g*., a GPCR) and the genes encoding the signal protein and protein of interest are expressed as a single transcriptional unit. The present disclosure is not limited to any particular signal protein. Indeed, the use of a variety of signal proteins for which easy assays exist is contemplated. These signal proteins include, but are not limited to, green fluorescent protein, luciferase, beta-galactosidase, and antibody heavy or light chains. It is contemplated that when the signal protein and protein of interest are co-expressed from a polycistronic sequence, the presence of the signal protein is indicative of the presence of the protein of interest. Accordingly, in some embodiments, the present disclosure provides methods for indirectly detecting the expression of a protein of interest comprising providing a host cell transfected with a vector encoding a polycistronic sequence, wherein the polycistronic sequence comprises a signal protein and a protein of interest operably linked by an IRES, and culturing the host cells under conditions such that the signal protein and protein of interest are produced, wherein the presence of the signal protein indicates the presence of the protein of interest.

### B. Screening Compounds for Activity

The present disclosure contemplates the use of the high copy number cell lines for screening compounds for activity, and in particular to high throughput screening of compound from combinatorial libraries (*e.g*., libraries containing greater than 10⁴ compounds). The high copy number cell lines of the present disclosure can be used in a variety of screening methods. In some embodiments, the cells can be used in second messenger assays that monitor signal transduction following activation of cell-surface receptors. In other embodiments, the cells can be used in reporter gene assays that monitor cellular responses at the transcription/translation level. In still further embodiments, the cells can be used in cell proliferation assays to monitor the overall growth/no growth response of cells to external stimuli.

In second messenger assays, the host cells are preferably transfected as described above with vectors encoding cell surface receptors, ion channels, cytoplasmic receptors, or other proteins involved in signal transduction (*e.g*., G proteins, protein kinases, or protein phosphatases) *(See, e.g.,* U.S Pat. Nos. 5,670,113; 5,807,689; 5,876,946; and 6,027,875). The host cells are then treated with a compound or plurality of compounds (*e.g*., from a combinatorial library) and assayed for the presence or absence of a response. It is contemplated that at least some of the compounds in the combinatorial library can serve as agonists, antagonists, activators, or inhibitors of the protein or proteins encoded by the vectors. It is also contemplated that at least some of the compounds in the combinatorial library can serve as agonists, antagonists, activators, or inhibitors of protein acting upstream or downstream of the protein encoded by the vector in a signal transduction pathway.

For instance, it is known that agonist engaged transmembrane receptors are functionally linked to the modulation of several well characterized promoter/enhancer elements (*e.g*., AP1, cAMP response element (CRE), serum response element (SRE), and nuclear factor of activated T-cells (NF-AT)). Upon activation of a G_{αs} coupling receptor, adenylyl cyclase is stimulated, producing increased concentrations of intracellular cAMP, stimulation of protein kinase A, phosphorylation of the CRE binding protein (CREB) and induction of promoters with CRE elements. G_{αi} coupling receptors dampen CRE activity by inhibition of the same signal transduction components. G_{αq} and some βγ pairs stimulate phospholipase C (PLC), and the generation of inositol triphosphate (IP3) and diacylglycerol (DAG). A transient flux in intracellular calcium promotes induction of calcineurin and NA-FT, as well as calmodulin (CaM)-dependent kinase and CREB. Increased DAG concentrations stimulate protein kinase C (PKC) and endosomal/lysosomal acidic sphingomyelinase (aSMase); while the aSMase pathway is dominant, both induce degradation of the NFκB inhibitor IκB as well as NFκB activation. In an alternative pathway, a receptor such as growth factor receptor is activated and recruits Sos to the plasma membrane, resulting in the stimulation of Ras, which in turn recruits the serine/threonine kinase Raf to the plasma membrane. Once activated, Raf phosphorylates MEK kinase, which phosphorylates and activates MAPK and the transcription factor ELK. ELK drives transcription from promoters with SRE elements, leading the synthesis of the transcription factors Fos and Jun, thus forming a transcription factor complex capable of activating AP1 sites. It is contemplated that the proteins forming the described pathways, as well as other receptors, kinases, phosphatases, and nucleic binding proteins, are targets for compounds in the combinatorial library, as well as candidates for expression in the host cells of the present invention.

In some embodiments, the second messenger assays measure fluorescent signals from reporter molecules that respond to intracellular changes (*e.g*., Ca²⁺ concentration, membrane potential, pH, IP₃, cAMP, arachidonic acid release) due to stimulation of membrane receptors and ion channels (*e.g*., ligand gated ion channels; see Denyer et al., Drug Discov. Today 3:323-32 [1998]; and Gonzales et al., Drug. Discov. Today 4:431-39 [1999]). Examples of reporter molecules include, but are not limited to, FRET (florescence resonance energy transfer) systems (*e.g*., Cuo-lipids and oxonols, EDAN/DABCYL), calcium sensitive indicators (*e.g*., Fluo-3, FURA 2, INDO 1, and FLUO3/AM, BAPTA AM), chloride-sensitive indicators (*e.g*., SPQ, SPA), potassium-sensitive indicators (*e.g*., PBFI), sodium-sensitive indicators (*e.g*., SBFI), and pH sensitive indicators (*e.g.*, BCECF).

In general, the host cells are loaded with the indicator prior to exposure to the compound. Responses of the host cells to treatment with the compounds can be detected by methods known in the art, including, but not limited to, fluorescence microscopy, confocal microscopy (*e.g*., FCS systems), flow cytometry, microfluidic devices, FLIPR systems (*See, e.g.,* Schroeder and Neagle, J. Biomol. Screening 1:75-80 [1996]), and plate-reading systems. In some embodiments, the response (*e.g*., increase in fluorescent intensity) caused by compound of unknown activity is compared to the response generated by a known agonist and expressed as a percentage of the maximal response of the known agonist. The maximum response caused by a known agonist is defined as a 100% response. Likewise, the maximal response recorded after addition of an agonist to a sample containing a known or test antagonist is detectably lower than the 100% response.

The cells are also useful in reporter gene assays. Reporter gene assays involve the use of host cells transfected with vectors encoding a nucleic acid comprising transcriptional control elements of a target gene (*i.e*., a gene that controls the biological expression and function of a disease target) spliced to a coding sequence for a reporter gene. Therefore, activation of the target gene results in activation of the reporter gene product. Examples of reporter genes finding use in the present disclosure include, but are not limited to, chloramphenicol transferase, alkaline phosphatase, firefly and bacterial luciferases, β-galactosidase, β-lactamase, and green fluorescent protein. The production of these proteins, with the exception of green fluorescent protein, is detected through the use of chemiluminescent, colorimetric, or bioluminecent products of specific substrates (*e.g*., X-gal and luciferin). Comparsions between compounds of known and unknown activities may be conducted as described above.

### C. Comparison of Variant Protein Activity

The present disclosure also contemplates the use of the high copy number host cells to produce variants of proteins so that the activity of the variants can be compared. In some embodiments, the variants differ by a single nucleotide polymorphism (SNP) causing a single amino acid difference. In other embodiments, the variants contain multiple amino acid substitutions. In some embodiments, the activity of the variant proteins are assayed in *vivo* or in cell extracts. In other embodiments, the proteins are purified and assayed *in vitro.* It is also contemplated that in some embodiments the variant proteins are fused to a sequence that allows easy purification (*e.g*., a his-tag sequence) or to a reporter gene (*e.g*., green fluorescent protein). Activity of the proteins may be assayed by appropriate methods known in the art (*e.g*., conversion of a substrate to a product). In some embodiments, the activity of a wild-type protein is determined, and the activity of variant versions of the wild-type proteins are expressed as a percentage of the activity of the wild-type protein. Furthermore, the intracellular activity of variant proteins may be compared by constructing a plurality of host cells lines, each of which expresses a different variant of the wild-type protein. The activity of the variant proteins (*e.g*., variants of proteins involved in signal transduction pathways) may then be compared using the reporter systems for second messenger assays described above. Therefore, in some embodiments, the direct or indirect response (*e.g.*, through downstream or upstream activation of signal transduction pathway) of variant proteins to stimulation or binding by agonists or antagonists is compared. In some emodiments, the response of a wild-type protein is determined, and the responses of variant versions of the wild-type proteins are expressed as a percentage of the response of the wild-type protein.

### EXPERIMENTAL

The following examples serve illustrate certain embodiments and aspects of the present disclosure and invention.

In the experimental disclosure which follows, the following abbreviations apply: M (molar); mM (millimolar); µM (micromolar); nM (nanomolar); mol (moles); mmol (millimoles); µmol (micromoles); nmol (nanomoles); gm (grams); mg (milligrams); µg (micrograms);pg (picograms); L (liters); ml (milliliters); µl (microliters); cm (centimeters); mm (millimeters); µm (micrometers); nm (nanometers); °C (degrees Centigrade); AMP (adenosine 5'-monophosphate); BSA (bovine serum albumin); cDNA (copy or complimentary DNA); CS (calf serum); DNA (deoxyribonucleic acid); ssDNA (single stranded DNA); dsDNA (double stranded DNA); dNTP (deoxyribonucleotide triphosphate); LH (luteinizing hormone); NIH (National Institues of Health, Besthesda, MD); RNA (ribonucleic acid); PBS (phosphate buffered saline); *g* (gravity); OD (optical density); HEPES (N-[2-Hydroxyethyl]piperazine-N-[2-ethanesulfonic acid]); HBS (HEPES buffered saline); PBS (phosphate buffered saline); SDS (sodium dodecylsulfate); Tris-HCl (tris[Hydroxymethyl]aminomethane-hydrochloride); Klenow (DNA polymerase I large (Klenow) fragment); rpm (revolutions per minute); EGTA (ethylene glycol-bis(β-aminoethyl ether) N, N, N', N'-tetraacetic acid); EDTA (ethylenediaminetetracetic acid); bla (β-lactamase or ampicillin-resistance gene); ORI (plasmid origin of replication); lace (lac repressor); X-gal (5-bromo-4-chloro-3-indolyl-β-D-galactoside); ATCC (American Type Culture Collection, Rockville, MD); GIBCO/BRL (GIBCO/BRL, Grand Island, NY); Perkin-Elmer (Perkin-Elmer, Norwalk, CT); and Sigma (Sigma Chemical Company, St. Louis, MO).

### Example 1

### Vector Construction

The following Example describes the construction of vectors used in the experiments below.

### A. CMV MN14

The CMV MN14 vector (SEQ ID NO:4; MN14 antibody is described in U.S. Pat. No. 5,874,540) comprises the following elements, arranged in 5' to 3' order: CMV promoter; MN14 heavy chain signal peptide, MN14 antibody heavy chain; IRES from encephalomyocarditis virus; bovine α-lactalbumin signal peptide; MN 14 antibody light chain; and 3' MoMuLV LTR. In addition to sequences described in SEQ ID NO: 4, the CMV MN14 vector further comprises a 5' MoMuLV LTR, a MoMuLV extended viral packaging signal, and a neomycin phosphotransferase gene (these additional elements are provided in SEQ ID NO:7; the 5' LTR is derived from Moloney Murine Sarcoma Virus in each of the constructs described herein, but is converted to the MoMuLV 5' LTR when integrated).

This construct uses the 5' MoMuLV LTR to control production of the neomycin phosphotransferase gene. The expression of MN14 antibody is controlled by the CMV promoter. The MN14 heavy chain gene and light chain gene are attached together by an IRES sequence. The CMV promoter drives production of a mRNA containing the heavy chain gene and the light chain gene attached by the IRES. Ribosomes attach to the mRNA at the CAP site and at the IRES sequence. This allows both heavy and light chain protein to be produced from a single mRNA. The mRNA expression from the LTR as well as from the CMV promoter is terminated and poly adenylated in the 3' LTR. The construct was cloned by similar methods as described in section B below.

The IRES sequence (SEQ ID NO:3) comprises a fusion of the IRES from the plasmid pLXIN (Clontech) and the bovine α-lactalbumin signal peptide. The initial ATG of the signal peptide was attached to the IRES to allow the most efficient translation initiation from the IRES. The 3' end of the signal peptide provides a multiple cloning site allowing easy attachment of any protein of interest to create a fusion protein with the signal peptide. The IRES sequence can serve as a translational enhancer as well as creating a second translation initiation site that allows two proteins to be produced from a single mRNA.

The IRES-bovine α-lactalbumin signal peptide was constructed as follows. The portion of the plasmid pLXIN (Clontech, Palo Alto, CA) containing the ECMV IRES was PCR amplified using the following primers.
Primer 1 (SEQ ID NO: 35):
   5' GATCCACTAGTAACGGCCGCCAGAATTCGC 3'
Primer 2 (SEQ ID NO: 36):
   5' CAGAGAGACAAAGGAGGCCATATTATCATCGTGTTTTTCAAAG 3'

Primer 2 attaches a tail corresponding to the start of the bovine α-lactalbumin signal peptide coding region to the IRES sequence. In addition, the second triplet codon of the α-lactalbumin signal peptide was mutated from ATG to GCC to allow efficient translation from the IRES sequence. This mutation results in a methionine to alanine change in the protein sequence. This mutation was performed because the IRES prefers an alanine as the second amino acid in the protein chain. The resulting IRES PCR product contains an EcoRI site on the 5' end of the fragment (just downstream of Primer 1 above).

Next, the α-lactalbumin signal peptide containing sequence was PCR amplified from the α-LA Signal Peptide vector construct using the following primers.
Primer 3 (SEQ II7 NO: 14):
   5' CTTTGAAAAACACGATGATAATATGGCCTCCTTTGTCTCTCTG 3'
Primer 4 (SEQ ID NO: 15):
   5' TTCGCGAGCTCGAGATCTAGATATCCCATG 3'

Primer 3 attaches a tail corresponding to the 3' end of the IRES sequence to the α-lactalbumin signal peptide coding region. As stated above, the second triplet codon of the bovine α-lactalbumin signal peptide was mutated to allow efficient translation from the IRES sequence. The resulting signal peptide PCR fragment contains NaeI, NcoI, EcoRV, XbaI, BglII and XhoI sites on the 3' end.

After the IRES and signal peptide were amplified individually using the primers shown above, the two reaction products were mixed and PCR was performed using primer 1 and primer 4. The resultant product of this reaction is a spliced fragment that contains the IRES attached to the full length α-lactalbumin signal peptide. The ATG encoding the start of the signal peptide is placed at the same location as the ATG encoding the start of the neomycin phosphotransferase gene found in the vector pLXIN. The fragment also contains the EcoRI site on the 5' end and NaeI, NcoI, EcoRV, XbaI, BglII and XhoI sites on the 3' end.

The spliced IRES/α-lactalbumin signal peptide PCR fragment was digested with EcoRI and XhoI. The α-LA Signal Peptide vector construct was also digested with EcoRI and XhoI. These two fragments were ligated together to give the pIRES construct.

The IRES/α-lactalbumin signal peptide portion of the pIRES vector was sequenced and found to contain mutations in the 5' end of the IRES. These mutations occur in a long stretch of C's and were found in all clones that were isolated.

To repair this problem, pLXIN DNA was digested with EcoRI and BsmFI. The 500bp band corresponding to a portion of the IRES sequence was isolated. The mutated IRES/α-lactalbmnin signal peptide construct was also digested with EcoRI and BsmFI and the mutated IRES fragment was removed. The IRES fragment from pLXIN was then substituted for the IRES fragment of the mutated IRES/α-lactalbumin signal peptide construct. The IRES/α-LA signal peptide portion of resulting plasmid was then verified by DNA sequencing.

The resulting construct was found to have a number of sequence differences when compared to the expected pLXIN sequence obtained from Clontech. We also sequenced the IRES portion of pLXIN purchased from Clontech to verify its sequence. The differences from the expected sequence also appear to be present in the pLXIN plasmid that we obtained from Clontech. Four sequence differences were identified:
bp 347 T - was G in pLXIN sequence
bp 786-788 ACG - was GC in LXIN sequence.

### B. CMV LL2

The CMV LL2 (SEQ ID NO:5; LL2 antibody is described in U.S. Pat. No. 6,187,287) construct comprises the following elements, arranged in 5' to 3' order: 5' CMV promoter (Clonetech), LL2 heavy chain signal peptide, LL2 antibody heavy chain; IRES from encephalomyocarditis virus, bovine α-LA signal peptide; LL2 antibody light chain; and 3' MoMuLV LTR. In addition to sequence described in SEQ ID NO:5, the CMV LL2 vector further comprises a 5' MoMuLV LTR, a MoMuLV extended viral packaging signal, and a neomycin phosphotransferase gene (these additional elements are provided in SEQ ID NO:7).

This construct uses the 5' MoMuLV LTR to control production of the neomycin phosphotransferase gene. The expression of LL2 antibody is controlled by the CMV promoter (Clontech). The LL2 heavy chain gene and light chain gene are attached together by an IRES sequence. The CMV promoter drives production of a mRNA containing the heavy chain gene and the light chain gene attached by the IRES. Ribosomes attach to the mRNA at the CAP site and at the IRES sequence. This allows both heavy and light chain protein to be produced from a single mRNA. The mRNA expression from the LTR as well as from the CMV promoter is terminated and poly adenylated in the 3' LTR.

The IRES sequence (SEQ ID NO:3) comprises a fusion of the IRES from the plasmid pLXIN (Clontech) and the bovine alpha-lactalbumin signal peptide. The initial ATG of the signal peptide was attached to the IRES to allow the most efficient translation initiation from the IRES. The 3' end of the signal peptide provides a multiple cloning site allowing easy attachment of any protein of interest to create a fusion protein with the signal peptide. The IRES sequence can serve as a translational enhancer as well as creating a second translation initiation site that allows two proteins to be produced from a single mRNA.

The LL2 light chain gene was attached to the IRES α-lactalbumin signal peptide as follows. The LL2 light chain was PCR amplified from the vector pCRLL2 using the following primers.
Primer 1 (SEQ ID NO: 16):
   5' CTACAGGTGTCCACGTCGACATCCAGCTGACCCAG 3'
Primer 2 (SEQ ID NO: 17):
   5' CTGCAGAATAGATCTCTAACACTCTCCCCTGTTG 3'

These primers add a HincII site right at the start of the coding region for mature LL2 light chain. Digestion of the PCR product with HincII gives a blunt end fragment starting with the initial GAC encoding mature LL2 on the 5' end. Primer 2 adds a BglII site to the 3' end of the gene right after the stop codon. The resulting PCR product was digested with HuncII and BglII and cloned directly into the IRES-Signal Peptide plasmid that was digested with NaeI and BglII.

The Kozak sequence of the LL2 heavy chain gene was then modified. The vector pCRMN14HC was digested with XhoI and AvrII to remove about a 400 bp fragment. PCR was then used to amplify the same portion of the LL2 heavy chain construct that was removed by the XhoI-AvrII digestion. This amplification also mutated the 5' end of the gene to add a better Kozak sequence to the clone. The Kozak sequence was modified to resemble the typical IgG Kozak sequence. The PCR primers are shown below.
Primer 1 (SEQ ID NO: 18):
   5'CAGTGTGATCTCGAGAATTCAGGACCTCACCATGGGATGGAGCTGTATCAT 3'
Primer 2 (SEQ ID NO: 19):
   5'AGGCTGTATTGGTGGATTCGTCT 3'

The PCR product was digested with XhoI and AvrII and inserted back into the previously digested plasmid backbone.

The "good" Kozak sequence was then added to the light chain gene. The "good" Kozak LL2 heavy chain gene construct was digested with EcoRI and the heavy chain gene containing fragment was isolated. The IRES α-Lactalbumin Signal Peptide LL2 light chain gene construct was also digested with EcoRI. The heavy chain gene was then cloned into the EcoRI site of IRES light chain construct. This resulted in the heavy chain gene being placed at the 5' end of the IRES sequence.

Next, a multiple cloning site was added into the LNCX retroviral backbone plasmid. The LNCX plasmid was digested with HindIII and ClaI. Two oligonucleotide primers were produced and annealed together to create an double stranded DNA multiple cloning site. The following primers were annealed together.
Primer 1 (SEQ ID NO: 20):
   5'AGCTTCTCGAGTTAACAGATCTAGGCCTCCTAGGTCGACAT3'
Primer 2 (SEQ ID NO: 21): 5'
   CGATGTCGACCTAGGAGGCCTAGATCTGTTAACTCGAGA 3'
After annealing, the multiple cloning site was ligated into LNCX to create LNC-MCS.

Next, the double chain gene fragment was ligated into the retroviral backbone gene construct. The double chain gene construct created above was digested with SalI and BglII and the double chain containing fragment was isolated. The retroviral expression plasmid LNC-MCS was digested with XhoI and BrlII. The double chain fragment was then cloned into the LNC-MCS retroviral expression backbone.

Next, an RNA splicing problem in the construct was corrected. The construct was digested with NsiI. The resulting fragment was then partially digested with EcoRI. The fragments resulting from the partial digest that were approximately 9300 base pairs in size were gel purified. A linker was created to mutate the splice donor site at the 3' end of the LL2 heavy chain gene. The linker was again created by annealing two oligonucleotide primers together to form the double stranded DNA linker. The two primers used to create the linker are shown below.

After annealing the linker was substituted for the original NsiI/EcoRI fragment that was removed during the partial digestion.

### C. MMTV MN14

The MMTV MN14 (SEQ ID NO:6) construct comprises the following elements, arranged in 5' to 3' order: 5' MMTV promoter; double mutated PPE sequence; MN 14 antibody heavy chain; IRES from encephalomyocarditis virus; bovine αLA signal peptide MN 14 antibody light chain; WPRE sequence; and 3' MoMuLV LTR. In addition to the sequences described in SEQ ID NO:6, the MMTV MN14 vector further comprises a MoMuLV LTR, MoMuLV extended viral packaging signal; neomycin phosphotransferase gene located 5' of the MMTV promoter (these additional elements are provided in SEQ ID NO: 7).

This construct uses the 5' MoMuLV LTR to control production of the neomycin phosphotransferase gene. The expression of MN14 antibody is controlled by the MMTV promoter (Pharmacia). The MN14 heavy chain gene and light chain gene are attached together by an IRES/ bovine α-LA signal peptide sequence (SEQ ID NO: 3). The MMTV promoter drives production of a mRNA containing the heavy chain gene and the light chain gene attached by the IRES/bovine α-LA signal peptide sequence. Ribosomes attach to the mRNA at the CAP site and at the IRES/ bovine α-LA signal peptide sequence. This allows both heavy and light chain protein to be produced from a single mRNA. In addition, there are two genetic elements contained within the mRNA to aid in export of the mRNA from the nucleus to the cytoplasm and aid in poly-adenylation of the mRNA. The PPE sequence is contained between the RNA CAP site and the start of the, MN14 protein coding region, the WPRE is contained between the end of MN14 protein coding and the poly-adenylation site. The mRNA expression from the LTR as well as from the MMTV promoter is terminated and poly-adenylated in the 3' LTR.

ATG sequences within the PPE element (SEQ ID NO:2) were mutated to prevent potential unwanted translation initiation. Two copies of this mutated sequence were used in a head to tail array. This sequence is placed just downstream of the promoter and upstream of the Kozak sequence and signal peptide-coding region. The WPRE is isolated from woodchuck hepatitis virus and also aids in the export of mRNA from the nucleus and creating stability in the mRNA. If this sequence is included in the 3' untranslated region of the RNA, level of protein expression from this RNA increases up to 10-fold.

### D. α-LA MN14

The α-LA MN14 (SEQ ID NO:7) construct comprises the following elements, arranged in 5' to 3' order: 5' MoMuLV LTR, MoMuLV extended viral packaging signal, neomycin phosphotransferase gene, bovine/human alpha-lactalbumin hybrid promoter, double mutated PPE element, MN14 heavy chain signal peptide, MN14 antibody heavy chain, IRES from encephalomyocarditis virus/bovine αLA signal peptide, MN14 antibody light chain, WPRE sequence; and 3' MoMuLV LTR

This construct uses the 5' MoMuLV LTR to control production of the neomycin phosphotransferase gene. The expression of MN14 antibody is controlled by the hybrid α-LA promoter (SEQ ID NO:1). The MN14 heavy chain gene and light chain gene are attached together by an IRES sequence/ bovine α-LA signal peptide (SEQ ID NO:3). The α-LA promoter drives production of a mRNA containing the heavy chain gene and the light chain gene attached by the IRES. Ribosomes attach to the mRNA at the CAP site and at the IRES sequence. This allows both heavy and light chain protein to be produced from a single mRNA.

In addition, there are two genetic elements contained within the mRNA to aid in export of the mRNA from the nucleus to the cytoplasm and aid in poly-adenylation of the mRNA. The mutated PPE sequence (SEQ ID NO:2) is contained between the RNA CAP site and the start of the MN14 protein coding region. ATG sequences within the PPE element (SEQ ID NO:2) were mutated to prevent potential unwanted translation initiation. Two copies of this mutated sequence were used in a head to tail array. This sequence is placed just downstream of the promoter and upstream of the Kozak sequence and signal peptide-coding region. The WPRE was isolated from woodchuck hepatitis virus and also aids in the export of mRNA from the nucleus and creating stability in the mRNA. If this sequence is included in the 3' untranslated region of the RNA, level of protein expression from this RNA increases up to 10-fold. The WPRE is contained between the end of MN14 protein coding and the poly-adenylation site. The mRNA expression from the LTR as well as from the bovine/human alpha-lactalbumin hybrid promoter is terminated and poly adenylated in the 3' LTR.

The bovine/human alpha-lactalbumin hybrid promoter (SEQ ID NO:1) is a modular promoter /enhancer element derived from human and bovine alpha-lactalbumin promoter sequences. The human portion of the promoter is from +15 relative to transcription start point (tsp) to -600 relative to the tsp. The bovine portion is then attached to the end of the human portion and corresponds to -550 to -2000 relative to the tsp. The hybrid was developed to remove poly-adenylation signals that were present in the bovine promoter and hinder retroviral RNA production. It was also developed to contain genetic control elements that are present in the human gene, but not the bovine.

For construction of the bovine/human α-lactalbumin promoter, human genomic DNA was isolated and purified. A portion of the human α-lactalbumin promoter was PCR amplified using the following two primers:
Primer 1 (SEQ ID NO: 24):
   5'AAAGCATATGTTCTGGGCCTTGTTACATGGCTGGATTGGTT 3'

This two primers created a NdeI site on the 5' end of the PCR fragment and a EcoRI site on the 3' end of the PCR fragment.

The human PCR fragment created using the above primers was double digested with the restriction enzymes NdeI and EcoRI. The plasmid pKBaP-1 was also double digested with NdeI and EcoRI. The plasmid pKBaP-1 contains the bovine α-lactalbumin 5' flanking region attached to a multiple cloning site. This plasmid allows attachment of various genes to the bovine α-lactalbumin promoter.

Subsequently, the human fragment was ligated/substituted for the bovine fragment of the promoter that was removed from the pKBaP-1 plasmid during the double digestion. The resulting plasmid was confirmed by DNA sequencing to be a hybrid of the Bovine and Human α-lactalbumin promoter/regulatory regions.

Attachment of the MN14 light chain gene to the IRES α-lactalbumin signal peptide was accomplished as follows. The MN14 light chain was PCR amplified from the vector pCRMN14LC using the following primers.
Primer 1 (SEQ ID NO: 26): 5' CTACAGGTGTCCACGTCGACATCCAGCTGACCCAG 3'
Primer 2 (SEQ ID NO: 27): 5' CTGCAGAATAGATCTCTAACACTCTCCCCTGTTG 3'

These primers add a HincII site right at the start of the coding region for mature MN14 light chain. Digestion of the PCR product with HincII gives a blunt end fragment starting with the initial GAC encoding mature MN14 on the 5' end. Primer 2 adds a BglII site to the 3' end of the gene right after the stop codon. The resulting PCR product was digested with HincII and BglII and cloned directly into the IRES-Signal Peptide plasmid that was digested with NaeI and BglII.

Next, the vector pCRMN14HC was digested with XhoI and NruI to remove about a 500 bp fragment. PCR was then used to amplify the same portion of the MN14 heavy chain construct that was removed by the XhoI-NruI digestion. This amplification also mutated the 5' end of the gene to add a better Kozak sequence to the clone. The Kozak sequence was modified to resemble the typical IgG Kozak sequence. The PCR primers are shown below.
Primer 1 (SEQ ID NO: 28):
   5'CAGTGTGATCTCGAGAATTCAGGACCTCACCATGGGATGGAGCTGTATCAT3'
Primer 2 (SEQ ID NO: 29):
   5'GTGTCTTCGGGTCTCAGGCTGT 3'

The PCR product was digested with XhoI and NruI and inserted back into the previously digested plasmid backbone.

Next, the "good" Kozak MN14 heavy chain gene construct was digested with EcoRI and the heavy chain gene containing fragment was isolated. The IRES α-Lactalbumin Signal Peptide MN14 light chain gene construct was also digested with EcoRI. The heavy chain gene was then cloned into the EcoRI site of IRES light chain construct. This resulted in the heavy chain gene being placed at the 5' end of the IRES sequence.

A multiple cloning site was then added to the LNCX retroviral backbone plasmid. The LNCX plasmid was digested with HindIII and ClaI. Two oligonucleotide primers were produced and annealed together to create an double stranded DNA multiple cloning site. The following primers were annealed together.
Primer 1 (SEQ ID NO: 30):
   5' AGCTTCTCGAGTTAACAGATCTAGGCCTCCTAGGTCGACAT 3'
Primer 2 (SEQ ID NO: 31):
   5' CGATGTCGACCTAGGAGGCCTAGATCTGTTAACTCGAGA 3'
After annealing the multiple cloning site was ligated into LNCX to create LNC-MCS.

The double chain gene fragment was then inserted into a retroviral backbone gene construct. The double chain gene construct created in step 3 was digested with SalI and BglII and the double chain containing fragment was isolated. The retroviral expression plasmid LNC-MCS was digested with XhoI and BglII. The double chain fragment was then cloned into the LNC-MCS retroviral expression backbone.

Next, a RNA splicing problem in the construct was repaired. The construct was digested with NsiI. The resulting fragment was then partially digested with EcoRI. The fragments resulting from the partial digest that were approximately 9300 base pairs in size, were gel purified. A linker was created to mutate the splice donor site at the 3' end of the MN14 heavy chain gene. The linker was again created by annealing two oligonucleotide primers together to form the double stranded DNA linker. The two primers used to create the linker are shown below.

After annealing the linker was substituted for the original NsiI/EcoRI fragment that was removed during the partial digestion.

Next, the mutated double chain fragment was inserted into the α-Lactalbumin expression retroviral backbone LN α-LA-Mertz-MCS. The gene construct produced above was digested with BamHI and BglII and the mutated double chain gene containing fragment was isolated. The LN α-LA-Mertz-MCS retroviral backbone plasmid was digested with BglII. The BamHI/BglII fragment was then inserted into the retroviral backbone plasmid.

A WPRE element was then inserted into the gene construct. The plasmid BluescriptII SK+ WPRE-B11 was digested with BaraHI and HincII to remove the WPRE element and the element was isolated. The vector created above was digested with BglII and HpaI. The WPRE fragment was ligated into the BglII and HpaI sites to create the final gene construct.

### E. α-LA Bot

The α-LA Bot (SEQ IlJ NO:8, botulinum toxin antibody) construct comprises the following elements, arranged in 5' to 3' order: bovine/human alpha-lactalbumin hybrid promoter, mutated PPE element, cc49 signal peptide, botulinum toxin antibody light chain, IRES from encephalomyocarditis virus/ bovine α-LA signal peptide, botulinum toxin antibody heavy chain, WPRE sequence, and 3' MoMuLV LTR. In addition, the α-LA botulinum toxin antibody vector further comprises a 5' MoMuLV LTR, a MoMuLV extended viral packaging signal, and a neomycin phosphotransferase gene (these additional elements are provided in SEQ ID NO: 7).

This construct uses the 5' MoMuLV LTR to control production of the neomycin phosphotransferase gene. The expression of botulinum toxin antibody is controlled by the hybrid α-LA promoter. The botulinum toxin antibody light chain gene and heavy chain gene are attached together by an IRES/ bovine α-LA signal peptide sequence. The bovine/human alpha-lactalbumin hybrid promoter drives production of a mRNA containing the light chain gene and the heavy chain gene attached by the IRES. Ribosomes attach to the RNA at the CAP site and at the IRES sequence. This allows both light and heavy chain protein to be produced from a single mRNA.

In addition, there are two genetic elements contained within the mRNA to aid in export of the mRNA from the nucleus to the cytoplasm and aid in poly-adenylation of the mRNA. The mutated PPE sequence (SEQ ID NO:2) is contained between the RNA CAP site and the start of the MN14 protein coding region. ATG sequences within the PPE element (SEQ ID NO:2) were mutated to prevent potential unwanted translation initiation. Two copies of this mutated sequence were used in a head to tail array. This sequence was placed just downstream of the promoter and upstream of the Kozak sequence and signal peptide-coding region. The WPRE was isolated from woodchuck hepatitis virus and also aids in the export of mRNA from the nucleus and creating stability in the mRNA. If this sequence is included in the 3' untranslated region of the RNA, level of protein expression from this RNA increases up to 10-fold. The WPRE is contained between the end of MN14 protein coding and the poly-adenylation site. The mRNA expression from the LTR as well as from the bovine/human alpha-lactalbumin hybrid promoter is terminated and poly adenylated in the 3' LTR.

The bovine/human α-lactalbumin hybrid promoter (SEQ ID NO:1) is a modular promoter/enhancer element derived from human and bovine α-lactalbumin promoter sequences. The human portion of the promoter is from +15 relative to transcription start point to -600 relative to the tsp. The bovine portion is then attached to the end of the human portion and corresponds to -550 to -2000 relative to the tsp. The hybrid was developed to remove poly-adenylation signals that were present in the bovine promoter and hinder retroviral RNA production. It was also developed to contain genetic control elements that are present in the human gene, but not the bovine. Likewise, the construct contains control elements present in the bovine but not in the human.

### F. LSRNL

The LSRNL (SEQ ID NO:9) construct comprises the following elements, arranged in 5' to 3' order: 5' MoMuLV LTR, MoMuLV viral packaging signal; hepatitis B surface antigen; RSV promoter; neomycin phosphotransferase gene; and 3' MoMuLV LTR.

This construct uses the 5' MoMuLV LTR to control production of the Hepatitis B surface antigen gene. The expression of the neomycin phosphotransferase gene is controlled by the RSV promoter. The mRNA expression from the LTR as well as from the RSV promoter is terminated and poly adenylated in the 3' LTR.

### G. α-LA cc49IL2

The α-LA cc49IL2 (SEQ ID NO:10; the cc49 antibody is described in U.S. Pat. Nos. 5,512,443; 5,993,813; and 5,892,019; each of which is herein incorporated by reference) construct comprises the following elements, arranged in 5' to 3' order: 5' bovine/human α-lactalbumin hybrid promoter; cc49-IL2 coding region; and 3' MoMuLV LTR. This gene construct expresses a fusion protein of the single chain antibody cc49 attached to Interleukin-2. Expression of the fusion protein is controlled by the bovine/human α-lactalbumin hybrid promoter.

The bovine/human α-lactalbumin hybrid promoter (SEQ ID NO:1) is a modular promoter/enhancer element derived from human and bovine alpha-lactalbumin promoter sequences. The human portion of the promoter is from +15 relative to transcription start point to -600 relative to the tsp. The bovine portion is then attached to the end of the human portion and corresponds to -550 to -2000 relative to the tsp. The hybrid was developed to remove poly-adenylation signals that were present in the bovine promoter and hinder retroviral RNA production. It was also developed to contain genetic control elements that are present in the human gene, but not the bovine. Likewise, the construct contains control elements present in the bovine but not in the human. The 3' viral LTR provide the poly-adenylation sequence for the mRNA.

### H. α-LA YP

The α-LA YP (SEQ ID NO: 11) construct comprises the following elements, arranged in 5' to 3' order: 5' bovine/human alpha-lactalbumin hybrid promoter; double mutated PPE sequence; bovine αLA signal peptide; *Yersenia pestis* antibody heavy chain Fab coding region; EMCV IRES/ bovine α-LA signal peptide; *Yersenia pestis* antibody light chain Fab coding region; WPRE sequence; 3' MoMuLV LTR.

This gene construct will cause the expression of *Yersenia pestis* mouse Fab antibody. The expression of the gene construct is controlled by the bovine/human α-lactalbumin hybrid promoter. The PPE sequence and the WPRE sequence aid in moving the mRNA from the nucleus to the cytoplasm. The IRES sequence allows both the heavy and the light chain genes to be translated from the same mRNA. The 3' viral LTR provides the poly-adenylation sequence for the mRNA.

In addition, there are two genetic elements contained within the mRNA to aid in export of the mRNA from the nucleus to the cytoplasm and aid in poly-adenylation of the mRNA. The mutated PPE sequence (SEQ ID NO:2) is contained between the RNA CAP site and the start of the MN14 protein coding region. ATG sequences within the PPE element (SEQ ID NO:2) were mutated (bases 4, 112, 131, and 238 of SEQ ID NO: 2 were changed from a G to a T) to prevent potential unwanted translation initiation. Two copies of this mutated sequence were used in a head to tail array. This sequence was placed just downstream of the promoter and upstream of the Kozak sequence and signal peptide-coding region. The WPRE was isolated from woodchuck hepatitis virus and also aids in the export of mRNA from the nucleus and creating stability in the mRNA. If this sequence is included in the 3' untranslated region of the RNA, level of protein expression from this RNA increases up to 10-fold. The WPRE is contained between the end of MN14 protein coding and the poly-adenylation site. The mRNA expression from the LTR as well as from the bovine/human alpha-lactalbumin hybrid promoter is terminated and poly adenylated in the 3' LTR.

The bovine/human alpha-lactalbumin hybrid promoter (SEQ ID NO:1) is a modular promoter /enhancer element derived from human and bovine alpha-lactalbumin promoter sequences. The human portion of the promoter is from +15 relative to transcription start point to -600 relative to the tsp. The bovine portion is then attached to the end of the human portion and corresponds to -550 to -2000 relative to the tsp. The hybrid was developed to remove poly-adenylation signals that were present in the bovine promoter and hinder retroviral RNA production. It was also developed to contain genetic control elements that are present in the human gene, but not the bovine. Likewise, the construct contains control elements present in the bovine but not in the human.

### Example 2

### Generation of Cell Lines Stably Expressing the MoMLV gag and pol Proteins

Examples 2-5 describe the production of pseudotyped retroviral vectors. These methods are generally applicable to the production of the vectors described above. The expression of the fusogenic VSV G protein on the surface of cells results in syncytium formation and cell death. Therefore, in order to produce retroviral particles containing the VSV G protein as the membrane-associated protein a two-step approach was taken. First, stable cell lines expressing the gag and pol proteins from MoMLV at high levels were generated (*e.g*., 293GP^{SD} cells). The stable cell line which expresses the gag and pol proteins produces noninfectious viral particles lacking a membrane-associated protein (*e.g*., an envelope protein). The stable cell line was then co-transfected, using the calcium phosphate precipitation, with VSV-G and gene of interest plasmid DNAs. The pseudotyped vector generated was used to infect 293GP^{SD} cells to produce stably transformed cell lines. Stable cell lines can be transiently transfected with a plasmid capable of directing the high level expression of the VSV G protein (see below). The transiently transfected cells produce VSV G-pseudotyped retroviral vectors which can be collected from the cells over a period of 3 to 4 days before the producing cells die as a result of syncytium formation.

The first step in the production of VSV G-pseudotyped retroviral vectors, the generation of stable cell lines expressing the MoMLV *gag* and *pol* proteins is described below. The human adenovirus Ad-5-transformed embryonal kidney cell line 293 (ATCC CRL 1573) was cotransfected with the pCMVgag-pol and the gene encoding for phleomycin. pCMV gag-pol contains the MoMLV *gag* and *pol* genes under the control of the CMV promoter (pCMV gag-pol is available from the ATCC).

The plasmid DNA was introduced into the 293 cells using calcium phosphate co-precipitation (Graham and Van der Eb, Virol. 52:456 [1973]). Approximately 5 x 10⁵ 293 cells were plated into a 100 mm tissue culture plate the day before the DNA co-precipitate was added. Stable transformants were selected by growth in DMEM-high glucose medium containing 10% FCS and 10 µg/ml phleomycin (selective medium). Colonies which grew in the selective medium were screened for extracellular reverse transcriptase activity (Goff *et al.,* J. Virol. 38:239 [1981]) and intracellular p30gag expression. The presence of p30gag expression was determined by Western blotting using a goat-anti p30 antibody (NCI antiserum 77S000087). A clone which exhibited stable expression of the retroviral genes was selected. This clone was named 293GP^{SD} (293 gag-pol-San Diego). The 293GP^{SD} cell line, a derivative of the human Ad-5-transformed embryonal kidney cell line 293, was grown in DMEM-high glucose medium containing 10% FCS.

### Example 3

### Preparation of Pseudotyped Retroviral Vectors Bearing the G Glycoprotein of VSV

In order to produce VSV G protein pseudotyped retrovirus the following steps were taken. The 293GP^{SD} cell line was co-transfected with VSV-G plasmid and DNA plasmid of interest. This co-transfection generates the infectious particles used to infect 293GP^{SD} cells to generate the packaging cell lines. This Example describes the production of pseudotyped LNBOTDC virus. This general method may be used to produce any of the vectors described in Example 1.

### a) Cell Lines and Plasmids

The packaging cell line, 293GP^{SD} was grown in alpha-MEM-high glucose medium containing 10% FCS The titer of the pseudo-typed virus may be determined using either 208F cells (Quade, Virol. 98:461 [1979]) or NIH/3T3 cells (ATCC CRL 1658); 208F and NIH/3T3 cells are grown in DMEM-high glucose medium containing 10% CS.

The plasmid LNBOTDC contains the gene encoding BOTD under the transcriptional control of cytomegalovirus intermediate-early promoter followed by the gene encoding neomycin phosphotransferase (Neo) under the transcriptional control of the LTR promoter. The plasmid pHCMV-G contains the VSV G gene under the transcriptional control of the human cytomegalovirus intermediate-early promoter (Yee *et al*., Meth. Cell Biol. 43:99 [1994]).

### b) Production of stable packaging cell lines, pseudotyped vector and Titering of Pseudotyped LNBOTDC Vector

LNBOTDC DNA (SEQ ID NO: 13) was co-transfected with pHCMV-G DNA into the packaging line 293GP^{SD} to produce LNBOTDC virus. The resulting LNBOTDC virus was then used to infect 293GP^{SD} cells to transform the cells. The procedure for producing pseudotyped LNBOTDC virus was carried out as described (Yee *et al*., Meth. Cell Biol. 43:99 [1994].

This is a retroviral gene construct that upon creation of infectious replication defective retroviral vector will cause the insertion of the sequence described above into the cells of interest. Upon insertion the CMV regulatory sequences control the expression of the botulinum toxin antibody heavy and light chain genes. The IRES sequence allows both the heavy and the light chain genes to be translated from the same mRNA. The 3' viral LTR provides the poly-adenylation sequence for the mRNA.

Both heavy and light chain protein for botulinum toxin antibody are produced from this signal mRNA. The two proteins associated to form active botulinum toxin antibody. The heavy and light chain proteins also appear to be formed in an equal molar ratio to each other.

Briefly, on day 1, approximately 5 x 10⁴ 293GP^{SD} cells were placed in a 75 cm² tissue culture flask. On the following day (day 2), the 293GP^{SD} cells were transfected with 25 µg of pLNBOTDC plasmid DNA and 25 pg of VSV-G plasmid DNA using the standard calcium phosphate co-precipitation procedure (Graham and Van der Eb, Virol. 52:456 [1973]). A range of 10 to 40 µg of plasmid DNA may be used. Because 293GP^{SD} cells may take more than 24 hours to attach firmly to tissue culture plates, the 293GP^{SD} cells may be placed in 75 cm² flasks 48 hours prior to transfection. The transfected 293GP^{SD} cells provide pseudotyped LNBOTDC virus.

On day 3, approximately 1 x 10⁵ 293GP^{SD} cells were placed in a 75 cm² tissue culture flask 24 hours prior to the harvest of the pseudotyped virus from the transfected 293GP^{SD} cells. On day 4, culture medium was harvested from the transfected 2093GP^{SD} cells 48 hours after the application of the pLNBOTDC and VSV-G DNA. The culture medium was filtered through a 0.45 µm filter and polybrene was added to a final concentration of 8 µg/ml. The culture medium containing LNBOTDC virus was used to infect the 293GP^{SD} cells as follows. The culture medium was removed from the 293GP^{SD} cells and was replaced with the LNBOTDC virus containing culture medium. Polybrene was added to the medium following addition to cells. The virus containing medium was allowed to remain on the 293GP^{SD} cells for 24 hours. Following the 16 hour infection period (on day 5), the medium was removed from the 293GP^{SD} cells and was replaced with fresh medium containing 400 µg/ml G418 (GIBCO/BRL). The medium was changed approximately every 3 days until G418-resistant colonies appeared approximately two weeks later.

The G418-resistant 293 colonies were plated as single cells in 96 wells. Sixty to one hundred G418-resistant colonies were screened for the expression of the BOTDC antibody in order to identify high producing clones. The top 10 clones in 96-well plates were transferred 6-well plates and allowed to grow to confluency.

The top 10 clones were then expanded to screen for high titer production. Based on protein expression and titer production, 5 clonal cell lines were selected. One line was designated the master cell bank and the other 4 as backup cell lines. Pseudotyped vector was generated as follows. Approximately 1 x 10⁶ 293GP^{SD}/LNBOTDC cells were placed into a 75cm² tissue culture flask. Twenty-four hours later, the cells were transfected with 25 µg of pHCMV-G plasmid DNA using calcium phosphate co-precipitation. Six to eight hours after the calcium-DNA precipitate was applied to the cells, the DNA solution was replaced with fresh culture medium (lacking G418). Longer transfection times (overnight) were found to result in the detachment of the majority of the 293GP^{SD}/LNBOTDC cells from the plate and are therefore avoided. The transfected 293GP^{SD}/LNBOTDC cells produce pseudotyped LNBOTDC virus.

The pseudotyped LNBOTDC virus generated from the transfected 293GP^{SD}/LNBOTDC cells can be collected at least once a day between 24 and 96 hr after transfection. The highest virus titer was generated approximately 48 to 72 hr after initial pHCMV-G transfection. While syncytium formation became visible about 48 hr after transfection in the majority of the transfected cells, the cells continued to generate pseudotyped virus for at least an additional 48 hr as long as the cells remained attached to the tissue culture plate. The collected culture medium containing the VSV G-pseudotyped LNBOTDC virus was pooled, filtered through a 0.45 µm filter and stored at -80°C or concentrated immediately and then stored at -80°C.

The titer of the VSV G-pseudotyped LNBOTDC virus was then determined as follows. Approximately 5 x 10⁴ rat 208F fibroblasts cells were plated into 6 well plates. Twenty-fours hours after plating, the cells were infected with serial dilutions of the LNBOTDC virus-containing culture medium in the presence of 8 µg/ml polybrene. Twenty four hours after infection with virus, the medium was replaced with fresh medium containing 400 µg/ml G418 and selection was continued for 14 days until G418-resistant colonies became visible. Viral titers were typically about 0.5 to 5.0 x 10⁶ colony forming units (cfu)/ml. The titer of the virus stock could be concentrated to a titer of greater than 10⁹ cfu/ml as described below.

### Example 4

### Concentration of Pseudotyped Retroviral Vectors

The VSV G-pseudotyped LNBOTDC viruses were concentrated to a high titer by one cycle of ultracentrifugation. However, two cycles can be performed for further concentration. The frozen culture medium collected as described in Example 2 which contained pseudotyped LNBOTDC virus was thawed in a 37°C water bath and was then transferred to Oakridge centrifuge tubes (50 ml Oakridge tubes with sealing caps, Nalge Nunc International) previously sterilized by autoclaving. The virus was sedimented in a JA20 rotor (Beckman) at 48,000 x g (20,000 rpm) at 4°C for 120 min. The culture medium was then removed from the tubes in a biosafety hood and the media remaining in the tubes was aspirated to remove the supernatent. The virus pellet was resuspended to 0.5 to 1% of the original volume of culture medium DMEM. The resuspended virus pellet was incubated overnight at 4°C without swirling. The virus pellet could be dispersed with gentle pipetting after the overnight incubation without significant loss of infectious virus. The titer of the virus stock was routinely increased 100- to 300-fold after one round of ultracentrifugation. The efficiency of recovery of infectious virus varied between 30 and 100%.

The virus stock was then subjected to low speed centrifugation in a microfuge for 5 min at 4°C to remove any visible cell debris or aggregated virions that were not resuspended under the above conditions. It was noted that if the virus stock is not to be used for injection into oocytes or embryos, this centrifugation step may be omitted.

The virus stock can be subjected to another round of ultracentrifugation to further concentrate the virus stock. The resuspended virus from the first round of centrifugation is pooled and pelleted by a second round of ultracentrifugation which is performed as described above. Viral titers are increased approximately 2000-fold after the second round of ultracentrifugation (titers of the pseudotyped LNBOTDC virus are typically greater than or equal to 1 x 10⁹ cfu/ml after the second round of ultracentrifugation).

The titers of the pre- and post-centrifugation fluids were determined by infection of 208F cells (NIH 3T3 or bovine mammary epithelial cells can also be employed) followed by selection of G418-resistant colonies as described above in Example 2.

### Example 5

### Preparation of Pseudotyped Retrovirus For Infection of Host Cells

The concentrated pseudotyped retroviruses were resuspended in 0.1X HBS (2.5 mM HEPES, pH 7.12, 14 mM NaCl, 75 µM Na₂HPO₄-H₂O) and 18 µl aliquots were placed in 0.5 ml vials (Eppendorf) and stored at -80°C until used. The titer of the concentrated vector was determined by diluting 1µl of the concentrated virus 10⁻⁷- or 10⁻⁸-fold with 0.1X HBS. The diluted virus solution was then used to infect 208F and bovine mammary epithelial cells and viral titers were determined as described in Example 2.

### Example 6

### Expression of MN14 by Host Cells

This Example describes the production of antibody MN14 from cells transfected with a high number of integrating vectors. Pseudotyped vector were made from the packaging cell lines for the following vectors: CMV MN14, α-LA MN14, and MMTV MN14. Rat fibroblasts (208F cells), MDBK cells (bovine kidney cells), and bovine mammary epithelial cells were transfected at a multiplicity of infection of 1000. One thousand cells were plated in a T25 flask and 10⁶ colony forming units (CFU's) of vector in 3 ml media was incubated with the cells. The duration of the infection was 24 hr, followed by a media change. Following transfection, the cells were allowed to grow and become confluent.

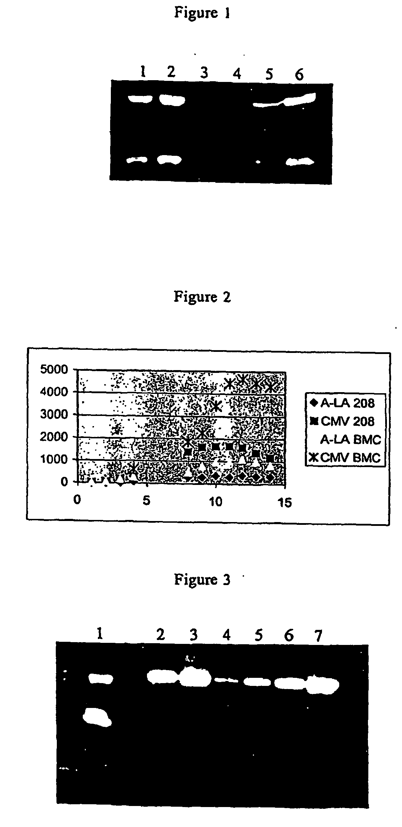

The cell lines were grown to confluency in T25 flasks and 5ml of media was changed daily. The media was assayed daily for the presence of MN14. All of the MN14 produced is active (an ELISA to detect human IgG gave the exact same values as the CEA binding ELISA) and Western blotting has shown that the heavy and light chains are produced at a ratio that appears to be a 1:1 ratio. In addition, a non-denaturing Western blot indicated that what appeared to be 100% of the antibody complexes were correctly formed *(See* Figure 1: Lane 1, 85 ng control Mn14; Lane 2, bovine mammary cell line, α-LA promoter; Lane 3, bovine mammary cell line, CMV promoter; Lane 4, bovine kidney cell line, α-LA promoter; Lane 5, bovine kidney cell line, CMV promoter; Lane 6, 208 cell line, α-LA promoter; Lane 7, 208 cell line, CMV promoter)).

Figure 2 is a graph showing the production of MN14 over time for four cell lines. The Y axis shows MN14 production in ng/ml of media. The X-axis shows the day of media collection for the experiment. Four sets of data are shown on the graph. The comparisons are between the CMV and α-LA promoter and between the 208 cells and the bovine mammary cells. The bovine mammary cell line exhibited the highest expression, followed by the 208F cells and MDBK cells. With respect to the constructs, the CMV driven construct demonstrated the highest level of expression, followed by the α-LA driven gene construct and the MMTV construct. At 2 weeks, the level of daily production of the CMV construct was 4.5 µg/ml of media (22.5 mg/day in a T25 flask). The level of expression subsequently increased slowly to 40 µg/day as the cells became very densely confluent over the subsequent week. 2.7 L of media from an α-lac-MN14 packaging cell line was processed by affinity chromatography to produce a purified stock of MN14.

Figure 3 is a western blot of a 15% SDS-PAGE gel run under denaturing conditions in order to separate the heavy and light chains of the MN14 antibody. Lane 1 shows MN14 from bovine mammary cell line, hybrid α-LA promoter; lane 2 shows MN14 from bovine mammary cell line, CMV promoter; lane 3 shows MN14 from bovine kidney cell line, hybrid αLA promoter; lane 4 shows MN14 from bovine kidney cell line, CMV promoter; lane 5 shows MN14 from rat fibroblast cell line, hybrid α-LA promoter; lane 6 shows MN14 from rat fibroblast, CMV promoter. In agreement with Figure 1 above, the results show that the heavy and light chains are produced in a ratio of approximately 1:1.

### Example 7

### Quantitation of Protein Produced Per Cell

This Example describes the quantitation of the amount of protein produced per cell in cell cultures produced according to the invention. Various cells (208F cells, MDBK cells, and bovine mammary cells) were plated in 25 cm² culture dishes at 1000 cells/dish. Three different vectors were used to infect the three cells types (CMV-MN14, MMTV-MN14, and α-LA-MN14) at an MOI of 1000 (titers: 2.8 X 10⁶, 4.9 X 10⁶, and 4.3 X 10⁶, respectively). Media was collected approximately every 24 hours from all cells. Following one month of media collection, the 208F and MDBK cells were discarded due to poor health and low MN14 expression. The cells were passaged to T25 flasks and collection of media from the bovine mammary cells was continued for approximately 2 months with continued expression of MN14. After two months in T25 flasks, the cells with CMV promoters were producing 22.5 pg/cell/day and the cells with α-LA promoters were producing 2.5 pg MN14/celVday.

After 2 months in T25 flasks, roller bottles (850 cm²) were seeded to scale-up production and to determine if MN14 expression was stable following multiple passages. Two roller bottles were seeded with bovine mammary cells expressing MN14 from a CMV promoter and two roller bottles were seeded with bovine mammary cells expressing MN14 from the α-LA promoter. The cultures reached confluency after approximately two weeks and continue to express MN14. Roller bottle expression is shown in Table 1 below.

| **Table 1** | | | |
|---|---|---|---|
| **Production of MN14 in Roller Bottles** | | | |
| Cell Line | Promoter | MN14 Production/ Week (µg/ml) | MN14 Production/ Week - Total (µg/ml) |
| Bovine mammary | CMV | 2.6 | 1 - 520 |
| Bovine mammary | CMV | 10.6 | 2 - 2120 |
| Bovine mammary | CMV | 8.7 | 3 - 1740 |
| Bovine mammary | CMV | 7.8 | 4 - 1560 |
| Bovine mammary | α-LA | 0.272 | 1 - 54.4 |
| Bovine mammary | α-LA | 2.8 | 2 - 560 |
| Bovine mammary | α-LA | 2.2 | 3 - 440 |
| Bovine mammary | α-LA | 2.3 | 4 - 460 |

### Example 8

### Transfection at Varied Multiplicities of Infection

This Example describes the effect of transfection at varied multiplicities of infection on protein expression. 208F rat fibroblast and bovine mammary epithelial cells (BMEC) were plated in a 25 cm² plates at varied cell numbers/25 cm². Cells were infected with either the CMV MN14 vector or the αLA MN14 vector at a MOI of 1,10, 1000, and 10,000 by keeping the number of CFUs kept constant and varying the number of cells infected.

Following infection, medium was changed daily and collected approximately every 24 hours from all cells for approximately 2 months. The results of both of the vectors in bovine mammary epithelial cells are shown in Table 2 below. Cells without data indicate cultures that became infected prior to the completion of the experiment. The "# cells" column represents the number of cells at the conclusion of the experiment. The results indicate that a higher MOI results in increased MN14 production, both in terms of the amount of protein produced per day, and the total accumulation.

| **Table 2** | | | | | | |
|---|---|---|---|---|---|---|
| **MOI vs. Protein Production** | | | | | | |
| Cell Line | Promoter | MOI | % cell Confluency | MN14 (ng/ml) | # Cells | MN14 Production /day (pg/cell) |
| BMEC | CMV | 10000 | 100% | 4228 | 4.5E5 | 47 |
| BMEC | CMV | 1000 | 100% | 2832 | 2.0E6 | 7.1 |
| BMEC | CMV | 100 | | | | |
| BMEC | CMV | 10 | 100% | 1873 | 2.5E6 | 3.75 |
| BMEC | CMV | 1 | | | | |
| BMEC | αLA | 10000 | 100% | 1024 | 1.5E6 | 3.4 |
| BMEC | αLA | 1000 | | | | |
| BMEC | αLA | 100 | 100% | 722 | 1.8E6 | 1.9 |
| BMEC | αLA | 10 | 100% | 421234 | 2.3E6 | .925 |
| BMEC | αLA | 1 | 100% | | 1.9E6 | .325 |

### Example 9

### Transfection at Varied Multiplicities of Infection

This experiment describes protein production from the CMV MN14 vector at a variety of MOI values. Bovine mammary cells, CHO cells, and human embryo kidney cells (293 cells) were plated in 24 well plates (2 cm²) at 100 cells/2 cm² well. Cells were infected at various dilutions with CMV MN14 to obtain MOI values of 1, 10, 100, 1000, and 10000. The CHO cells reached confluency at all MOI within 11 days of infection. However, the cells infected at a MOI of 10,000 grew more slowly. The bovine mammary and 293 cells grew slower, especially at the highest MOI of 10,000. The cells were then passaged into T25 flasks to disperse cells. Following dispersion, cells reached confluence within 1 week. the medium was collected after one week and analyzed for MN14 production. The CHO and human 293 cells did not exhibit good growth in extended culture. Thus, data were not collected from these cells. Data for bovine mammary epithelial cells are shown in Table 3 below. The results indicate that production of MN14 increased with higher MOI.

| **Table 3** | | | | |
|---|---|---|---|---|
| **MOI vs. Protein Production** | | | | |
| Cell Line | Promoter | MOI | % confluency | MN14 Production (ng/ml) |
| BMEC | CMV | 10000 | 100% | 1312 |
| BMEC | CMV | 1000 | 100% | 100 |
| BMEC | CMV | 100 | 100% | 7.23 |
| BMEC | CMV | 10 | 100% | 0 |
| BMEC | CMV | 1 | 100% | 0 |

### Example 10

### Expression of LL2 Antibody by Bovine Mammary Cells

This Example describes the expression of antibody LL2 by bovine mammary cells. Bovine mammary cells were infected with vector CMV LL2 (7.85 x 10⁷ CFU/ml) at MOI's of 1000 and 10,000 and plated in 25cm² culture dishes. None of the cells survived transfection at the MOI of 10,000. At 20% confluency, 250 ng/ml of LL2 was present in the media.

### Example 11

### Expression of Botulinum Toxin Antibody by Bovine Mammary Cells

This Example describes the expression of Botulinum toxin antibody in bovine mammary cells. Bovine mammary cells were infected with vector α-LA Bot (2.2 X 10² CFU/ml) and plated in 25cm² culture dishes. At 100% confluency, 6 ng/ml of Botulinum toxin antibody was present in the media.

### Example 12

### Expression of Hepatitis B Surface Antigen by Bovine Mammary Cells

This Example describes the expression of hepatitis B surface antigen (HBSAg) in bovine mammary cells. Bovine mammary cells were infected with vector LSRNL (350 CFU/ml) and plated in 25cm² culture dishes. At 100% confluency, 20 ng/ml of HBSAg was present in the media.

### Example 13

### Expression of cc49IL2 Antigen Binding Protein by Bovine Mammary Cells

This Example describes the expression of cc49IL2 in bovine mammary cells. Bovine mammary cells were infected with vector cc49IL2 (3.1 X 10⁵ CFU/ml) at a MOI of 1000 and plated in 25cm² culture dishes. At 100% confluency, 10 µg/ml of cc49IL2 was present in the media.

### Example 14

### Expression of Multiple Proteins by Bovine Mammary Cells

This Example describes the expression of multiple proteins in bovine mammary cells. Mammary cells producing MN14 (infected with CMV-MN14 vector) were infected with cc49IL2 vector (3.1 X 10⁵ CFU/ml) at an MOI of 1000, and 1000 cells were plated in 25cm² culture plates. At 100% confluency; the cells expressed MN14 at 2.5 µg/ml and cc49IL2 at 5 µg/ml.

### Example 15

### Expression of Multiple Proteins by Bovine Mammary Cells

This Example describes the expression of multiple proteins in bovine mammary cells. Mammary cells producing MN14 (infected with CMV-MN14 vector) were infected with LSNRL vector (100 CFU/ml) at an MOI of 1000, and 1000 cells were plated in 25cm² culture plates. At 100% confluency, the cells expressed MN14 at 2.5 µg/ml and hepatitis surface antigen at 150 ng/ml.

### Example 16

### Expression of Multiple Proteins by Bovine Mammary Cells

This Example describes the expression of multiple proteins in bovine mammary cells. Mammary cells producing hepatitis B surface antigen (infected with LSRNL vector) were infected with cc49IL2 vector at an MOI of 1000, and 1000 cells were plated in 25cm² culture plates. At 100% confluency, the cells expressed MN14 at 2.4 µg/ml and hepatitis B surface antigen at 13 ng/ml. It will be understood that multiple proteins may be expressed in the other cell lines described above.

### Example 17

### Expression of Hepatitis B Surface Antigen and Botulinum Toxin Antibody in Bovine Mammary Cells

This Example describes the culture of transfected cells in roller bottle cultures. 208F cells and bovine mammary cells were plated in 25cm² culture dishes at 1000 cells/25cm². LSRNL or α-LA Bot vectors were used to infect each cell line at a MOI of 1000. Following one month of culture and media collection, the 208F cells were discarded due to poor growth and plating. Likewise, the bovine mammary cells infected with α-LA Bot were discarded due to low protein expression. The bovine mammary cells infected with LSRNL were passaged to seed roller bottles (850 cm²). Approximately 20 ng/ml hepatitis type B surface antigen was produced in the roller bottle cultures.

### Example 18

### Expression in Clonally Selected Cell Lines

This experiment describes expression of MN14 from clonally selected cell lines. Cell lines were grown to confluency in T25 flasks and 5ml of media were collected daily. The media was assayed daily for the presence of MN14. All the MN14 produced was active and Western blotting indicated that the heavy and light chains were produce at a ratio that appears to be almost exactly 1:1. In addition, a non-denaturing western blot indicated that approximately 100% of the antibody complexes were correctly formed. After being in culture for about two months, the cells were expanded into roller bottles or plated as single cell clones in 96 well plates.

The production of MN14 in the roller bottles was analyzed for a 24 hour period to determine if additional medium changing would increase production over what was obtained with weekly medium changes. Three 24 hour periods were examined. The CMV promoter cells in 850 cm² roller bottles produced 909 ng/ml the first day, 1160 ng/ml the second day and 1112 ng/ml the third day. The α-LA promoter cells produced 401 ng/ml the first day, 477 ng/ml the second day and 463 ng/ml the third day. These values correspond well to the 8-10 mg/ml/week that were obtained for the CMV cells and the 2-3 mg/ml that were obtained for the α-LA cells. It does not appear that more frequent media changing would increase MN14 production in roller bottles.

Single cell lines were established in 96 well plates and then passaged into the same wells to allow the cells to grow to confluency. Once the cells reached confluency, they were assayed for MN14 production over a 24 hour period. The clonal production of MN14 from CMV cell lines ranged from 19 ng/ml/day to 5500 ng/ml/day. The average production of all cell clones was 1984 ng/ml/day. The α-LA cell clones yielded similar results. The clonal production of MN14 from α-LA cell lines ranged from 1 ng/ml/day to 2800 ng/ml/day. The average production of these cell clones was 622 ng/ml/day. The results are provided in Table 4 below.

| **Table 4** | | | |
|---|---|---|---|
| **Expression in Clonal Cell Lines** | | | |
| CMV Clonal Cell Line Number | MN14 Production (ng/ml) | Alpha-lactalbumin Clonal Cell Line Number | MN14 Production (ng/ml) |
| 22 | 19 | 27 | 0 |
| 6 | 88 | 29 | 0 |
| 29 | 134 | 12 | 0.7 |
| 34 | 151 | 50 | 8 |
| 32 | 221 | 28 | 55 |
| 23 | 343 | 43 | 57 |
| 27 | 423 | 8 | 81 |
| 4 | 536 | 13 | 154 |
| 41 | 682 | 48 | 159 |
| 45 | 685 | 7 | 186 |
| 40 | 696 | 36 | 228 |
| 11 | 1042 | 39 | 239 |
| 8 | 1044 | 51 | 275 |
| 5 | 1066 | 31 | 283 |
| 19 | 1104 | 54 | 311 |
| 48 | 1142 | 38 | 317 |
| 12 | 1224 | 21 | 318 |
| 26 | 1315 | 16 | 322 |
| 39 | 1418 | 47 | 322 |
| 37 | 1610 | 17 | 325 |
| 20 | 1830 | 37 | 367 |
| 21 | 1898 | 45 | 395 |
| 47 | 1918 | 25 | 431 |
| 35 | 1938 | 5 | 441 |
| 15 | 1968 | 20 | 449 |
| 3 | 1976 | 19 | 454 |
| 28 | 1976 | 22 | 503 |
| 1 | 2166 | 55 | 510 |
| 16 | 2172 | 14 | 519 |
| 17 | 2188 | 41 | 565 |
| 33 | 2238 | 46 | 566 |
| 30 | 2312 | 23 | 570 |
| 38 | 2429 | 1 | 602 |
| 2 | 2503 | 9 | 609 |
| 14 | 2564 | 53 | 610 |
| 24 | 2571 | 56 | 631 |
| 9 | 2708 | 2 | 641 |
| 42 | 2729 | 40 | 643 |
| 44 | 2971 | 32 | 653 |
| 7 | 3125 | 24 | 664 |
| 43 | 3125 | 26 | 671 |
| 25 | 3650 | 52 | 684 |
| 46 | 3706 | 6 | 693 |
| 50 | 3947 | 33 | 758 |
| 49 | 4538 | 42 | 844 |
| 18 | 4695 | 10 | 1014 |
| 31 | 4919 | 3 | 1076 |
| 10 | 5518 | 44 | 1077 |
| | | 35 | 1469 |
| | | 34 | 1596 |
| | | 18 | 1820 |
| | | 30 | 2021 |
| | | 11 | 2585 |
| | | 4 | 2800 |

### Example 19

### Estimation of Insert Copy Number

This example describes the relationship of multiplicity of infection, gene copy number, and protein expression. Three DNA assays were developed using the INVADER Assay system (Third Wave Technologies, Madison, WI). One of the assays detects a portion of the bovine α-lactalbumin 5' flanking region. This assay is specific for bovine and does not detect the porcine or human α-lactalbumin gene. This assay will detect two copies of the α-lactalbumin gene in all control bovine DNA samples and also in bovine mammary epithelial cells. The second assay detects a portion of the extended packaging region from the MLV virus. This assay is specific for this region and does not detect a signal in the 293 human cell line, bovine mammary epithelial cell line or bovine DNA samples. Theoretically, all cell lines or other samples not infected with MLV should not produce a signal. However, since the 293GP cell line was produced with the extended packaging region of DNA, this cell line gives a signal when the assay is run. From the initial analysis, it appears that the 293GP cell line contains two copies of the extended packing region sequence that are detected by the assay. The final assay is the control assay. This assay detects a portion of the insulin-like growth factor I gene that is identical in bovine, porcine, humans and a number of other species. It is used as a control on every sample that is run in order to determine the amount of signal that is generated from this sample for a two copy gene. All samples that are tested should contain two copies of the control gene.

DNA samples can be isolated using a number of methods. Two assays are then performed on each sample. The control assay is performed along with either the bovine α-lactalbumin assay or the extended packaging region assay. The sample and the type of information needed will determine which assay is run. Both the control and the transgene detection assay are run on the same DNA sample, using the exact same quantity of DNA.

The data resulting from the assay are as follows (Counts indicate arbitrary fluorescence units):
Extended Packaging Region or α-Lactalbumin Background counts
Extended Packaging Region or α-Lactalbumin counts
Internal Control background counts
Internal Control counts

To determine net counts for the assay the background counts are subtracted from the actual counts. This occurs for both the control and transgene detection assay. Once the net counts are obtained, a ratio of the net counts for the transgene detection assay to the net counts of the control assay can be produced. This value is an indication of the number of copies of transgene compared to the number of copies of the internal control gene (in this case IGF-I). Because the transgene detection assay and the control assay are two totally different assays, they do not behave exactly the same. This means that one does not get an exact 1:1 ratio if there are two copies of the transgene and two copies of the control gene in a specific sample. However the values are generally close to the 1:1 ratio. Also, different insertion sites for the transgene may cause the transgene assay to behave differently depending on where the insertions are located.

Therefore, although the ratio is not an exact measure of copy number, it is a good indication of relative copy number between samples. The greater the value of the ratio the greater the copy number of the transgene. Thus, a ranking of samples from lowest to highest will give a very accurate comparison of the samples to one another with regard to copy number. Table 5 provides actual data for the EPR assay:

| **Table 5** | | | | | | | |
|---|---|---|---|---|---|---|---|
| Sample # | Control Counts | Control Background Counts | Net Control Counts | Transgene Counts | Transgene Background Counts | Net Transgene Counts | Net Ratio |
| 293 | 116 | 44 | 72 | 46.3 | 46 | 0.3 | 0 |
| 293GP | 112 | 44 | 68 | 104 | 46 | 58 | .84 |
| 1 | 74 | 40 | 34 | 88 | 41 | 47 | 1.38 |
| 2 | 64 | 40 | 24 | 83 | 41 | 43 | 1.75 |
| 3 | 62 | 44 | 18 | 144 | 46 | 98 | 5.57 |

From this data, it can be determined that the 293 cell line has no copies of the extended packaging region/transgene. However the 293 GP cells appear to have two copies of the extended packaging region. The other three cell lines appear to have three or more copies of the extended packaging region (one or more additional copies compared to 293GP cells).

### Invader Assay Gene Ratio and Cell Line Protein Production

Bovine mammary epithelial cells were infected with either the CMV driven MN14 construct or the α-lactalbumin driven MN14 construct. The cells were infected at a 1000 to 1 vector to cell ratio. The infected cells were expanded. Clonal cell lines were established for both the α-LA and CMV containing cells from this initial pooled population of cells. Approximately 50 cell lines were produced for each gene construct. Individual cells were placed in 96 well plates and then passaged into the same well to allow the cells to grow to confluency. Once the cells lines reached confluency, they were assayed for MN14 production over a 24 hour period. The clonal production of MN14 from CMV cell lines ranged from 0 ng/ml/day to 5500 ng/ml/day. The average production of all cell clones was 1984 ng/ml/day. The α-LA cell clones showed similar trends. The clonal production of MN14 from α-LA cell lines ranged from 0 ng/ml/day to 2800 ng/ml/day. The average production of these cell clones was 622 ng/ml/day.

For further analysis of these clonal lines, fifteen CMV clones and fifteen α-LA clones were selected. Five highest expressing, five low expressing and five mid-level expressing lines were chosen. These thirty cell lines were expanded and banked. DNA was isolated from most all of the thirty cell lines. The cell lines were passed into 6 well plates and grown to confluency. Once at confluency, the media was changed every 24 hours and two separate collections from each cell line were assayed for MN14 production. The results of these two assays were averaged and these numbers were used to create Tables 6 and 7 below. DNA from the cell lines was run using the Invader extended packaging region assay and the results are shown below. The Tables show the cell line number, corresponding gene ratio and antibody production.

| **Table 6** | | |
|---|---|---|
| CMV Clonal Cell Line Number | Invader Gene | Ratio MN14 Production (ng/ml) |
| 6 | 0.19 | 104 |
| 7 | 1.62 | 2874 |
| 10 | 2.57 | 11202 |
| 18 | 3.12 | 7757 |
| 19 | 1.62 | 2483 |
| 21 | 1.53 | 3922 |
| 22 | 0 | 0 |
| 29 | 0.23 | 443 |
| 31 | 3.45 | 5697 |
| 32 | 0.27 | 346 |
| 34 | 0.37 | 305 |
| 38 | 1.47 | 2708 |
| 41 | 1.54 | 5434 |
| 49 | 2.6 | 7892 |
| 50 | 1.56 | 5022 |
| Average of All Clones | 1.48 | 3746 |

| **Table 7** | | |
|---|---|---|
| α-LA Clonal Cell Line Number | Invader Gene Ratio | MN14 Production (ng/ml) |
| 4 | 4.28 | 3600 |
| 6 | 1.15 | 959 |
| 12 | 0.35 | 21 |
| 17 | 0.54 | 538 |
| 28 | 0.75 | 60 |
| 30 | 1.73 | 2076 |
| 31 | 0.74 | 484 |
| 34 | 4.04 | 3332 |
| 41 | 1.33 | 771 |
| Average of All Clones | 1.66 | 1316 |

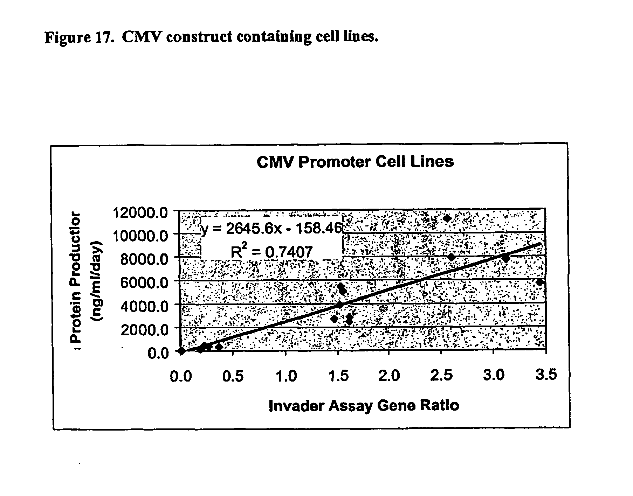

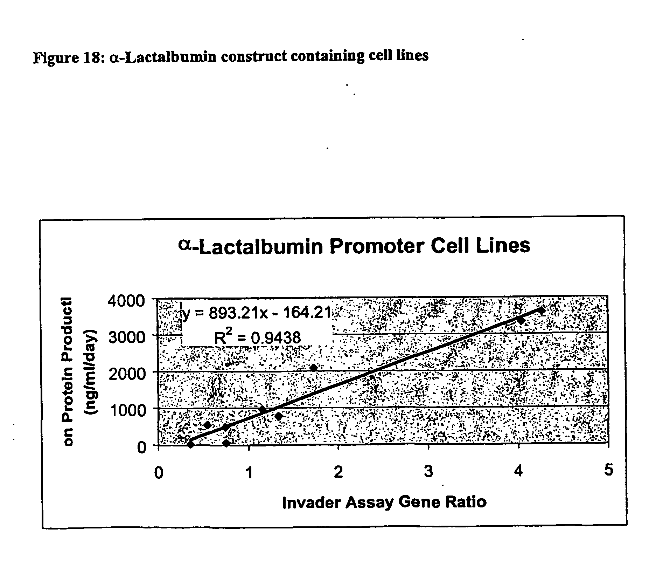

The graphs (Figs. 17 and 18) show the comparison between protein expression and invader assay gene ratio. The results indicate that there is a direct correlation between invader assay gene ratio and protein production. It also appears that the protein production has not reached a maximum and if cells containing a higher invader assay gene ratio were produced, higher protein production would occur.

### Invader Assay Gene Ratio and Multiple Cell Line Infections

Two packaging cell lines (293GP) produced using previously described methods were used to produce replication defective retroviral vector. One of the cell lines contains a retroviral gene construct that expresses the botulinum toxin antibody gene from the CMV promoter (LTR-Extended Viral Packaging Region-Neo Gene-CMV Promoter-Bot Light Chain Gene-IRES-Bot Heavy Chain Gene-LTR), the other cell line contains a retroviral gene construct that expresses the YP antibody gene from the CMV promoter (LTR-Extended Viral Packaging Region-Neo Gene-CMV Promoter-YP Heavy Chain Gene-IRES-YP Light Chain Gene-WPRE-LTR). In addition to being able to produce replication defective retroviral vector, each of these cell lines also produce either botulinum toxin antibody or YP antibody.

The vector produced from these cell lines was then used to re-infect the parent cell line. This procedure was performed in order to increase the number of gene insertions and to improve antibody production from these cell lines. The botulinum toxin parent cell line was infected with a new aliquot of vector on three successive days. The titer of the vector used to perform the infection was 1 X 10⁸ cfu/ml. Upon completion of the final 24 hour infection, clonal selection was performed on the cells and the highest protein producing line was established for botulinum toxin antibody production. A similar procedure was performed on the YP parent cell line. This cell line was also infected with a new aliquot of vector on three successive days. The titer of the YP vector aliquots was 1 X 10⁴. Upon completion of the final 24 hour infection, clonal selection was performed on the cells and the highest protein producing line was established for YP production.

Each of the parent cell lines and the daughter production cell lines were examined for Invader gene ratio using the extended packaging region assay and for protein production. The Bot production cell line which was generated using the highest titer vector had the highest gene ratio. It also had the highest protein production, again suggesting that gene copy number is proportional to protein production. The YP production cell line also had a higher gene ratio and produced more protein than its parent cell line, also suggesting that increasing gene copy is directly related to increases in protein production. The data is presented in Table 8.

| **Table 8** | | |
|---|---|---|
| Cell Line | Invader Gene Ratio | Antibody Production (Bot/YP) |
| Bot Parent Cell Line | 1.12 | 4.8 mg/ml |
| Bot Production Cell Line | 3.03 | 55 mg/ml |
| YP Parent Cell Line | 1.32 | 4 mg/ml |
| YP Production Cell Line | 2.04 | 25 mg/ml |

### Example 20

### Transfection with Lentivirus Vectors

This example describes methods for the production of lentivirus vectors and their use to infect host cells at a high multiplicity of infection. Replication-defective viral particles are produced by the transient cotransfection of the plasmids described in U.S. Pat. No. 6,013,516 in 293T human kidney cells. All plasmids are transformed and grown in *E*. *coli* HB101 bacteria following standard molecular biology procedures. For transfection of eukaryotic cells, plasmid DNA is purified twice by equilibrium centrifugation in CsCl-ethidium bromide gradients. A total of 40 µg DNA is used for the transfection of a culture in a 10 cm dish, in the following proportions: 10 µg pCMVΔR8, 20 µg pHR', and 10 µg env plasmids, either MLV/Ampho, MLV/Eco or VSV-G. 293T cells are grown in DMEM supplemented with 10% fetal calf serum and antibiotics in a 10% CO₂ incubator. Cells are plated at a density of 1.3×10⁶/10 cm dish the day before transfection. Culture medium is changed 4 to 6 hrs before transfection. Calcium phosphate-DNA complexes are prepared according to the method of Chen and Okayama (Mol. Cell. Biol., 7:2745, 1987), and incubated overnight with the cells in an atmosphere of 5% CO₂, The following morning, the medium is replaced, and the cultures returned to 10% CO₂, Conditioned medium is harvested 48 to 60 hrs after transfection, cleared of cellular debris by low speed centrifugation (300xg 10 min), and filtered through 0.45 µm low protein binding filters.

To concentrate vector particles, pooled conditioned medium harvested as described above is layered on top of a cushion of 20% sucrose solution in PBS and centrifuged in a Beckman SW28 rotor at 50,000xg for 90 min. The pellet is resuspended by incubation and gentle pipetting in 1-4 ml PBS for 30-60 min, then centrifuged again at 50,000x for 90 min in a Beckmann SW55 rotor. The pellet is resuspended in a minimal volume (20-50 µl) of PBS and either used directly for infection or stored in frozen aliquots at -80° C.

The concentrated lentivirus vectors are titered and used to transfect an appropriate cell line (*e.g.,* 293 cells, Hela cells, rat 208F fibroblasts)) at a multiplicity of infection of 1,000. Analysis of clonally selected cell lines expressing the exogenous protein will reveal that a portion of the selected cell lines contain more than two integrated copies of the vector. These cell lines will produce more of the exogenous protein than cell lines containing only one copy of the integrated vector.

### Example 21

### Expression and Assay of G-protein Coupled Receptors

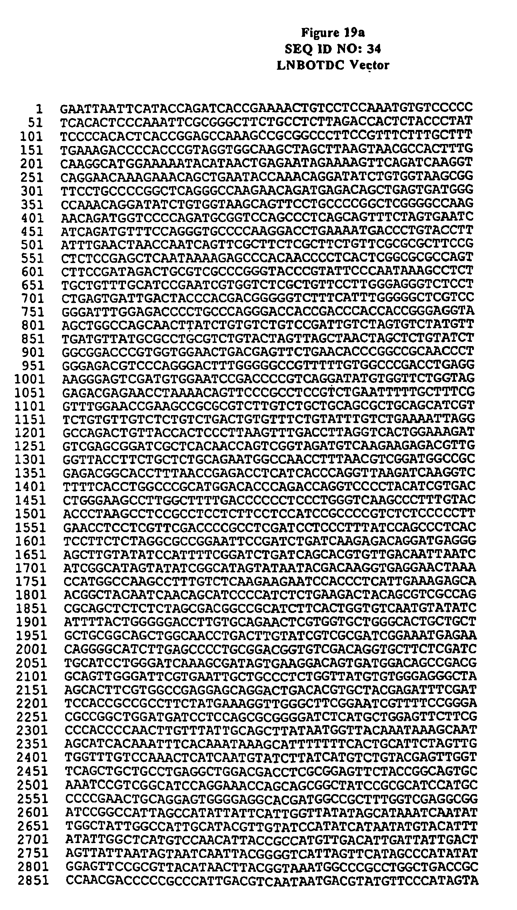

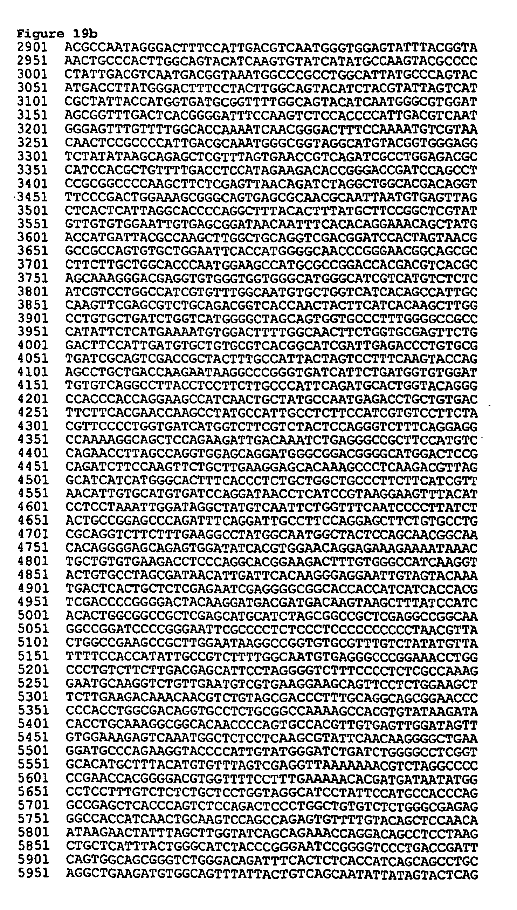

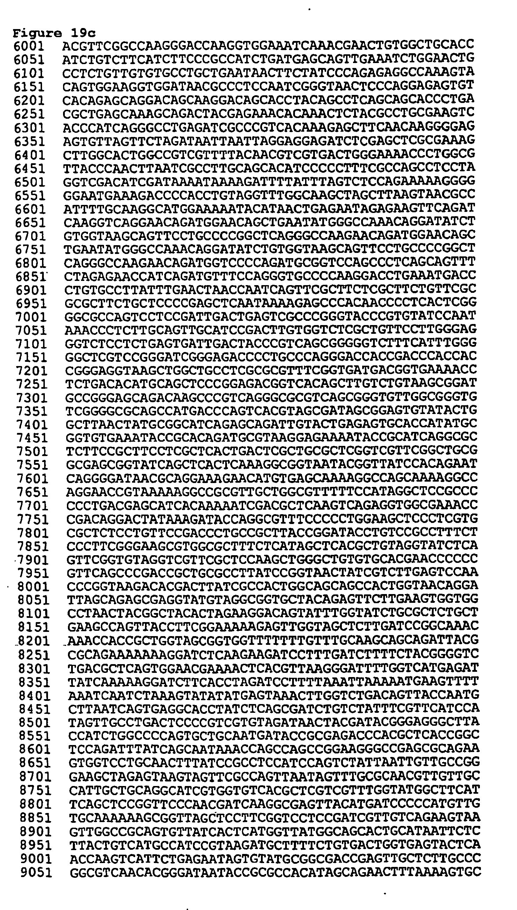

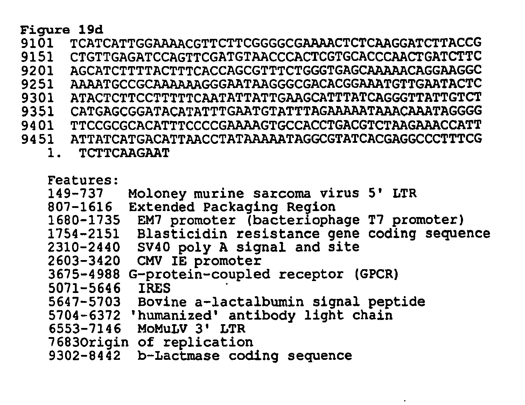

This example describes the expression of a G-Protein Coupled Receptor protein (GPCR) from a retroviral vector. This example also describes the expression of a signal protein from an IRES as a marker for expression of a difficult to assay protein or a protein that has no assay such as a GPCR. The gene construct (SEQ ID NO: 34; Figure 19) comprises a G-protein-coupled receptor followed by the IRES-signal peptide-antibody light chain cloned into the MCS of pLBCX retroviral backbone. Briefly, a PvuII/PvuII fragment (3057 bp) containing the GPCR-IRES-antibody light chain was cloned into the StuI site of pLBCX. pLBCX contains the EM7 (T7) promoter, Blasticidin gene and SV40 polyA in place of the Neomycin resistance gene from pLNCX.

The gene construct was used to produce a replication defective retroviral packaging cell line and this cell line was used to produce replication defective retroviral vector. The vector produced from this cell line was then used to infect 293GP cells (human embryonic kidney cells). After infection, the cells were placed under Blasticidin selection and single cell Blasticidin resistant clones were isolated. The clones were screened for expression of antibody light chain. The top 12 light chain expressing clones were selected. These 12 light chain expressing clones were then screened for expression of the GPCR using a ligand binding assay. All twelve of the samples also expressed the receptor protein. The clonal cell lines and there expression are shown in Table 9.

| **Table 9** | | |
|---|---|---|
| Cell Clone Number | Antibody Light Chain Expression | GPCR Expression |
| 4 | + | + |
| 8 | + | + |
| 13 | + | + |
| 19 | + | + |
| 20 | + | + |
| 22 | + | + |
| 24 | + | + |
| 27 | + | + |
| 30 | + | + |
| 45 | + | + |
| 46 | + | + |
| 50 | + | + |

### Example 22

### Multiple infection of 293 cells with replication defective retroviral vector

This example describes the multiple serial transfection of cells with retroviral vectors. The following gene construct was used to produce a replication defective retroviral packaging cell line.
- 5' LTR =: Moloney murine sarcoma virus 5' long terminal repeat.
- EPR =: Moloney murine leukemia virus extended packaging region.
- Blast =: Blasticidin resistance gene.
- CMV =: Human cytomegalovirus immediate early promoter.
- Gene =: Gene encoding test protein
- WPRE =: RNA transport element
- 3' LTR =: Moloney murine leukemia virus 3' LTR.

This packaging cell line was then used to produce a replication defective retroviral vector arranged as follows. The vector was produced from cells grown in T150 flasks and frozen. The frozen vector was thawed at each infection. For infection # 3 a concentrated solution of vector was used to perform the infection. All other infections were performed using non-concentrated vector. The infections were performed over a period of approximately five months by placing 5 ml of vector/media solution on a T25 flask containing 30% confluent 293 cells. Eight mg/ml of polybrene was also placed in the vector solution during infection. The vector solution was left on the cells for 24 hours and then removed. Media (DMEM with 10% fetal calf serum) was then added to the cells. Cells were grown to full confluency and passaged into a new T25 flask. The cells were then grown to 30% confluency and the infection procedure was repeated. This process was repeated 12 times and is outlined Table 10 below. After infections 1, 3, 6, 9 and 12, cells left over after passaging were used to obtain a DNA sample. The DNA was analyzed using the INVADER assay to determine an estimate of the number of vector inserts in the cells after various times in the infection procedure. The results indicate that the number of vector insertions goes up over time with the highest level being after the 12^{th} infection. Since a value of 0.5 is approximately an average of one vector insert copy per cell, after twelve infections the average vector insert copy has yet to reach two. These data indicates that the average vector copy per cell is a little less that 1.5 copies per cell. Also, there was no real change in gene copy number from infection #6 to infection #9. Furthermore, these data indicate that transfection conducted at a standard low multiplicity of infection fail to introduce more than one copy of the retroviral vector into the cells.

| **Table 10** | | |
|---|---|---|
| Cell Line or Infection Number | Vector Titer (CFU/ml) | "Invader" Gene Ratio |
| 293 | | 0.053 |
| Infection #1 | 1.05 X 10³ | 0.39 |
| Infection #2 | 1.05 X 10³ | |
| Infection #3 | 7.6 X 10⁴ | 0.45 |
| Infection #4 | 1.05 X 10³ | |
| Infection #5 | 1.05 X 10³ | |
| Infection #6 | 1.05 X 10³ | 0.54 |
| Infection #7 | 1.05 X 10³ | |
| Infection #8 | 1.05 X 10³ | |
| Infection #9 | 1.05 X 10³ | 0.52 |
| Infection #10 | 1.05 X 10³ | |
| Infection #11 | 1.05 X 10³ | |
| Infection #12 | 1.05 X 10³ | 0.69 |

### Example 23

### Production of YP antibody

This Example demonstrates the production of *Yersinia pestis* antibody by bovine mammary epithelial cells and human kidney fibroblast cells (293 cells). Cells lines were infected with the α-LA YP vector. Both of the cell lines produced YP antibody. All of the antibody is active and the heavy and light chains are produced in a ratio approximating 1:1.

### Example 24

### Stability of Vector Insertions in Cell Lines Over Time

Two cell lines that contain gene inserts of the LN-CMV-Bot vector were analyzed for there ability to maintain the vector inserts over a number of passages with and without neomycin selection. The first cell line is a bovine mammary epithelial cell line that contains a low number of insert copies. The second cell line is a 293GP line that contains multiple copies of the vector insert. At the start of the experiment, cell cultures were split. This was at passage 10 for the bovine mammary epithelial cells and passage 8 for the 293GP cells. One sample was continually passaged in media containing the neomycin analog G418, the other culture was continually passaged in media without any antibiotic. Every 3-6 passages, cells were collected and DNA was isolated for determination of gene ratio using the INVADER assay. Cell were continually grown and passaged in T25 flasks. The results of the assays are shown below:

| **Table 11** | | |
|---|---|---|
| **Low Gene Copy Cell Line** | | |
| Cell Line and Treatment | Passage Number | INVADER Gene Ratio |
| BMEC/Bot #66 + G418 | 10 | 0.67 |
| BMEC/Bot #66 - G418 | 10 | 0.89 |
| BMEC/Bot #66 + G418 | 16 | 0.67 |
| BMEC/Bot #66 - G418 | 16 | 0.64 |
| BMEC/Bot #66 + G418 | 21 | 0.62 |
| BMEC/Bot #66 - G418 | 21 | 0.58 |
| BMEC/Bot #66 + G418 | 27 | 0.98 |
| BMEC/Bot #66-G418 | 27 | 0.56 |
| BMEC/Bot #66 + G418 | 33 | 0.80 |
| BMEC/Bot #66 - G418 | 33 | 0.53 |

| **Table 12** | | |
|---|---|---|
| **High Gene Copy Cell Line** | | |
| Cell Line and Treatment | Passage Number | INVADER Gene Ratio |
| 293GP/Bot #23 + G418 | 8 | 3.46 |
| 293GP/Bot #23 - G418 | 8 | 3.73 |
| 293GP/Bot #23 + G418 | 14 | 3.28 |
| 293GP/Bot #23 - G418 | 14 | 3.13 |
| 293GP/Bot #23 + G418 | 17 | 3.12 |
| 293GP/Bot #23 - G418 | 17 | 2.91 |
| 293GP/Bot #23 + G418 | 22 | 3.6 |
| 293GP/Bot #23 - G418 | 22 | 2.58 |
| 293GP/Bot #23 + G418 | 28 | 2.78 |
| 293GP/Bot #23 - G418 | 28 | 3.44 |
| 293GP/Bot #23 + G418 | 36 | 2.6 |
| 293GP/Bot #23 - G418 | 36 | 2.98 |

These data show that there are no consistent differences in gene ratio between cells treated with G418 and those not treated with antibiotic. This suggests that G418 selection is not necessary to maintain the stability of the vector gene insertions. Also, these vector inserts appear to be very stable over time.

## Claims

1. A clonally selected host cell comprising a genome, said genome comprising at least 10 copies of a pseudo-typed retroviral vector, wherein said retroviral vector comprises at least one exogenous gene operably linked to a promoter internal to the 5' and 3' long terminal repeats of said retroviral vector, said gene encoding a secreted protein, wherein said host cell is **characterized in that** it synthesizes at least 1 picogram of said exogenous protein per day.

2. The host cell of Claim 1, wherein said retroviral vectors further comprise a secretion signal sequence operably linked to said exogenous gene.

3. The host cell of Claim 1, wherein said retroviral vectors further comprise an RNA export element operably linked to said exogenous gene, and
wherein said retroviral vectors preferably comprise at least two exogenous genes.

4. The host cell of Claim 3, wherein one of said at least two exogenous genes is a selectable marker.

5. The host cell of Claim 1, wherein said retroviral vector comprises long terminal repeats selected from the group consisting of MoMLV, MoMuSV, and MMTV long terminal repeats, and
wherein said retroviral vector preferably is a lentiviral vector.

6. The host cell of Claim 1, wherein said host cell is selected from Chinese hamster ovary cells, baby hamster kidney cells, and bovine mammary epithelial cells, and
wherein the genome preferably is stable for greater than 10 passages, preferably greater than 50 passages or more preferably for greater than 100 passages.

7. The host cell of Claim 1, wherein said promoter is selected from the group consisting of alpha-lactalbumin promoter, cytomegalovirus promoter and the long terminal repeat of Moloney murine leukemia virus,
preferably further comprising a first integrating vector encoding a first secreted protein of interest, further comprising at least 1 integrated copy of a
second integrating vector encoding a second secreted protein of interest.

8. An ex-vivo method for transfecting host cells, wherein more than 10 copies of an expression vector for the protein of interest are inserted into the genome of the host cell so that the transfected host cells synthesize at least 1 picogram per cell per day of the protein of interest, **characterized in that** a host cell comprising a genome is contacted with a pseudotyped retroviral vector and that subsequently a transfected host cell is clonally selected, wherein the retroviral vector comprises at least one exogenous gene operably linked to a promoter internal to the 5' and 3' retroviral long terminal repeats, and the host cell is selected from the group consisting of Chinese Hamster Ovary cells, baby hamster kidney cells and bovine mammary epithelial cells.

9. A host cell obtainable by the method of Claim 8.

10. A method for expressing a protein of interest comprising:
1) providing:
a) a host cell of any one of Claims 1-7;
2) culturing said host cells under conditions such that said protein of interest is expressed;
3) isolating said protein of interest.

11. The method of Claim 10, wherein said genome of said host comprises at least two integrating vectors, wherein each of said at least two integrating vectors comprises a different exogenous gene.

12. A method comprising:
1) providing:
a) a mammalian host cell comprising a genome, said genome comprising multiple copies of a pseudotyped retroviral vector comprising a promoter operably linked to an exogenous gene encoding a first secreted protein of interest, and
b) second pseudotyped retroviral vectors comprising an exogenous gene encoding a second secreted protein of interest; and
2) contacting said mammalian host cell with said second retroviral vectors, wherein at least 10 copies of said second retroviral vectors are inserted into the genome of the mammalian host cell, so that the host cells synthesize at least 1 picogram of the second protein of interest per day, **characterized in that** a host cell comprising a genome is contacted with a retroviral vector at a multiplicity of infection of greater than 100, and that subsequently a transfected host cell is clonally selected.

13. The method of Claim 12, wherein said conditions comprise contacting said host at a multiplicity of infection from 100 to 1000.

14. The method of Claim 8, 10 or 12, wherein said retroviral vector is a lentiviral vector, and
wherein said retroviral vector preferably is a pseudo-typed retrovirus vector comprising a G glycoprotein.

15. The method of Claim 8, further comprising the step of isolating said protein of interest.

## Patentansprüche

1. Klonal selektierte Wirtszelle umfassend ein Genom welches mindestens 10 Kopien eines pseudotypisierten retroviralen Vektors umfasst, wobei der retrovirale Vektor mindestens ein exogenes Gene umfasst, das in operativer Weise mit einem zu den 5' und 3' Long Terminal Repeats des retroviralen Vektors internen Promotor verbunden ist und das Gen ein sezernierendes Protein kodiert, wobei die Wirtszelle **dadurch gekennzeichnet ist, dass** sie mindestens 1 Pikogramm des exogenen Proteins pro Tag synthetisiert.

2. Wirtszelle nach Anspruch 1, wobei die retroviralen Vektoren des Weiteren eine in operativer Weise mit dem exogenen Gen verbundene Sekretionssignalsequenz umfassen.

3. Wirtszelle nach Anspruch 1, wobei die retroviralen Vektoren des Weiteren ein in operativer Weise mit dem exogenen Gen verbundenes RNA-Exportelement umfassen und wobei die retroviralen Vektoren vorzugsweise mindestens zwei exogene Gene umfassen.

4. Wirtszelle nach Anspruch 3, wobei eines der mindestens zwei exogenen Gene ein Selektionsmarker ist.

5. Wirtszelle nach Anspruch 1, wobei der retrovirale Vektor Long Terminal Repeats umfasst, die ausgewählt sind aus der Gruppe bestehend aus MoMLV, MoMuSV und MMTV Long Terminal Repeats, und wobei der retrovirale Vektor vorzugsweise ein lentiviraler Vektor ist.

6. Wirtszelle nach Anspruch 1, wobei die Wirtszelle ausgewählt ist aus Eierstockzellen des chinesischen Hamsters, Babyhamsternierenzellen und Rindermilchdrüsenepithelzellen, und wobei das Genom vorzugsweise über mehr als 10 Passagen, vorzugsweise über mehr als 50 Passagen oder weiter bevorzugt über mehr als 100 Passagen stabil ist.

7. Wirtszelle nach Anspruch 1, wobei der Promotor ausgewählt ist aus der Gruppe bestehend aus dem alpha-Laktalbuminpromotor, dem Cytomegaloviruspromotor und dem Long Terminal Repeat des Moloney-Mausleukämievirus, des Weiteren vorzugsweise umfassend einen ersten Integrationsvektor, der ein erstes sezernierendes Protein von Interesse kodiert, des Weiteren umfassend zumindest 1 integrierte Kopie eines zweiten Integrationsvektors, der ein zweites sezernierendes Protein von Interesse kodiert.

8. Ex-vivo Verfahren zur Transfektion von Wirtszellen, wobei mehr als 10 Kopien eines Expressionsvektors für ein Protein von Interesse in das Genom der Wirtszelle eingefügt werden, so dass die transfizierten Wirtszellen mindestens 1 Pikogramm des Proteins von Interesse pro Zelle pro Tag synthetisieren, **dadurch gekennzeichnet, dass** eine ein Genom umfassende Wirtszelle mit einem pseudotypisierten retroviralen Vektor in Kontakt gebracht und nachfolgend eine transfizierte Wirtszelle klonal selektiert wird, wobei der retrovirale Vektor mindestens ein exogenes Gen umfasst, das in operativer Weise mit einem zu den 5' und 3' Long Terminal Repeats des retroviralen Vektors internen Promotor verbunden ist, und die Wirtszelle ist ausgewählt aus der Gruppe bestehend aus Eierstockzellen des chinesischen Hamsters, Babyhamsternierenzellen und Rindermilchdrüsenepithelzellen.

9. Wirtszelle erhältlich durch das Verfahren nach Anspruch 8.

10. Verfahren zur Expression eines Proteins von Interesse, umfassend:
1) Bereitstellung:
a) einer Wirtszelle nach einem der Ansprüche 1-7;
2) Kultivierung der Wirtszellen unter solchen Bedingungen, dass das Protein von Interesse exprimiert wird;
3) Isolierung des Proteins von Interesse.

11. Verfahren nach Anspruch 10, wobei das Genom des Wirts mindestens zwei integrierte Vektoren umfasst, wobei jeder der mindestens zwei integrierten Vektoren ein anderes exogenes Gen umfasst.

12. Verfahren umfassend:
1) Bereitstellung:
a) einer Säugetierwirtszelle umfassend ein Genom das eine Vielzahl an Kopien eines pseudotypisierten retroviralen Vektors umfasst, der einen in operativer Weise mit einem exogenen Gen, welches für ein erstes Sekretionsprotein von Interesse kodiert, verbundenen Promotor umfasst, und
b) zweiter pseudotypisierter retroviraler Vektoren umfassend ein exogenes Gen, das ein zweites Sekretionsprotein von Interesse kodiert; und
2) Kontaktieren der Säugetierwirtszelle mit den zweiten retroviralen Vektoren, wobei mindestens 10 Kopien der zweiten retroviralen Vektoren in das Genom der Säugetierwirtszelle inseriert werden, so dass die Wirtszellen mindestens 1 Pikogramm des zweiten Proteins von Interesse pro Tag synthetisieren, **dadurch gekennzeichnet, dass** die das Genom umfassende Wirtszelle mit einem pseudotypisierten retroviralen Vektor mit einer Multiplizität der Infektion von mehr als 100 in Kontakt gebracht und dass nachfolgend eine transfizierte Wirtszelle klonal selektiert wird.

13. Verfahren nach Anspruch 12, wobei die Bedingungen ein Kontaktieren der Wirtszelle mit einer Multiplizität der Infektion von 100 bis 1000 umfassen.

14. Verfahren nach Anspruch 8, 10 oder 12, wobei der retrovirale Vektor ein lentiviraler Vektor ist und wobei der retrovirale Vektor vorzugsweise ein pseudotypisierter Retrovirusvektor ist der ein G-Glykoprotein umfasst.

15. Verfahren nach Anspruch 8, des Weiteren umfassend den Schritt der Isolierung des Proteins von Interesse.

## Revendications

1. Cellule hôte obtenue par sélection clonale comprenant un génome, ledit génome comprenant au moins 10 copies d'un vecteur rétroviral pseudotypé, où ledit vecteur rétroviral comprend au moins un gène exogène en liaison fonctionnelle avec un promoteur interne aux longues répétions terminales 5' et 3' dudit vecteur rétroviral, ledit gène codant pour une protéine sécrétée, où ladite cellule hôte est **caractérisée en ce qu'**elle synthétise au moins 1 picogramme de ladite protéine exogène par jour.

2. Cellule hôte selon la revendication 1, dans laquelle lesdits vecteurs rétroviraux comprennent en outre une séquence signal de sécrétion en liaison fonctionnelle avec ledit gène exogène.

3. Cellule hôte selon la revendication 1, dans laquelle lesdits vecteurs rétroviraux comprennent en outre un élément d'exportation de l'ARN en liaison fonctionnelle avec ledit gène exogène, et
où lesdits vecteurs rétroviraux comprennent de préférence au moins deux gènes exogènes.

4. Cellule hôte selon la revendication 3, dans laquelle l'un desdits au moins deux gènes exogènes est un marqueur de sélection.

5. Cellule hôte selon la revendication 1, dans laquelle ledit vecteur rétroviral comprend des longues répétitions terminales sélectionnées dans le groupe consistant en les longues répétitions terminales du MoMLV, MoMuSV, et MMTV, et
où ledit vecteur rétroviral est de préférence un vecteur lentiviral.

6. Cellule hôte selon la revendication 1, où ladite cellule hôte est sélectionnée parmi des cellules d'ovaire de hamster chinois, des cellules rénales de hamster nouveau-né, et des cellules épithéliales mammaires bovines, et
où le génome est de préférence stable pendant plus de 10 passages, de préférence plus de 50 passages ou de manière davantage préférée pendant plus de 100 passages.

7. Cellule hôte selon la revendication 1, dans laquelle ledit promoteur est sélectionné dans le groupe consistant en le promoteur de l'alpha-lactalbumine, le promoteur du cytomégalovirus et la longue répétition terminale du virus de la leucémie murine de Moloney,
comprenant en outre de préférence un premier vecteur d'intégration codant pour une première protéine sécrétée d'intérêt, comprenant en outre au moins 1 copie intégrée d'un second vecteur d'intégration codant pour une seconde protéine sécrétée d'intérêt.

8. Procédé *ex vivo* pour transfecter des cellules hôtes, dans lequel plus de 10 copies d'un vecteur d'expression de la protéine d'intérêt sont insérées dans le génome de la cellule hôte de sorte que les cellules hôtes transfectées synthétisent au moins 1 picogramme de la protéine d'intérêt par cellule par jour, **caractérisé en ce qu'**une cellule hôte comprenant un génome est mise en contact avec un vecteur rétroviral pseudotypé et que par la suite une cellule hôte transfectée est obtenue par sélection clonale, où le vecteur rétroviral comprend au moins un gène exogène en liaison fonctionnelle avec un promoteur interne aux longues répétitions terminales rétrovirales 5' et 3', et la cellule hôte est sélectionnée dans le groupe consistant en des cellules d'ovaire de hamster chinois, des cellules rénales de hamster nouveau-né et des cellules épithéliales mammaires bovines.

9. Cellule hôte pouvant être obtenue par le procédé selon la revendication 8.

10. Procédé pour exprimer une protéine d'intérêt, consistant à :
1) mettre à disposition :
a) une cellule hôte selon l'une quelconque des revendications 1 à 7 ;
2) cultiver lesdites cellules hôtes dans des conditions telles que ladite protéine d'intérêt est exprimée ;
3) isoler ladite protéine d'intérêt.

11. Procédé selon la revendication 10, dans lequel ledit génome dudit hôte comprend au moins deux vecteurs d'intégration, où chacun desdits au moins deux vecteurs d'intégration comprend un gène exogène différent.

12. Procédé consistant à :
1) mettre à disposition :
a) une cellule hôte de mammifère comprenant un génome, ledit génome comprenant de multiples copies d'un vecteur rétroviral pseudotypé comprenant un promoteur en liaison fonctionnelle avec un gène exogène codant pour une première protéine sécrétée d'intérêt, et
b) des seconds vecteurs rétroviraux pseudotypés comprenant un gène exogène codant pour une seconde protéine sécrétée d'intérêt ; et
2) mettre en contact ladite cellule hôte de mammifère avec lesdits seconds vecteurs rétroviraux, où au moins 10 copies desdits seconds vecteurs rétroviraux sont insérées dans le génome de la cellule hôte de mammifère, de sorte que les cellules hôtes synthétisent au moins 1 picogramme de la seconde protéine d'intérêt par jour, **caractérisé en ce qu'**une cellule hôte comprenant un génome est mise en contact avec un vecteur rétroviral à une multiplicité d'infection supérieure à 100, et que par la suite une cellule hôte transfectée est obtenue par sélection clonale.

13. Procédé selon la revendication 12, dans lequel lesdites conditions consistent à mettre en contact ledit hôte à une multiplicité d'infection comprise entre 100 et 1000.

14. Procédé selon la revendication 8, 10 ou 12, dans lequel ledit vecteur rétroviral est un vecteur lentiviral, et
dans lequel ledit vecteur rétroviral est de préférence un vecteur rétroviral pseudotypé comprenant une glycoprotéine G.

15. Procédé selon la revendication 8, comprenant en outre l'étape consistant à isoler ladite protéine d'intérêt.
